# FASCICULE DE BREVET EUROPEEN

(11) **EP 3 233 836 B1**
(45) Date de publication et mention de la délivrance du brevet: **30.01.2019**
(21) Numéro de dépôt: 15816174.5
(22) Date de dépôt: 18.12.2015
(51) Int. Cl.: C07D 405/06, C07D 403/06, C07D 231/56, C07D 401/06, C07D 413/06, C07D 405/04, C07D 403/04, C07D 405/14, A61K 31/416, A61P 17/00

(54) **DÉRIVÉS INDAZOLES SULFONAMIDES EN TANT QU'AGONISTES INVERSES DU RÉCEPTEUR GAMMA ORPHELIN ASSOCIÉ AUX RÉTINOÏDES ROR GAMMA (T)**
INDAZOLSULFONAMIDDERIVATE ALS INVERSE AGONISTEN VON RETINOID-RELATED ORPHAN RECEPTOR GAMMA (ROR GAMMA (T))
INDAZOLE SULFONAMIDE DERIVATIVES AS INVERSE AGONISTS OF RETINOID-RELATED ORPHAN RECEPTOR GAMMA (ROR GAMMA (T))

(30) Priorité: 19.12.2014 FR 1463035; 03.07.2015 FR 1556341
(43) Date de publication de la demande: 25.10.2017
(73) Titulaire: Galderma Research & Development, 06410 Biot (FR)
(72) Inventeur: MUSICKI, Branislav, 06000 Nice (FR); OUVRY, Gilles, 06410 Biot (FR); BOUIX-PETER, Claire, 06220 Vallauris (FR); THOREAU, Etienne, 06460 Saint Vallier de Thiey (FR)
(74) Mandataire: Casalonga
(86) Numéro de dépôt international: PCT/EP2015/080689
(87) Numéro de publication internationale: WO 2016/097391

(56) Documents cités:
- WO-A1-2006/052190
- WO-A1-2013/160418
- WO-A1-2014/090712
- YAN ZHANG ET AL: "Discovery of 2-oxo-1,2-dihydrobenzo[cd]indole-6-sulfona mide derivatives as new ROR[gamma] inhibitors using virtual screening, synthesis and biological evaluation", EUROPEAN JOURNAL OF MEDICINAL CHEMISTRY, vol. 78, 22 mars 2014 (2014-03-22), pages 431-441, XP028847891, ISSN: 0223-5234, DOI: 10.1016/J.EJMECH.2014.03.065

## Description

La présente invention concerne des dérivés sulfonamides bicycles particuliers, leurs sels d'addition pharmaceutiquement acceptables leurs hydrates et/ou leurs solvates ainsi que leur utilisation en tant qu'agoniste inverse du récepteur gamma orphelin associé aux rétinoïdes RORyt.

L'invention est également relative à une composition pharmaceutique comprenant de tels composés ainsi que son utilisation pour le traitement par voie topique et/ou orale des maladies inflammatoires médiées par les récepteurs RORyt, notamment l'acné, dermatite atopique et/ou le psoriasis.

Les récepteurs nucléaires forment une grande famille (appelée superfamille) de facteurs de transcription qui correspondent à des protéines capables d'être activées par un ligand, de se fixer sur des séquences d'ADN spécifiques et de réguler la transcription de gènes cibles. Ainsi ces récepteurs se retrouvent impliqués dans la régulation d'une grande variété de fonctions biologiques, dont la croissance, le développement, la reproduction, la différenciation et le métabolisme dans une multitude d'organismes vivants.

Les premiers membres de cette superfamille à avoir été identifiés et décrits dans la littérature scientifique sont les récepteurs nucléaires des hormones stéroïdes tels que les récepteurs aux glucocorticoïdes et les récepteurs oestrogènes. Cette superfamille comprend également parmi ses membres de nombreux récepteurs pour lesquels aucun ligand n'a été identifié. Ces récepteurs nucléaires sont appelés « récepteurs orphelins ».

Les récepteurs orphelins associés aux rétinoïdes (*Retinoid-related orphan receptors* en langue anglaise) constituent donc une sous-famille des récepteurs nucléaires. Cette sous-famille est composée de trois membres ayant chacun leur propre profil d'expression : ROR alpha (dénommé RORα), ROR beta (dénommé RORβ) et ROR gamma (dénommé RORγ). Deux isoformes des récepteurs orphelins RORγ ont déjà été identifiés, à savoir RORγ1, qui s'exprime dans une variété de tissus tels que le thymus, les reins, les muscles et le foie, et RORγ2 (aussi nommé RORγt) qui s'exprime exclusivement dans les cellules du système immunitaire.

En particulier, le récepteur RORγt joue un rôle important de régulateur dans la différenciation cellulaire des lymphocytes Th17 qui correspondent à des lymphocytes T auxiliaires ayant pour fonction d'assurer la défense de l'organisme par rapport à un grand nombre d'agents pathogènes extracellulaires tels que les bactéries et les infections fongiques.

Toutefois, il a été démontré que les lymphocytes Th17 sont également impliqués dans une large variété de désordres inflammatoires, tels que l'acné, et de maladies auto-immunes telles que le psoriasis, l'arthrite rhumatoïde ou encore la sclérose en plaques (Peck A, Mellins ED. Precarious balance; Th17 cells in host defense. Infect Immun. 2010 Jan ; 78(1) :32-8 ; Suarez-Farinas: J. Allergy Clin. Immunol. 2014; J. Invest. Dermatol. 2008, 128(11), 2625).

En effet, les lymphocytes Th17 produisent de nombreuses cytokines ayant des profils distincts telles que l'interleukine-17A (IL-17A), l'interleukine-17F (IL-17F), l'interleukine-26 (IL-26), l'interleukine-21 (IL-21), l'interleukine-22 (IL-22) et le TNFα dont leur développement, leur survie et leur prolifération dépendent de l'interleukine-23 (IL-23). Ces cytokines sont capables d'activer différents types de cellules effectrices, telles que les kératinocytes, conduisant ainsi à leur hyperprolifération et à la production supplémentaire de cytokines pro-inflammatoires, de chimiokines et de peptides antimicrobiens, qui à leur tour recrutent et activent d'autres cellules du système immunitaire dans la peau enflammée, ce qui peut conduire à une amplification de la réponse inflammatoire.

Ainsi l'activation des lymphocytes Th17 est responsable du recrutement de cytokines, notamment d'interleukine-17 (IL17), et d'autres types de cellules pro-inflammatoires qui vont conduire à la médiation de désordres inflammatoires tels que l'acné et/ou de maladies auto-immunes telles que le psoriasis.

Des expériences menées sur des souris montrent qu'une diminution au niveau de l'expression du récepteur RORγt conduit à une baisse de l'activité des lymphocytes Th17 ce qui permet, par conséquent, de fortement réduire l'expression d'interleukine-17 (IL-17) (Ivanov II, McKenzie BS, Zhou L, Tadokoro CE, Lepelley A, Lafaille JJ, Cua DJ, Littman DR : Cell 2006, 126, 1121-1133) et de traiter efficacement les désordres inflammatoires et les maladies auto-immunes médiées par ces cytokines, notamment celles pour lesquelles des taux importants en interleukine-17 (IL-17) sont détectés.

A cet effet, la demande de brevet WO 2013/160418 décrit des composés sulfonamides utilisés comme agonistes inverses du récepteur RORyt afin de pouvoir traiter les désordres inflammatoires et les maladies auto-immunes. De la même façon, d'autres composés ont également été développés en tant qu'agonistes inverses du récepteur RORyt comme ceux décrits dans les demandes de brevet WO 2014/090712, WO 2014/008214, WO2013/169588, WO2013/160419, WO2013/1002027, WO2013/092939, WO2013/092941, WO2013/085890 et WO2012/100732.

Il existe donc un réel besoin de développer de nouveaux composés en tant qu'agonistes inverses du récepteur RORγt afin de pouvoir traiter efficacement les maladies médiées par un tel récepteur, notamment les désordres inflammatoires, tels que l'acné et/ou les maladies auto-immunes tels que le psoriasis ou la dermatite atopique.

Ce but est atteint grâce à la mise en oeuvre de dérivés sulfonamides bicycles particuliers tels que décrits ci-après qui permettent de moduler l'activité du récepteur RORγt et de traiter par conséquent efficacement les désordres inflammatoires et les maladies auto-immunes de certaines pathologies.

La présente invention a donc notamment pour objet un ou des composés de formule (I), leurs sels d'addition pharmaceutiquement acceptables, leurs hydrates et/ou leurs solvates

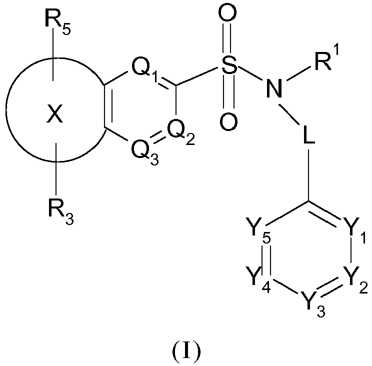

Formule (I) dans laquelle :
- L représente une liaison simple ou un groupe méthylène CH₂,
- X représente un radical cyclique choisi parmi les radicaux X₁ et X₂ suivants : 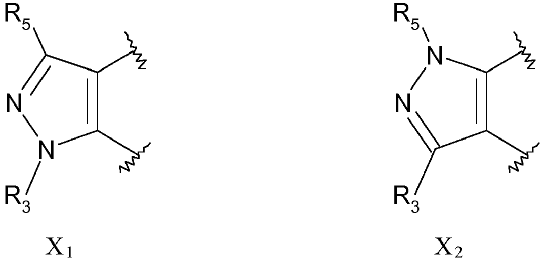
- un ou deux des éléments Y¹, Y², Y³, Y⁴ et Y⁵ représente(nt) un atome d'azote et les autres éléments correspondent à un groupement -CR², ou chacun des éléments Y¹, Y², Y³, Y⁴ et Y⁵ correspond à un groupement -CR²,
- un ou deux des éléments Q¹, Q² et Q³ représente(nt) un atome d'azote et le ou les autres éléments corresponde(nt) à un groupement - CR^{2a}, ou chacun des éléments Q¹, Q² et Q³ correspond à un groupement -CR^{2a},
- R¹ représente un radical alkyle, linéaire ou ramifié, en C₃-C₅, éventuellement substitué par un groupement hydroxyle et/ou un atome d'halogène, un radical cycloalkyle en C₃-C₅, un radical alkényle, linéaire ou ramifié, en C₂-C₅, un radical -CH₂-cycloalkyle en C₃-C₅, un radical hétérocycloalkyle en C₄-C₅, un radical -CH₂-hétérocycloalkyle en C₄-C₆,
- R² représente un atome d'hydrogène ou un atome d'halogène, un radical alkyle en C₁-C₅, linéaire ou ramifié, un radical alkényle en C₂-C₄, linéaire ou ramifié, un radical alcoxy en C₁-C₄, un groupement cyano -CN, un radical -C(=O)R'² avec R'² désignant un radical alcoxy en C₁-C₃, un radical -CF₃ ; lesdits radicaux alkyle, alkényle et alcoxy pouvant être substitués par un ou plusieurs atomes d'halogène ;
- R^{2a} représente un atome d'hydrogène ou un atome d'halogène, un radical alkyle en C₁-C₅, linéaire ou ramifié, un radical alkényle en C₂-C₄, linéaire ou ramifié, un radical alcoxy en C₁-C₄, un groupement -CN, un groupement hydroxy -OH, un groupement - CH(R^{3a})OH, un groupement carboxylique -COOH, un groupement carbamoyle -CONR^{2c}R^{2d}, un groupement amido -NR^{2c}COR^{2d}, un groupement -SO₂R^{2c} , un groupement -SOR^{2c} un groupement - S(=O)(=NH-R^{2c}), lesdits radicaux alkyle, alkényle et alcoxy pouvant être substitués par un ou plusieurs atomes d'halogène,
- R^{2c} et R^{2d}, identiques ou différents, représentent un atome d'hydrogène ou un radical alkyle, linéaire ou ramifié, en C₁-C₅ ;
- R^{3a} représente un atome d'hydrogène ou un radical alkyle, linéaire ou ramifié, en C₁-C₅ ;
- R³ représente un atome d'hydrogène, un atome d'halogène, un groupement (CHR⁶)ₙ-(Z)ₒ-(CHR^{,6})ₚ-R⁷, ou un groupement CH=R⁷,
- n, o et p, identiques ou différents, représentent zéro ou un entier naturel allant de 1 à 3,
- Z représente un groupement divalent choisi parmi un groupement méthylène -CH₂-, un groupement amino -NH- et un atome d'oxygène -O-,
- R⁶ et R'⁶, identiques ou différents, représentent un atome d'hydrogène, un groupement méthyle -CH₃, un groupement -OH, un groupement hydroxyméthyle, une fonction carboxylique -COOH,
- R⁷ représente :
   - un atome d'hydrogène ou un atome d'halogène,
   - un groupement COOR'⁷ avec R'⁷ désignant alkyl(C₁)hétérocycle(C₆),
   - un radical hétérocycloalkyle non cationique éventuellement substitué par un ou plusieurs atomes d'halogène, un ou plusieurs groupements alkyle en C₁-C₃, linéaire ou ramifié, un ou plusieurs groupements -OH, une ou plusieurs fonctions carbonyles, un ou plusieurs groupements hydroxyalkyle en C₁-C₄, linéaire ou ramifié, un ou plusieurs groupements amino, un ou plusieurs groupements -C(=O)R^{7a}, un ou plusieurs groupements S(=O)₂R^{7a} ; R^{7a} représentant un radical alkyle, linéaire ou ramifié, en C₁-C₃, un radical alcoxy en C₁-C₃, linéaire ou ramifié, ou un radical amino N(R^{8a})(R^{8b}),
   - un radical cycloalkyle non cationique en C₃-C₆ éventuellement substitué par un ou plusieurs radicaux méthyle, un ou plusieurs atomes d'halogène, un groupement cyano -CN ou un ou plusieurs groupements COR¹³; R¹³ désignant un radical alcoxy en C₁-C₃, linéaire ou ramifié, ou un groupement hydroxy,
   - un radical aromatique ou hétéroaromatique, non cationique, éventuellement substitué par un ou plusieurs atomes d'halogène, un ou plusieurs groupements alkyle en C₁-C₃, linéaire ou ramifié, éventuellement substitué par un ou plusieurs atomes d'halogène, un ou plusieurs groupements alcoxy en C₁-C₃, un ou plusieurs groupements amino -NR¹¹R¹², un ou plusieurs groupements -COR¹¹, un ou plusieurs groupements -COOR¹¹, un ou plusieurs groupements amido - CONR¹¹R¹², un ou plusieurs groupements -SOR¹¹, un ou plusieurs groupements -SO₂R¹¹, un ou plusieurs groupements - NHCOR¹¹, un ou plusieurs groupements -NHCOOR¹¹, un ou plusieurs groupements -SO₂NR¹¹R¹² ou un ou plusieurs groupements -CN ; R¹¹ et R¹², identiques ou différents, représentant un atome d'hydrogène, un radical hydroxy -OH, un radical alkyle, linéaire ou ramifié, en C₁-C₃, éventuellement substitué par un ou plusieurs atomes d'halogène,
- lorsque R³ représente un groupement -CH=R⁷ alors R⁷ ne représente pas un atome d'hydrogène, un atome d'halogène ou un groupement COOR'⁷,
- R⁵ représente un atome d'hydrogène ou un atome d'halogène, un radical alkyle en C₁-C₃, linéaire ou ramifié éventuellement substitué par un ou plusieurs atomes d'halogène; un radical amino -NH₂, un radical hétérocyclique en C₄-C₅, un radical OCH₂-hétérocyclique en C₄-C₅, un radical CH₂R^{'7a} avec R'^{7a} désignant un radical méthoxy, un groupement hydroxy -OH, un groupement -CH₂COOH, un groupement -CH(R^{5b})OH, un groupement carboxylique -COOH, un groupement -CN, une fonction thioxo,
- R^{5b} représente un atome d'hydrogène ; un radical alkyle, linéaire ou ramifié, en C₁-C₃ éventuellement substitué par une ou plusieurs fonctions carboxyliques ; un radical cyclopropyle,
- R^{8a} et R^{8b}, identiques ou différents, désignent un atome d'hydrogène, un radical alkyle, linéaire ou ramifié, en C₁-C₃ ou un radical cyclopropyle.

Le ou les composés selon l'invention correspondent ainsi à des dérivés sulfonamides bicycles, soit donc à un ou des composés sulfonamides comportant dans leur structure au moins deux cycles qui sont condensés l'un sur l'autre.

En d'autres termes, X est un radical cyclique condensé sur le noyau aromatique comportant les éléments Q¹, Q² et Q³ tels que définis ci-avant.

Conformément à la définition de la formule (I), la liaison intracyclique entre le radical cyclique X₁ ou X₂, tel que représenté ci-avant, et le noyau aromatique comportant les éléments Q₁ à Q³ est une liaison double. Ainsi la double liaison est commune entre le radical cyclique X₁ ou X₂ et le noyau aromatique comportant les éléments Q₁ à Q₃.

Les composés selon l'invention permettent de moduler, c'est-à-dire d'inhiber, l'activité du récepteur RORyt.

La présente invention a également pour objet le ou les composés tels que définis précédemment en tant que médicament et cosmétique.

Un autre objet de l'invention porte sur le ou les composés tels que définis précédemment pour leur utilisation dans le traitement des maladies médiées par le récepteur RORyt, notamment les désordres inflammatoires et/ou les maladies auto-immunes médiées par le récepteur RORyt.

Par ailleurs, l'invention concerne également une composition pharmaceutique comprenant, dans un milieu pharmaceutiquement acceptable, un ou plusieurs composés de formule (I) telle que définie précédemment, leurs sels d'addition pharmaceutiquement acceptables, leurs hydrates et/ou leurs solvates.

La présente invention a aussi trait à la composition pharmaceutique telle que décrite précédemment pour son utilisation dans le traitement des maladies médiées par le récepteur RORyt, notamment les désordres inflammatoires et/ou les maladies auto-immunes.

Enfin, l'invention est relative à une méthode de traitement des maladies médiées par le récepteur RORyt comprenant l'administration, notamment par voie topique ou orale, d'une quantité thérapeutiquement efficace d'un ou plusieurs composés tels que définis ci-avant à un patient.

D'autres objets et caractéristiques, aspects et avantages de l'invention apparaîtront encore plus clairement à la lecture de la description et des exemples qui suivent.

Selon un mode de réalisation, dans la formule (I) :
- R³ représente un atome d'hydrogène, un groupement (CHR⁶)ₙ-(Z)ₒ-(CHR'⁶)ₚ-R⁷, un groupement CH=R⁷,
- R⁵ représente un atome d'hydrogène ou un atome d'halogène, un radical alkyle en C₁-C₃, linéaire ou ramifié éventuellement substitué par un ou plusieurs atomes d'halogène; un radical amino -NH₂, un radical CH₂R'^{7a} avec R'^{7a} désignant un radical méthoxy, un groupement hydroxy -OH, un groupement - CH₂COOH, un groupement -CH(R^{5b})OH, un groupement carboxylique -COOH, un groupement -CN, une fonction thioxo.

Selon un mode de réalisation, dans la formule (I), L représente une liaison simple.

Selon un autre mode de réalisation, dans la formule (I), L représente un groupe méthylène -CH₂.

Préférentiellement, dans la formule (I), L représente une liaison simple.

De préférence :
- lorsque R⁵ est relié à un atome d'azote alors R⁵ représente un atome d'hydrogène ou un groupement -CH₂COOH,
- lorsque R⁵ est relié à un atome de carbone appartenant au radical cyclique X alors R⁵ représente un groupement hydroxy -OH, un groupement -CH(R^{5b})OH, un groupement amino -NH₂, un groupement carboxylique -COOH, un atome d'halogène ou un groupement -CN.

Préférentiellement, lorsque X = X₁ alors R³ est différent d'un atome d'halogène et lorsque X = X₂ alors R⁵ représente un atome d'hydrogène ou un radical alkyle en C₁-C₃, linéaire ou ramifié, éventuellement substitué par un ou plusieurs atomes d'halogène.

Préférentiellement encore, R³ et R⁵ sont différents.

Encore plus préférentiellement, R³ représente un atome d'hydrogène ou un groupement (CHR⁶)n-(Z)ₒ-(CHR,⁶)ₚ-R⁷.

Selon un mode de réalisation, R³ représente un atome d'hydrogène ou un groupement (CHR⁶)ₙ-(Z)ₒ-(CHR'⁶)ₚ-R⁷ et R⁵ représente un atome d'hydrogène ou un radical alkyle en C₁-C₃, linéaire ou ramifié, éventuellement substitué par un ou plusieurs atomes d'halogène.

De préférence, R¹¹ et R¹² sont différents d'un groupement -OH.

Selon un mode de réalisation, dans la formule (I), R³ représente un atome d'hydrogène.

Selon un mode de réalisation, dans la formule (I), R³ représente un groupement (CHR ⁶)ₙ-(Z)ₒ-(CHR'⁶)ₚ-R⁷.

Selon un mode de réalisation, dans la formule (I), les indices n, o, et p, identiques ou différents, désignent zéro.

Selon un mode de réalisation, dans la formule (I), les indices n, o, et p, identiques ou différents, désignent un entier naturel variant de 1 à 3.

Selon un mode de réalisation, dans la formule (I), les indices n et p désignent zéro et l'indice o vaut 1.

Selon un mode de réalisation, dans la formule (I), Z représente un groupe méthylène -CH₂.

Selon un mode de réalisation, dans la formule (I), Z représente un groupe divalent -O-.

Selon un mode de réalisation, dans la formule (I), Z représente un groupe divalent -NH-.

Selon un mode de réalisation, dans la formule (I), R³ représente un groupement Z-R⁷, avec Z ayant la signification précédemment décrite.

Selon un mode de réalisation particulier, dans la formule (I), R³ représente un groupement -CH₂-R⁷.

Selon un mode de réalisation particulier, dans la formule (I), R³ représente un groupement -O-R⁷

Selon un mode de réalisation particulier, dans la formule (I), R³ représente un groupement -NH-R⁷.

Selon un mode de réalisation, dans la formule (I), R⁷ représente un radical hétérocyclique choisi parmi les hétérocycles suivants : 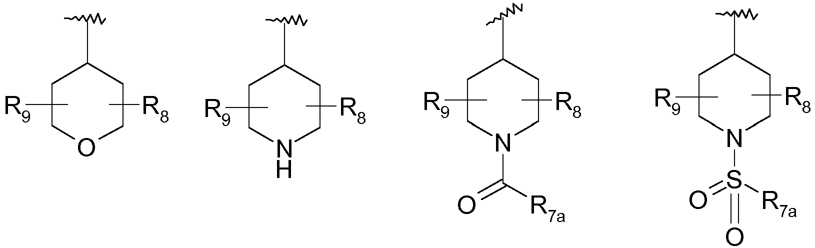 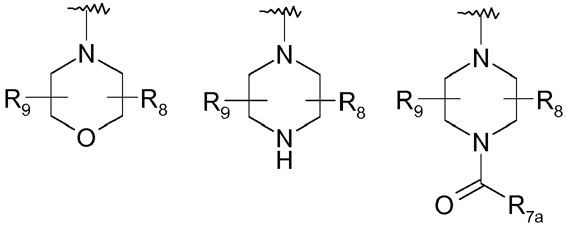 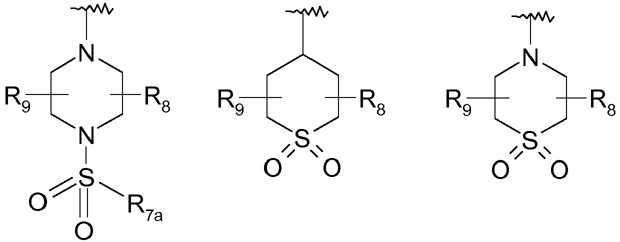 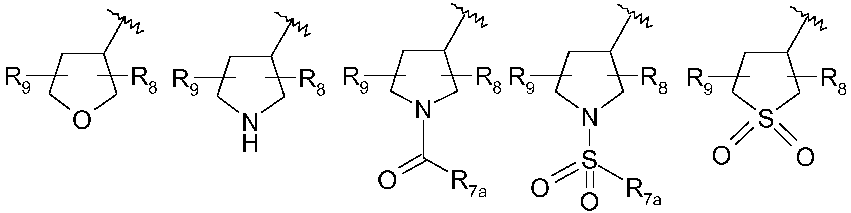 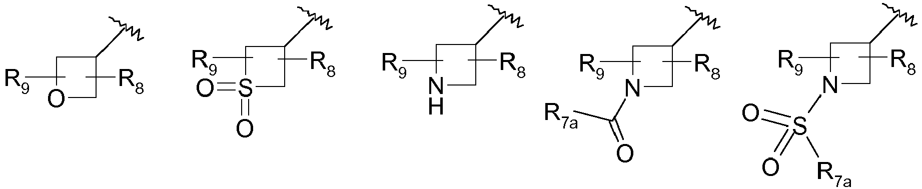 dans lesquels :
- R₇ₐ représente un radical alkyle, linéaire ou ramifié, en C₁-C₃, un radical alcoxy, linéaire ou ramifié, en C₁-C₃ ou un radical amino en N(R^{8a})(R^{8b}),
- R^{8a} et R^{8b}, identiques ou différents, désignent un atome d'hydrogène, un radical alkyle, linéaire ou ramifié, en C₁-C₃ ou un radical cyclopropyle,
- R₈ et R₉, identiques ou différents, représentent un atome d'hydrogène, un radical alkyle, linéaire ou ramifié, en C₁-C₃, un groupement hydroxy -OH, une fonction carbonyle =O, un radical hydroxyalkyle en C₁ (-CH₂OH), un groupe amino NH₂,
- R₈ et R₉ peuvent former ensemble avec les atomes de carbone auxquels ils sont attachés un cycle carbocyclique comportant de 5 à 7 chaînons.

Selon un mode de réalisation, dans la formule (I), R⁷ représente un radical aromatique ou hétéroaromatique choisi parmi : 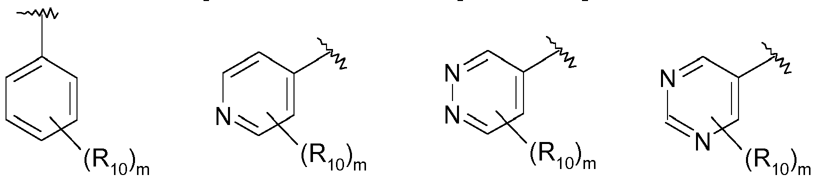 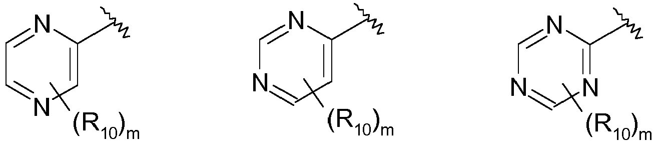 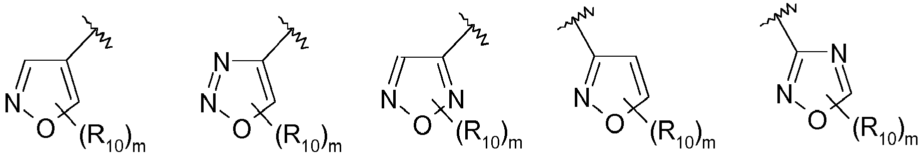 dans lesquels :
- R₁₀ représente un atome d'hydrogène ou un atome d'halogène, un groupement alkyle en C₁-C₃, linéaire ou ramifié, éventuellement substitué par un ou plusieurs atomes d'halogène ; un groupement alcoxy en C₁-C₃, un groupement amino -NR¹¹R¹², un groupement -COR¹¹, un groupement -COOR¹¹, un groupement amido - CONR¹¹R¹², un groupement -SOR¹¹, un groupement -SO₂R¹¹, un groupement -NHCOR¹¹, un groupement -NHCOOR¹¹, un groupement - SO₂NR¹¹R¹² ou un groupement -CN ; R¹¹ et R¹², identiques ou différents, représentant un atome d'hydrogène ou un radical alkyle, linéaire ou ramifié, en C₁-C₃ éventuellement substitué par un ou plusieurs atomes d'halogène,
- m désigne zéro ou un entier naturel allant de 1 à 3.

De préférence, R¹¹ et R¹² sont différents d'un groupement -OH.

Préférentiellement, R⁷ représente un radical aromatique ou hétéroaromatique tel que défini précédemment éventuellement substitué par un ou plusieurs groupements méthyle -CH₃, un ou plusieurs groupements méthoxy -OCH₃, un ou plusieurs groupements hydroxy - OH, un ou plusieurs groupements amino -NH₂, un ou plusieurs groupements -CH₂OH, un ou plusieurs groupements cyano -CN, un ou plusieurs atomes d'halogène ou une ou plusieurs fonctions carbonyles.

Selon un mode de réalisation, l'indice m vaut zéro.

Selon un mode de réalisation, l'indice m désigne un entier naturel allant de 1 à 3.

Préférentiellement, l'indice m vaut 1.

Selon un mode de réalisation, dans la formule (I), chacun des éléments Y¹, Y², Y³, Y⁴ et Y⁵ correspond à un groupement -CR² avec R² ayant la même signification que celle décrite précédemment.

Selon un mode de réalisation, dans la formule (I), chacun des éléments Y¹, Y², Y³, Y⁴ et Y⁵ correspond à un groupement -CR² avec R² représentant un atome d'hydrogène.

Selon un mode de réalisation, dans la formule (I), chacun des éléments Y¹, Y², Y³, Y⁴ et Y⁵ correspond à un groupement -CR² avec R² représentant un radical alkyle, linéaire ou ramifié en C₁-C₅.

Selon un mode de réalisation, dans la formule (I), chacun des éléments Q¹, Q² et Q³ représente un groupement -CR^{2a} avec R^{2a} ayant la même signification que celle décrite précédemment.

Selon un mode de réalisation, dans la formule (I), chacun des éléments Q¹, Q² et Q³ représente un groupement -CR^{2a} avec R^{2a} représentant un atome d'hydrogène.

Selon un mode de réalisation, dans la formule (I), Q¹ et Q² représentent un groupement -CR^{2a} avec R^{2a} représentant un atome d'hydrogène et Q³ représente un groupement -CR^{2a} avec R^{2a} représentant un radical alkyle, linéaire ou ramifié, en C₁-C₅.

Selon un mode de réalisation, dans la formule (I), R¹ représente un radical alkyle, linéaire ou ramifié, en C₃-C₅, de préférence un radical alkyle ramifié en C₃-C₅, plus préférentiellement ramifié en C₄.

Selon un mode de réalisation, dans la formule (I), R¹ représente un radical cycloalkyle en C₃-C₅, de préférence cyclopropyle.

Selon un mode de réalisation, dans la formule (I), R¹ représente un radical alkényle, linéaire ou ramifié, en C₂-C₅.

Selon un mode de réalisation, dans la formule (I), R¹ représente un radical CH₂-cycloalkyle en C₃-C₅.

Selon un mode de réalisation, dans la formule (I), R¹ représente un radical hétérocycloalkyle en C₄-C₅.

Selon un mode de réalisation, dans la formule (I), R¹ représente un radical CH₂-hétérocycloalkyle en C₄-C₆, en particulier un radical CH₂-hétérocycloalkyle en C₄-C₅.

Préférentiellement, R¹ représente un radical alkyle, linéaire ou ramifié, en C₃-C₅ ou un radical CH₂-hétérocycloalkyle en C₄-C₅.

Selon un mode de réalisation, R⁵ représente un atome d'hydrogène.

De préférence, le ou les composés de formule (I) est ou sont choisis parmi le ou les composés de formule (II), leurs sels d'addition pharmaceutiquement acceptables, leurs hydrates et/ou leurs solvates :

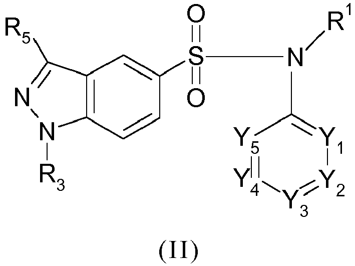

Formule (II) dans laquelle R¹, R³, R⁵ et Y¹ à Y⁵ ont les mêmes significations que dans la formule (I) précédemment décrite.

Préférentiellement, R³ représente un groupement (CHR⁶)ₙ-(Z)ₒ-(CHR'⁶)ₚ-R⁷ avec R⁶, Z, R'⁶, R⁷ et les indices n, o et p ayant les mêmes significations que celles indiquées précédemment.

Préférentiellement encore, R³ représente un groupement CH₂-R⁷ avec R⁷ représentant un radical hétérocyclique non cationique.

De préférence, le ou les composés de formule (I) est ou sont choisis parmi le ou les composés de formule (III), leurs sels d'addition pharmaceutiquement acceptables, leurs hydrates et/ou leurs solvates :

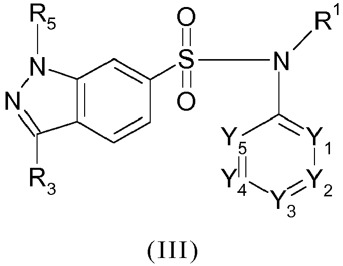

Formule (III) dans laquelle R¹, R³, R⁵ et Y¹ à Y⁵ ont les mêmes significations que dans la formule (I) précédemment décrite.

Préférentiellement, dans la formule (III), R⁵ représente un atome d'hydrogène ou un radical alkyle en C₁-C₃ linéaire ou ramifié éventuellement substitué par un ou plusieurs atomes d'halogène.

Les composés de formule (I) peuvent se présenter sous la forme de sels pharmaceutiquement acceptables. Des exemples de sels pharmaceutiquement acceptables sont décrits dans Berge et al., 1977, «sels pharmaceutiquement acceptables», J. Pharm. Sci., Vol. 66, pp 1 - 19.

En particulier, lorsque que les composés de formule selon l'invention se présentent sous la forme de sels alors l'électroneutralité desdits composés est assurée par un contre ion cationique Y externe pouvant être organique ou minérale.

Y peut être choisi parmi les cations inorganiques appropriés tels que les ions de métaux alcalins, notamment Na⁺, K+, les ions métaux alcalino-terreux, notamment Ca²⁺, Mg²⁺, ou encore d'autres cations tels que l'ion aluminium Al³⁺.

Y peut être choisi parmi les cations organiques appropriés tels que l'ion ammonium NH₄⁺, les ions ammonium substitués tels que NH₃R+, NHR₂ , NR₄⁺ avec R représentant un radical alkyle en C₁-C₄.

En particulier, les ions ammonium substitués sont ceux choisis parmi les dérivés de l'éthylamine, la diéthylamine, la dicyclohexylamine, la triéthylamine, la butylamine, l'étylènediamine, l'éthanolamine, la diéthanolamine, la pipérazine, la benzylamine, la phénylbenzylamine, la choline, la meglumine, et trométhamine, les acides aminés tels que la lysine et l'arginine.

Un exemple d'un ion ammonium quaternaire peut être l'ion N⁺ (CH₃)₄.

Le ou les composés selon l'invention peuvent se présenter sous la forme de leurs solvates.

Au sens de la présente invention, le terme « solvate » signifie un complexe de soluté (c'est-à-dire le composé selon l'invention ou le sel dudit composé) et de solvant.

Si le solvant est l'eau alors le solvate peut être commodément considéré comme un hydrate, par exemple, un semi-hydrate, un monohydrate, un dihydrate, un trihydrate, etc.

Par exemple, les solvates et/ou hydrates peuvent être obtenus directement à la fin du processus de synthèse, le composé cible étant isolé sous la forme d'un hydrate, par exemple un monohydrate ou hémi-hydrate, ou sous la forme d'un solvate du solvant de réaction et/ou du solvant de purification.

Sauf indication contraire, toute référence à un composé selon l'invention inclut également le solvate ou l'hydrate du composé correspondant.

Des procédures typiques pour la préparation et l'identification des hydrates et des solvates sont bien connus de l'homme du métier, voir par exemple, pages 202-209 de KJ Guillory, « Génération of Polymorphs, Hydrates, Solvates, and Amorphous Solids » dans Polymorphism in Pharmaceutical Solids, édition. Harry G. Britain, Vol. 95, Marcel Dekker, Inc., New York, 1999.

Les hydrates et les solvates peuvent être isolés et caractérisés par des méthodes connues dans l'art telles que l'analyse thermogravimétrique (TGA), la spectroscopie TGA-masse, la spectroscopie TGA-infrarouge, la diffraction de poudres aux rayons X, le titrage de Karl Fisher, la diffraction haute résolution aux rayons X et analogue.

De préférence, le ou les composés de formule (Ia) sont choisis parmi les composés tels que décrits dans les tableaux ci-après, ainsi que leurs sels d'addition pharmaceutiquement acceptable, leurs hydrates et/ou leurs solvates :

**Tableau 1 :**

| | | IC50 hRORg | IC50 hCD4/IL17 |
|---|---|---|---|
| 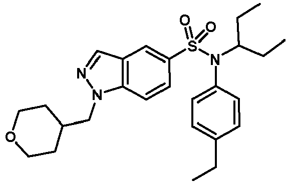 | Acide 1-(tétrahydro-pyran-4-ylméthyl)-1H-indazole-5-sulfonique (4-éthyl-phényl)-(1-éthyl-propyl)-amide | B | B |
| | Composé 1 | | |
| 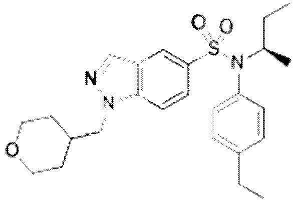 | Acide 1-(tetrahydro-pyran-4-ylmethyl)-1H-indazole-5-sulfonique ((R)-sec-butyl)-(4-ethyl-phenyl)-amide | B | ND |
| | Composé 2 | | |
| 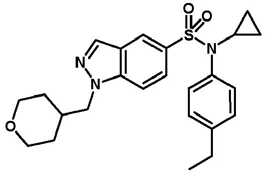 | Acide 1-(tétrahydro-pyran-4-ylméthyl)-1H-indazole-5-sulfonique cyclopropyl-(4-éthyl-phényl)-amide | C | ND |
| | Composé 3 | | |
| 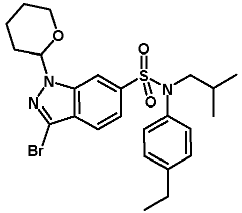 | Acide 3-bromo-1-(tétrahydro-pyran-2-yl)-1H-indazole-6-sulfonique (4-éthyl-phényl)-isobutyl-amide | C | ND |
| | Composé 4 | | |
| 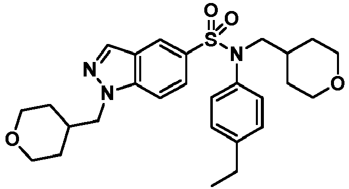 | Acide 1-(Tétrahydro-pyran-4-ylméthyl)-1H-indazole-5-sulfonique (4-éthyl-phényl)-(tétrahydro-pyran-4-ylméthyl)-amide | C | N D |
| | Composé 5 | | |
| 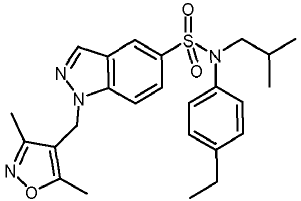 | 1-((3,5-diméthylisoxazol-4-yl)méthyl)-N-(4-éthylphényl)-N-isobutyl-1H-indazole-5-sulfonamide | B | ND |
| | Composé 6 | | |
| 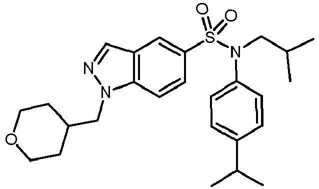 | Acide 1-(tétrahydro-pyran-4-ylméthyl)-1H-indazole-5-sulfonique butyl-(4-isopropyl-phényl)-amide | B | ND |
| | Composé 7 | | |
| 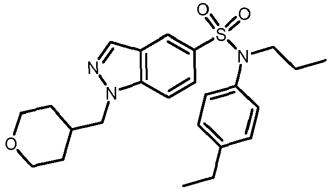 | Acide 1-(tétrahydro-pyran-4-ylmethyl)-1H-indazole-5-sulfonique (4-éthyl-phényl)-propyl | C | ND |
| | Composé 8 | | |
| 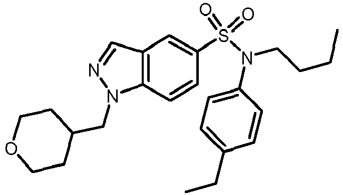 | Acide 1-(tétrahydro-pyran-4-ylmethyl)-1H-indazole-5-sulfonique butyl-(4-éthyl-phényl)-amide | C | ND |
| | Composé 9 | | |
| 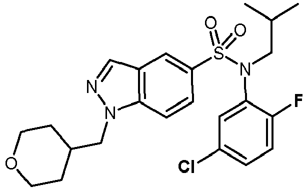 | Acide 1-(tétrahydro-pyran-4-ylméthyl)-1H-indazole-5-sulfonique (5-chloro-2-fluoro-phényl)-isobutyl-amide | C | ND |
| | Composé 10 | | |
| 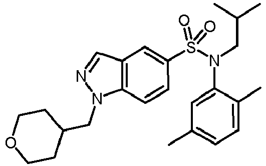 | Acide 1-(tétrahydro-pyran-4-ylmethyl)-1H-indazole-5-sulfonique (2,5-diméthyl-phényl)-isobutyl-amide | B | ND |
| | Composé 11 | | |
| 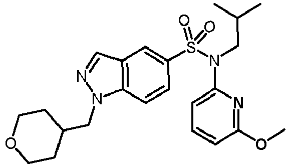 | Acide 1-(Tétrahydro-pyran-4-ylméthyl)-1H-indazole-5-sulfonique (3-méthoxy-pyridin-2-yl)-isobutyl-amide | C | ND |
| | Composé 12 | | |
| 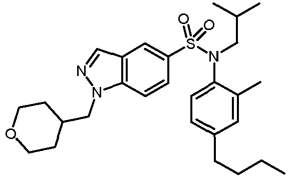 | Acide 1-(tétrahydro-pyran-4-ylméthyl)-1H-indazole-5-sulfonique (4-butyl-2-méthyl-phényl)-isobutyl-amide | B | ND |
| | Composé 13 | | |
| 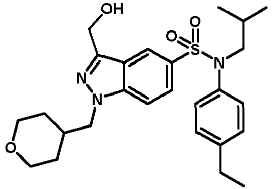 | N-(4-éthylphényl)-N-isobutyl-1-((tétrahydro-2H-pyran-4-yl)méthyl)-1H-indazole-5-sulfonamide | C | ND |
| | Composé 14 | | |
| 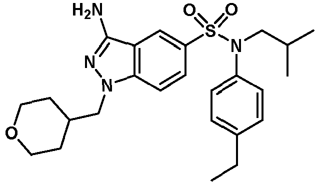 | Acide 3-amino-1H-indazole-5-sulfonique (4-éthyl-phényl)-isobutyl-amide | C | ND |
| | Composé 15 | | |
| 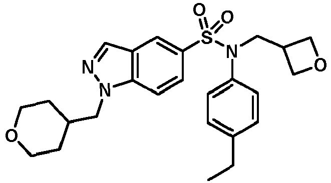 | Acide 1-(tétrahydro-pyran-4-ylméthyl)-1H-indazole-5-sulfonique (4-éthyl-phényl)-oxetan-3-ylmethyl-amide | C | ND |
| | Composé 16 | | |
| 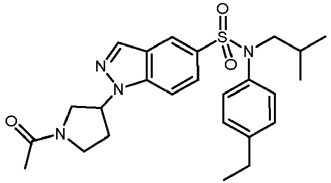 | Acide 1-(1-acétyl-pyrrolidin-3-yl)-1H-indazole-5-sulfonique (4-éthyl-phényl)-isobutyl-amide | B | ND |
| | Composé 17 | | |
| 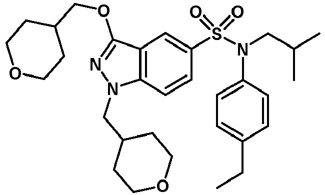 | Acide 3-(tétrahydro-pyran-4-ylméthoxy)-1-(tétrahydro-pyran-4-ylméthyl)-1H-indazole-5-sulfonique (4-éthyl-phényl)-isobutyl-amide | C | ND |
| | Composé 18 | | |
| 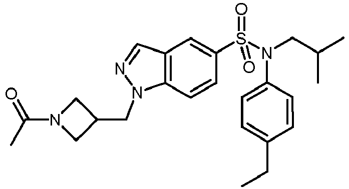 | Acide 1-(3,5-diméthyl-isoxazol-4-ylméthyl)-1H-indazole-5-sulfonique (4-éthyl-phényl)-isobutyl-amide | C | ND |
| | Composé 19 | | |
| 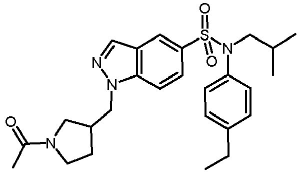 | 1-((1-acétylpyrrolidin-3-yl)méthyl)-N-(4-éthylphényl)-N-isobutyl-1H-indazole-5-sulfonamide | C | ND |
| | Composé 20 | | |
| 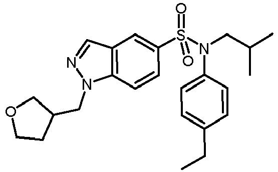 | N-(4-éthylphényl)-N-isobutyl-1-((tétrahydro-furan-3-yl)méthyl)-1H-indazole-5-sulfonamide | C | ND |
| | Composé 21 | | |
| 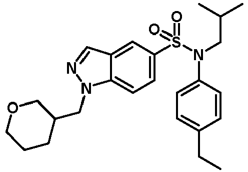 | N-(4-éthylphényl)-N-isobutyl-1-((tétrahydro-2H-pyran-3-yl)méthyl)-1H-indazole-5-sulfonamide | C | ND |
| | Composé 22 | | |
| 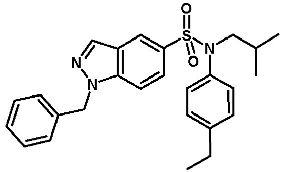 | 1-benzyl-N-(4-éthylphényl)-N-isobutyl-1H-indazole-5-sulfonamide | C | ND |
| | Composé 23 | | |
| 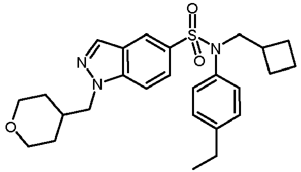 | N-(cyclobutylméthyl)-N-(4-éthylphényl)-1-((tétrahydro-2H-pyran-4-yl)méthyl)-1H-indazole-5-sulfonamide | B | ND |
| | Composé 24 | | |
| 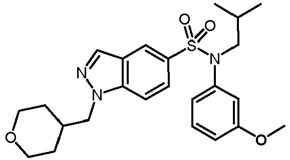 | Acide 1-(tétrahydro-pyran-4-ylméthyl)-1H-indazole-5-sulfonique (3-méthoxy-phényl)-isobutyl-amide | C | ND |
| | Composé 25 | | |
| 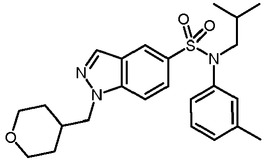 | Acide 1-(tétrahydro-pyran-4-ylméthyl)-1H-indazole-5-sulfonique (3-méthyl-phényl)-isobutyl-amide | C | ND |
| | Composé 26 | | |
| 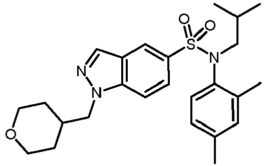 | Acide 1-(tétrahydro-pyran-4-ylméthyl)-1H-indazole-5-sulfonique (2,4-diméthyl-phényl)-isobutyl-amide | B | B |
| | Composé 27 | | |
| 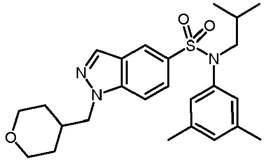 | Acide 1-(tétrahydro-pyran-4-ylmethyl)-1H-indazole-5-sulfonique (3,5-diméthyl-phényl)-isobutyl-amide | C | ND |
| | Composé 28 | | |
| 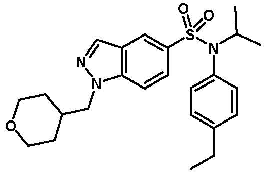 | N-(4-éthylphényl)-N-isopropyl-1-((tétrahydro-2H-pyran-4-yl)méthyl)1H-indazole-5-sulfonamide | C | ND |
| | Composé 29 | | |
| 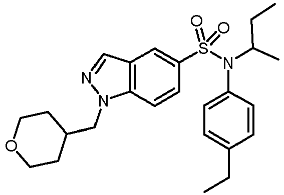 | Acide 1-(tétrahydro-pyran-4-ylméthyl)-1H-indazole-5-sulfonique sec-butyl-(4-éthyl-phényl)-amide | B | ND |
| | Composé 30 | | |
| 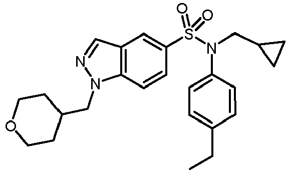 | N-(cyclopropylméthyl)-N-(4-éthylphényl)-1-((tétrahydro-2H-pyran-4-yl)méthyl)-1H-indazole-5-sulfonamide | C | ND |
| | Composé 31 | | |
| 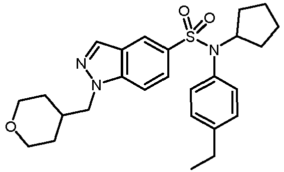 | Acide 1-(tétrahydro-pyran-4-ylméthyl)-1H-indazole-5-sulfonique cyclopentyl-(4-éthyl-phényl)-amide | A | ND |
| | Composé 32 | | |
| 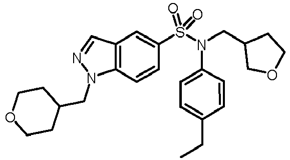 | Acide 1-(tétrahydro-pyran-4-ylméthyl)-1H-indazole-5-sulfonique (4-éthyl-phényl)-(tétrahydro-furan-3-ylméthyl)-amide | C | ND |
| | Composé 33 | | |
| 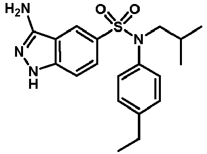 | Acide 3-amino-1H-indazole-5-sulfonique (4-éthyl-phényl)-isobutyl-amide | C | ND |
| | Composé 34 | | |
| 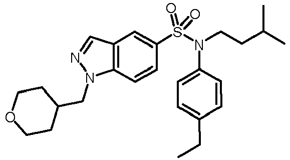 | N-(4-éthylphényl)-N-isopentyl-1-((tétrahydro-2H-pyran-4-yl)méthyl)-1H-indazole-5-sulfonamide | C | ND |
| | Composé 35 | | |
| 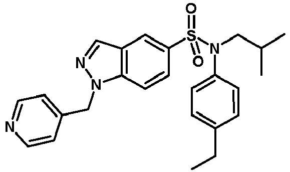 | N-(4-éthylphényl)-N-isobutyl-1-(pyridin-4-ylméthyl)-1H-indazole-5-sulfonamide | B | A |
| | Composé 36 | | |
| 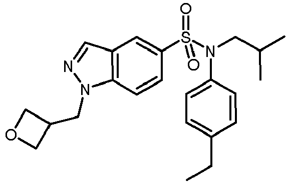 | N-(4-éthylphényl)-N-isobutyl-1-(oxetan-3-ylméthyl)-1H-indazole-5-sulfonamide | B | A |
| | Composé 37 | | |
| 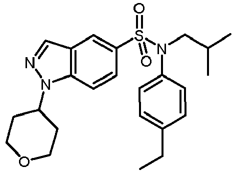 | Acide 1-(tétrahydro-pyran-4-yl)-1H-indazole-5-sulfonique (4-éthyl-phényl)-isobutyl-amide | C | ND |
| | Composé 38 | | |
| 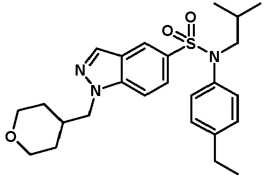 | N-(4-éthylphényl)-N-isobutyl-1-((tétrahydro-2H-pyran-4-yl)méthyl)-1H-indazole-5-sulfonamide | B | B |
| | Composé 39 | | |

**Tableau 2 :**

| | | IC50 hRORg | IC50 hCD4/IL17 |
|---|---|---|---|
| 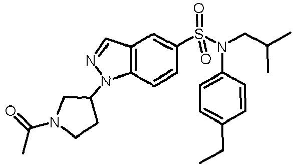 | Acide 1-(1-acétyl-pyrrolidin-3 -yl)-1H-indazole-5-sulfonique (4-éthyl-phényl)-isobutyl-amide | C | ND |
| | Composé 40 | | |
| 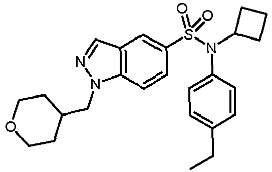 | Acide 1-(tétrahydro-pyran-4-ylméthyl)-1H-indazole-5-sulfonique cyclobutyl-(4-éthyl-phényl)-amide | C | ND |
| | Composé 41 | | |
| 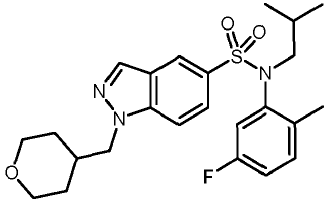 | Acide 1-(tétrahydro-pyran-4-ylméthyl)-1H-indazole-5-sulfonique (5-fluoro-2-méthyl-phényl)-isobutyl-amide | B | ND |
| | Composé 42 | | |
| 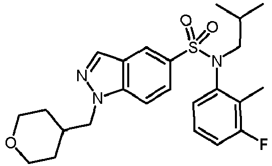 | Acide 1-(tétrahydro-pyran-4-ylméthyl)-2H-indazole-5-sulfonique (3-fluoro-2-méthyl-phényl)-isobutyl-amide | B | ND |
| | Composé 43 | | |
| 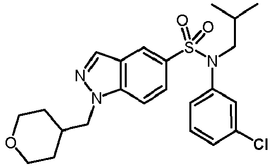 | Acide 1-(tétrahydro-pyran-4-ylmethyl)-1H-indazole-5-sulfonique (5-chloro-phényl)-isobutyl-amide | C | ND |
| | Composé 44 | | |
| 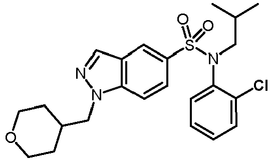 | Acide 1-(tétrahydro-pyran-4-ylméthyl)-1H-indazole-5-sulfonique (2-chloro-phenyl)-isobutyl-amide | C | ND |
| | Composé 45 | | |
| 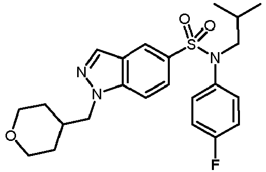 | Acide 1-(tétrahydro-pyran-4-ylméthyl)-1H-indazole-5-sulfonique (4-fluoro-phényl)-isobutyl-amide | C | ND |
| | Composé 46 | | |
| 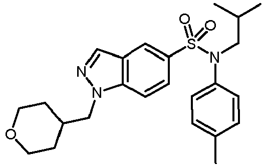 | Acide 1-(tétrahydro-pyran-4-ylméthyl)-1H-indazole-5-sulfonique isobutyl-p-tolyl-amide | C | ND |
| | Composé 47 | | |
| 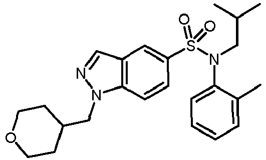 | Acide 1-(tétrahydro-pyran-4-ylméthyl)-1H-indazole-5-sulfonique isobutyl-o-tolyl-amide | C | ND |
| | Composé 48 | | |
| 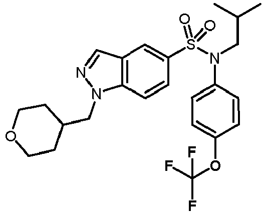 | Acide 1-(tétrahydro-pyran-4-ylméthyl)-1H-indazole-5-sulfonique (4-trifluorométhoxy-phényl)-isobutyl-amide | C | ND |
| | Composé 49 | | |
| 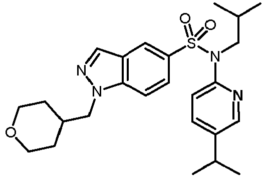 | Acide 1-(tétrahydro-pyran-4-ylméthyl)-1H-indazole-5-sulfonique isobutyl-(5-isopropyl-pyridin-2-yl)-amide | C | ND |
| | Composé 50 | | |
| 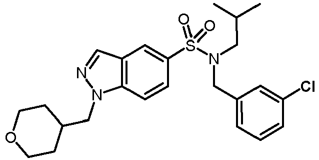 | Acide 1-(tétrahydro-pyran-4-ylméthyl)-1H-indazole-5-sulfonique (3-chloro-benzyl)-isobutyl-amide | C | ND |
| | Composé 51 | | |
| 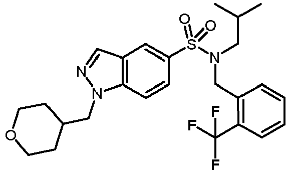 | Acide 1-(tétrahydro-pyran-4-ylméthyl)-1H-indazole-5-sulfonique isobutyl-(2-trifluorométhyl-benzyl)-amide | B | ND |
| | Composé 52 | | |
| 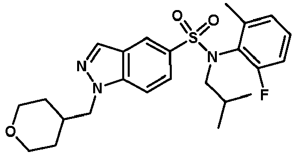 | Acide 1-(tétrahydro-pyran-4-ylméthyl)-1H-indazole-5-sulfonique (2-fluoro-6-méthyl-phényl)-isobutyl-amide | C | ND |
| | Composé 53 | | |
| 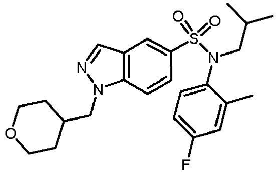 | Acide 1-(tétrahydro-pyran-4-ylméthyl)-1H-indazole-5-sulfonique (4-fluoro-2-méthyl-phényl)-isobutyl-amide | B | ND |
| | Composé 54 | | |
| 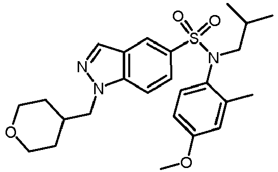 | Acide 1-(tétrahydro-pyran-4-ylméthyl)-1H-indazole-5-sulfonique isobutyl-(4-methoxy-2-methyl-phenyl)-amide | B | ND |
| | Composé 55 | | |
| 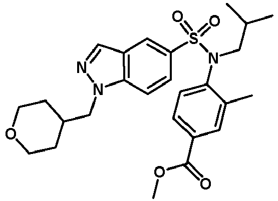 | Ester de l'acide méthyl 4-{Isobutyl-[1-(tetrahydro-pyran-4-ylmethyl)-1H-indazole-5-sulfonyl]-amino}-3-methyl-benzoïque | B | A |
| | Composé 56 | | |
| 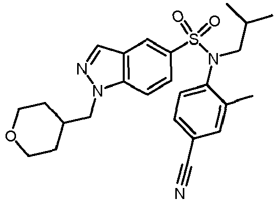 | Acide 1-(Tétrahydro-pyran-4-ylméthyl)-1H-indazole-5-sulfonique (4-cyano-2-méthyl-phényl)-isobutyl-amide | B | ND |
| | Composé 57 | | |
| 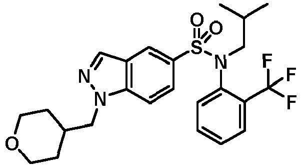 | Acide 1-(tétrahydro-pyran-4-ylméthyl)-1H-indazole-5-sulfonique isobutyl-(2-trifluorométhyl-phenyl)-amide | B | ND |
| | Composé 58 | | |
| 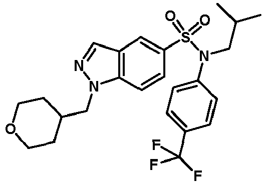 | Acide 1-(tétrahydro-pyran-4-ylméthyl)-1H-indazole-5-sulfonique isobutyl-(4-trifluorométhyl-phenyl)-amide | C | ND |
| | Composé 59 | | |
| 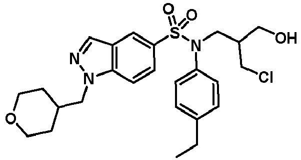 | Acide 1-(tétrahydro-pyran-4-ylmethyl)-1H-indazole-5-sulfonique (3-chloro-2-hydroxymethyl-propyl)-(4-éthyl-phényl)-amide | C | ND |
| | Composé 60 | | |
| 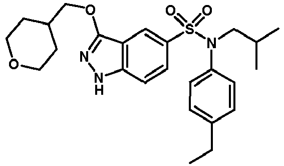 | Acide 3-(tétrahydro-pyran-4-ylméthoxy)-1H-indazole-5-sulfonique (4-éthyl-phényl)-isobutyl-amide | C | ND |
| | Composé 61 | | |
| 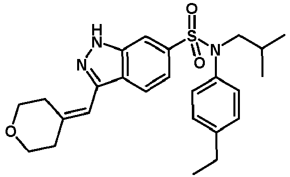 | Acide 3-(tétrahydro-pyran-4-ylideneméthyl)-1H-indazole-6-sulfonique (4-éthyl-phényl)-isobutyl-amide | A | ND |
| | Composé 62 | | |
| 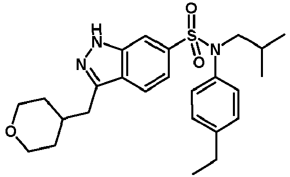 | Acide 3-(tétrahydro-pyran-4-ylméthyl)-1H-indazole-6-sulfonique (4-éthyl-phényl)-isobutyl-amide | A | A |
| | Composé 63 | | |
| 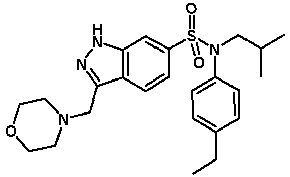 | Acide 3-morpholin-4-ylmethyl-1H-indazole-6-sulfonique (4-éthyl-phényl)-isobutyl-amide | A | A |
| | Composé 64 | | |
| 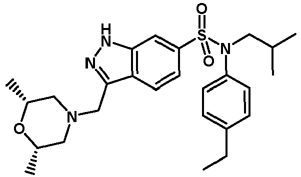 | Acide 3-((cis)-2,6-Dimethyl-morpholin-4-ylmethyl)-1H-indazole-6-sulfonique (4-ethyl-phenyl)-isobutyl-amide | C | ND |
| | Composé 65 | | |
| 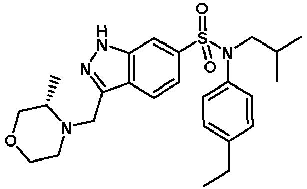 | Acide 3-((S)-3-méthyl-morpholin-4-ylméthyl)-1H-indazole-6-sulfonique (4-éthyl-phényl)-isobutyl-amide | B | ND |
| | Composé 66 | | |
| 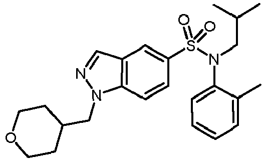 | Acide 1-(tétrahydro-pyran-4-ylméthyl)-1H-indazole-5-sulfonique isobutyl-o-tolyl-amide | C | ND |
| | Composé 69 | | |
| 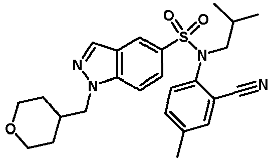 | Acide 1-(tétrahydro-pyran-4-ylméthyl)-1H-indazole-5-sulfonique (2-cyano-4-méthyl-phényl)-isobutyl-amide | B | ND |
| | Composé 70 | | |
| 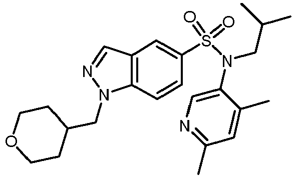 | Acide 1-(tetrahydro-pyran-4-ylmethyl)-1H-indazole-5-sulfonique (4,6-diméthyl-pyridin-3-yl)-isobutyl-amide | C | C |
| | Composé 71 | | |
| 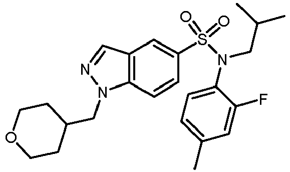 | Acide 1-(tétrahydro-pyran-4-ylméthyl)-1H-indazole-5-sulfonique (2-fluoro-4-méthyl-phényl)-isobutyl-amide | ND | ND |
| | Composé 73 | | |
| 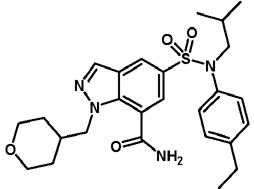 | Acide 5-[(4-Ethyl-phenyl)-isobutyl-sulfamoyl]-l-(tetrahydro-pyran-4-ylmethyl)-1H-indazole-7-carboxylique amide | ND | ND |
| | Composé 75 | | |

| | | | |
|---|---|---|---|
| ND : non déterminé ; A : IC50 <100 nM.; B : IC50 = 100nM-1µM; C : IC50 > 1µM | | | |

Dans les tableaux décrits ci-avant, les concentrations inhibitrices médianes IC₅₀ pour les composés appartenant à la formule (I) selon l'invention ont été données selon les modèles suivants :

### Transactivation GAL4-RORγ

Le modèle de transactivation RORy a été développé à partir de la lignée HG5LN qui est une lignée HeLa exprimant stablement un gène reporter luciférase contrôlé par un pentamère du domaine de reconnaissance GAL4 de levure et d'un promoteur β-globine. La lignée HG5LN a été transfectée stablement par le DNA-binding domain (DBD) (ou domaine de liaison à l'ADN) de GAL4 fusionné au ligand-binding domain (LBD) ROR gamma. Les molécules inhibant l'activité constitutive ROR gamma réduisent l'expression de la luciférase induisant ainsi une baisse de la luminescence émise.

Les cellules sont ensemencées en plaques 384 puits (5 000 cellules dans 45µL/puits de milieu de culture contenant 10% de sérum de veau foetal) et incubées pendant 4 heures à 37°C, 5% CO₂. 5µL des molécules à tester (composés décrits dans les tableaux décrits ci-avant) sont ensuite ajoutés à chaque puits et les plaques sont incubées pendant 18 heures à une température de 37°C sous 5% de CO₂. 20µL du substrat de la luciférase (Promega) sont ajoutés à chaque puits et la luminescence émise est lue par un lecteur de microplaques.

Les unités de luminescence ('RLU') sont normalisées par des contrôles positifs ('POS' contenant une concentration saturante de benzenesulfonamide, N-(2,2,2-trifluoroéthyl)-*N*-[4-[2,2,2-trifluoro-1-hydroxy-1-(trifluorométhyl)éthyl]phényl]) et des contrôles négatifs ('NEG' contenant du DMSO) : % inhibition=((RLU-NEG)*100)/(POS-NEG). Les IC50 sont calculées à partir d'un modèle logistique à 4 paramètres à l'aide du logiciel XLFit (IDBS).

### Sécrétion IL-17A

Ce modèle permet de mesurer l'effet d'inhibiteurs sur la sécrétion d'IL-17A par des cellules CD4+. Les cellules sont des CD4+ congelées (STEMCELL, # 70026), isolées de sang humain périphérique et activées par les anticorps anti-CD3 et anti-CD28. La quantité d'IL-17A sécrété est mesurée par la technologie TR-FRET (kit HTRF® Human Interleukin 17A (Cisbio, #64H17PEC)).

Les cellules sont décongelées rapidement, resuspendues dans leur milieu de culture (RPMI 10% SVF inactivé) supplémenté avec des anticorps anti-CD28 solubles et ensemencées (100 000 cellules/puits) dans des plaques 96 puits préalablement coatées avec des anticorps anti-CD3. Les cellules sont ensuite traitées par les gammes des inhibiteurs à tester (de 1000nM à 0.05nM, 0.1% DMSO). Après 4 jours d'incubation, le signal HTRF est mesuré à l'aide d'un lecteur de microplaque (λexcitation=337nm, λemission=620/665nm). Les ratios obtenus (665/620) sont normalisés par rapport au contrôle positif (cellules activées par anti-CD3 et anti-CD28, 0.1% DMSO). Les IC₅₀ sont calculées à partir d'un modèle logistique à 4 paramètres à l'aide du logiciel XLFit (IDBS).

Préférentiellement, les composés de formule (I) selon l'invention sont choisis parmi les composés suivants :

**Tableau 3 :**

| | |
|---|---|
| 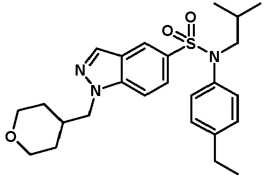 | N-(4-éthylphényl)-N-isobutyl-1-((tétrahydro-2H-pyran-4-yl)méthyl)-1H-indazole-5-sulfonamide |
| | Composé 39 |
| 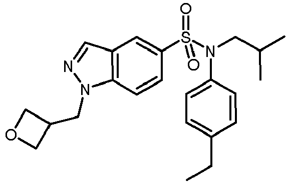 | N-(4-éthylphényl)-N-isobutyl-1-(oxetan-3-ylméthyl)-1H-indazole-5-sulfonamide |
| | Composé 37 |
| 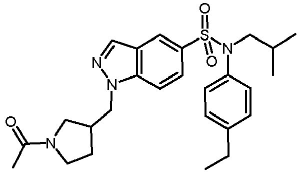 | 1-((1-acétylpyrrolidin-3-yl)méthyl)-N-(4-éthylphényl)-N-isobutyl-1H-indazole-5-sulfonamide |
| | Composé 20 |
| 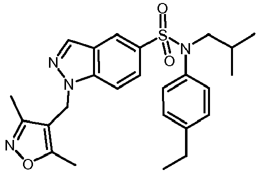 | 1-((3,5-diméthylisoxazol-4-yl)méthyl)-N-(4-éthylphényl)-N-isobutyl-1H-indazole-5-sulfonamide |
| | Composé 24 |
| 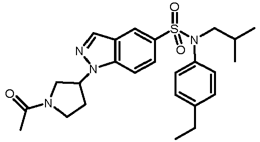 | Acide 1-(1-acétyl-pyrrolidin-3-yl)-1H-indazole-5-sulfonique (4-éthyl-phényl)-isobutyl-amide |
| | Composé 40 |
| 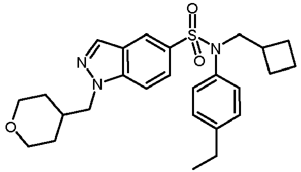 | N-(cyclobutylméthyl)-N-(4-éthylphényl)-1-((tétrahydro-2H-pyran-4-yl)méthyl)-1H-indazole-5-sulfonamide |
| | Composé 24 |
| 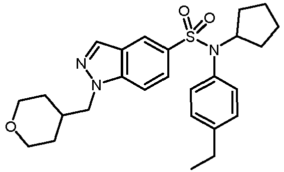 | Acide 1-(tétrahydro-pyran-4-ylméthyl)-1H-indazole-5-sulfonique cyclopentyl-(4-éthyl-phényl)-amide |
| | Composé 32 |
| 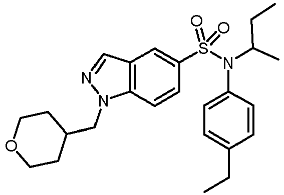 | Acide 1-(tétrahydro-pyran-4-ylméthyl)-1H-indazole-5-sulfonique sec-butyl-(4-éthyl-phényl)-amide |
| | Composé 30 |
| 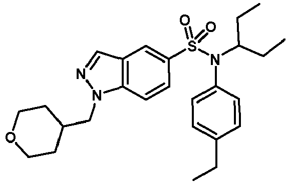 | Acide 1-(Tétrahydro-pyran-4-ylméthyl)-1H-indazole-5-sulfonique (4-éthyl-phényl)-(1-éthyl-propyl)-amide |
| | Composé 1 |
| 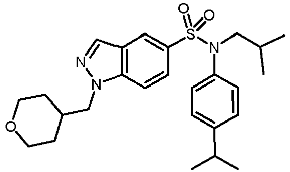 | Acide 1-(tétrahydro-pyran-4-ylméthyl)-1H-indazole-5-sulfonique butyl-(4-isopropyl-phényl)-amide |
| | Composé 7 |
| 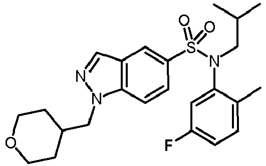 | Acide 1-(tétrahydro-pyran-4-ylméthyl)-1H-indazole-5-sulfonique (5-fluoro-2-méthyl-phényl)-isobutyl-amide |
| | Composé 42 |
| 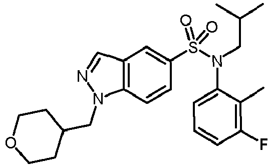 | Acide 1-(tétrahydro-pyran-4-ylméthyl)-2H-indazole-5-sulfonique (3-fluoro-2-méthyl-phényl)-isobutyl-amide |
| | Composé 43 |
| 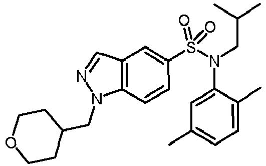 | Acide 1-(tétrahydro-pyran-4-ylmethyl)-1H-indazole-5-sulfonique (2,5-diméthyl-phényl)-isobutyl-amide |
| | Composé 11 |
| 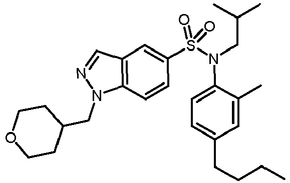 | Acide 1-(tétrahydro-pyran-4-ylméthyl)-1H-indazole-5-sulfonique (4-butyl-2-méthyl-phényl)-isobutyl-amide |
| | Composé 13 |
| 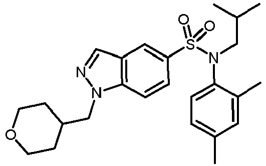 | Acide 1-(tétrahydro-pyran-4-ylméthyl)-1H-indazole-5-sulfonique (2,4-diméthyl-phényl)-isobutyl-amide |
| | Composé 27 |
| 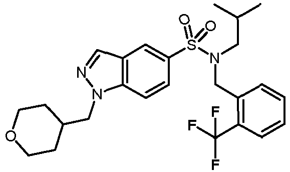 | Acide 1-(tétrahydro-pyran-4-ylméthyl)-1H-indazole-5-sulfonique isobutyl-(2-trifluorométhyl-benzyl)-amide |
| | Composé 52 |
| 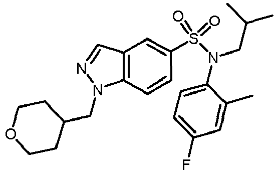 | Acide 1-(tétrahydro-pyran-4-ylméthyl)-1H-indazole-5-sulfonique (4-fluoro-2-méthyl-phényl)-isobutyl-amide |
| | Composé 54 |
| 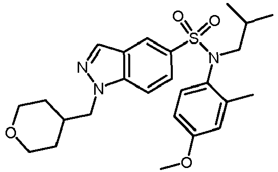 | Acide 1-(tétrahydro-pyran-4-ylméthyl)-1H-indazole-5-sulfonique isobutyl-(4-methoxy-2-methyl-phenyl)-amide |
| | Composé 55 |
| 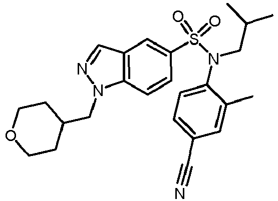 | Acide 1-(Tétrahydro-pyran-4-ylméthyl)-1H-indazole-5-sulfonique (4-cyano-2-méthyl-phényl)-isobutyl-amide |
| | Composé 57 |
| 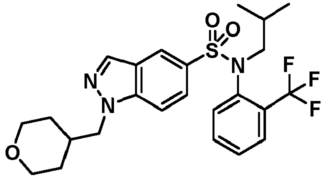 | Acide 1-(tétrahydro-pyran-4-ylméthyl)-1H-indazole-5-sulfonique isobutyl-(2-trifluorométhyl-phenyl)-amide |
| | Composé 58 |
| 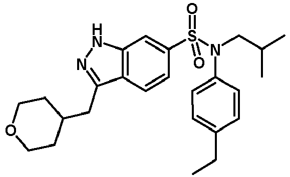 | Acide 3-(tétrahydro-pyran-4-ylméthyl)-1H-indazole-6-sulfonique (4-éthyl-phényl)-isobutyl-amide |
| | Composé 63 |
| 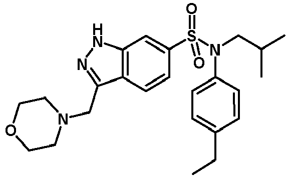 | Acide 3-morpholin-4-ylmethyl-1H-indazole-6-sulfonique (4-éthyl-phényl)-isobutyl-amide |
| | Composé 64 |
| 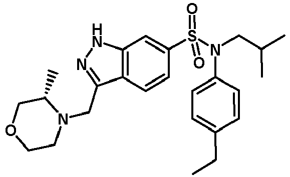 | Acide 3-((S)-3-méthyl-morpholin-4-ylméthyl)-1H-indazole-6-sulfonique (4-éthyl-phényl)-isobutyl-amide |
| | Composé 66 |

En particulier, parmi les composés de formule (II), les composés 1, 2, 7, 11, 21, 24, 27, 30, 32, 36, 37, 39, 40, 42, 43, 52, 54, 55, 57 et 58 sont préférés.

Parmi les composés de formule (III), les composés 63, 64 et 66 sont préférés.

L'invention concerne également le ou les composés tels que décrits précédemment en tant que médicament et cosmétique.

De préférence, l'invention concerne également le ou les composés tels que décrits précédemment en tant que médicament.

En effet, les composés selon l'invention présentent des propriétés pharmacologiques intéressantes étant donné que lesdits composés modulent, c'est-à-dire inhibent, l'activité du récepteur RORyt.

Ainsi ces propriétés rendent le ou les composés de formule (I) telle que décrite précédemment utilisables en tant que médicament dans le traitement des maladies médiées par le récepteur RORyt.

De préférence, le ou les composés selon l'invention sont utilisés dans le traitement des désordres inflammatoires et/ou des maladies auto-immunes médiées par le récepteur RORγt.

Plus préférentiellement, le ou les composés selon l'invention, de préférence ceux choisis parmi les composés répondant aux formules (II) et (III), sont utilisés dans le traitement de l'acné, le psoriasis et/ou la dermatite atopique.

Selon un autre mode de réalisation, les composés selon l'invention sont utilisés pour le traitement cosmétique de la peau.

Comme indiqué ci-avant, la présente invention est aussi relative à une composition pharmaceutique comprenant, dans un milieu pharmaceutiquement acceptable, un ou plusieurs composés de formule (I) telle que définie précédemment, leurs sels d'addition pharmaceutiquement acceptables, leurs hydrates et/ou leurs solvates.

De préférence, la composition pharmaceutique comprend un ou plusieurs composés choisis parmi les composés de formule (II) et (III) telles que définies précédemment, leurs sels d'addition pharmaceutiquement acceptables, leurs hydrates et/ou leurs solvates.

Plus préférentiellement, la composition pharmaceutique comprend un ou plusieurs composés de formule (I) choisis parmi les composés (1) à (75) définis précédemment.

Encore plus préférentiellement, la composition pharmaceutique comprend un ou plusieurs composés de formule (I) choisis parmi les composés 1, 2, 7, 11, 21, 24, 27, 30, 32, 36, 37, 39, 40, 42, 43, 52, 54, 55, 57, 58, 63, 64 et 66.

L'administration de la composition pharmaceutique selon l'invention telle que décrite précédemment peut être effectuée par voie orale ou topique.

De préférence, la composition pharmaceutique est conditionnée sous une forme convenant à une application par voie topique.

Par voie orale, la composition, peut se présenter sous forme de comprimés, de gélules, de dragées, de sirops, de suspensions, de solutions, de poudres, de granulés, d'émulsions, de suspensions de microsphères ou nanosphères ou de vésicules lipidiques ou polymériques permettant une libération contrôlée.

Par voie topique, la composition pharmaceutique selon l'invention est plus particulièrement destinée au traitement de la peau et des muqueuses et peut se présenter sous forme liquide, pâteuse, ou solide, et plus particulièrement sous forme d'onguents, de crèmes, de laits, de pommades, de poudres, de tampons imbibés, de syndets, de solutions, de gels, de sprays, de mousses, de suspensions, de sticks, de shampoings, ou de bases lavantes. Elle peut également se présenter sous forme de suspensions de microsphères ou nanosphères ou de vésicules lipidiques ou polymériques ou de patches polymériques ou gélifiés permettant une libération contrôlée.

La composition pharmaceutique est utilisée pour le traitement des désordres inflammatoires et/ou les maladies auto-immunes médiées par le récepteur RORγt.

Plus préférentiellement, la composition pharmaceutique est utilisée dans le traitement de l'acné et/ou le psoriasis.

L'invention concerne aussi un procédé de traitement des maladies médiées par le récepteur RORγt comprenant l'administration, notamment par voie topique ou orale, d'une quantité thérapeutiquement efficace de la composition pharmaceutique telle que définie ci-avant à un patient.

De préférence, la composition pharmaceutique est appliquée par voie topique.

Conformément à un autre mode de réalisation, la présente invention a aussi pour objet un ou des composés répondant à la formule (II) telle que définie précédemment, leurs sels d'addition pharmaceutiquement acceptables, leurs hydrates et/ou leurs solvates :

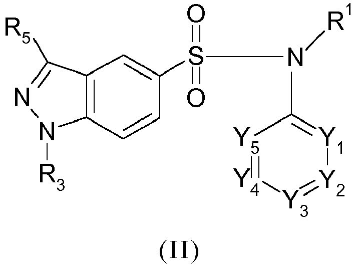

Formule (II) dans laquelle R¹, R³, R⁵ et Y¹ à Y⁵ ont les mêmes significations que dans la formule (Ia) précédemment décrite.

De préférence, R³ représente un groupement (CHR⁶)ₙ-(Z)ₒ-(CHR'⁶)ₚ-R⁷ avec R⁷ représentant un radical hétérocyclique non cationique, un radical cycloalkyle non cationique ou un radical aromatique ou hétéroaromatique non cationique tel que défini précédemment.

Parmi les composés de formule (II), les composés 1, 2, 7, 11, 21, 24, 27, 30, 32, 36, 37, 39, 40, 42, 43, 52, 54, 55, 57 et 58 sont préférés.

Selon ce mode de réalisation, l'invention porte aussi sur le ou les composés de formule (II) en tant que médicament et cosmétique.

En particulier, l'invention concerne le ou les composés de formule (II) pour leur utilisation dans le traitement des désordres inflammatoires et/ou les maladies auto-immunes médiées par le récepteur RORyt.

Préférentiellement, l'invention a pour objet le ou les composés de formule (II) pour leur utilisation dans le traitement de l'acné.

En variante, l'invention a également pour objet le ou les composés de formule (II) pour leur utilisation dans le traitement du psoriasis.

Alternativement, le ou les composés de formule (II) selon l'invention sont utilisés pour le traitement cosmétique de la peau.

De plus, l'invention est aussi relative à une composition pharmaceutique comprenant, dans un milieu pharmaceutiquement acceptable, un ou plusieurs composés de formule (II) telle que définie précédemment, leurs sels d'addition pharmaceutiquement acceptables, leurs hydrates et/ou leurs solvates.

La composition pharmaceutique est utilisée pour le traitement des désordres inflammatoires et/ou les maladies auto-immunes médiées par le récepteur RORγt.

Conformément à un autre mode de réalisation, la présente invention a aussi pour objet un ou des composés répondant à la formule (III) telle que définie précédemment, leurs sels d'addition pharmaceutiquement acceptables, leurs hydrates et/ou leurs solvates :

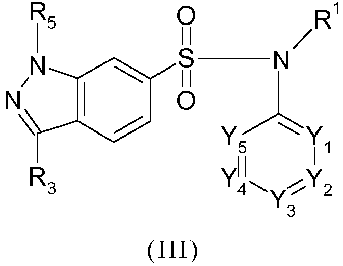

Formule (III) dans laquelle R¹, R³, R⁵ et Y¹ à Y⁵ ont les mêmes significations que dans la formule (I) précédemment décrite.

De préférence, R³ représente un groupement (CHR⁶)ₙ-(Z)ₒ-(CHR'⁶)ₚ-R⁷ avec R⁷ représentant un radical hétérocyclique non cationique, un radical cycloalkyle non cationique ou un radical aromatique ou hétéroaromatique non cationique tel que défini précédemment.

Parmi les composés de formule (III), les composés 63, 64 et 66 sont préférés.

Selon ce mode de réalisation, l'invention porte aussi sur le ou les composés de formule (III) en tant que médicament et cosmétique.

En particulier, l'invention concerne le ou les composés de formule (III) pour leur utilisation dans le traitement des désordres inflammatoires et/ou les maladies auto-immunes médiées par le récepteur RORγt.

Préférentiellement, l'invention a pour objet le ou les composés de formule (III) pour leur utilisation dans le traitement de l'acné.

En variante, l'invention a également pour objet le ou les composés de formule (III) pour leur utilisation dans le traitement du psoriasis.

Alternativement, le ou les composés de formule (III) selon l'invention sont utilisés pour le traitement cosmétique de la peau.

De plus, l'invention est aussi relative à une composition pharmaceutique comprenant, dans un milieu pharmaceutiquement acceptable, un ou plusieurs composés de formule (III) telle que définie précédemment, leurs sels d'addition pharmaceutiquement acceptables, leurs hydrates et/ou leurs solvates.

La composition pharmaceutique est utilisée pour le traitement des désordres inflammatoires et/ou les maladies auto-immunes médiées par le récepteur RORγt.

Les exemples suivants servent à illustrer l'invention sans toutefois présenter un caractère limitatif.

### EXEMPLES

La méthode standard LCMS pour l'analyse des produits est la suivante : colonne standard BEH C₁₈ (150*2.1mm, 1.8 µm) solvant eau/acétonitrile 0.1% acide formique.

Les purifications par HPLC préparative ont été réalisées sur colonne C₁₈, avec pour éluant : 85% d'acétonitrile dans eau/0.1% d'acide formique.

### Partie I : Synthèse des sulfonamides bicycliques à partir du schéma réactionnel 1

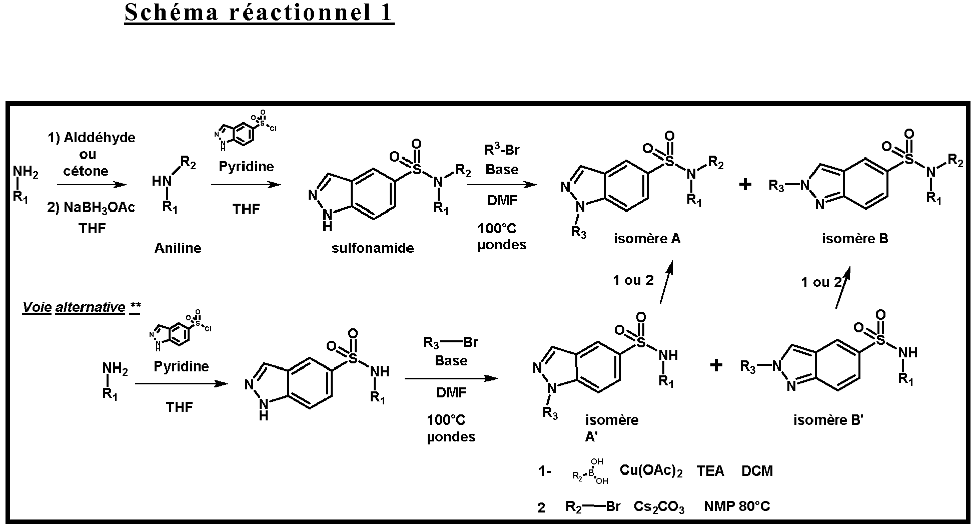

### Exemple 1 : Synthèse du N-(4-éthylphényl)-N-isobutyl-1-((tetrahydro-2H-pyran-4-yl)méthyl)-1H-indazole-5-sulfonamide

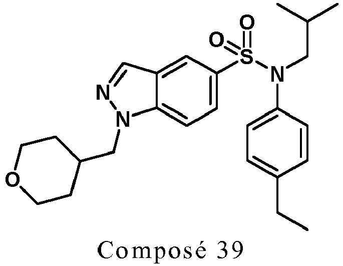

### 1. Synthèse de l'intermédiaire 1.1

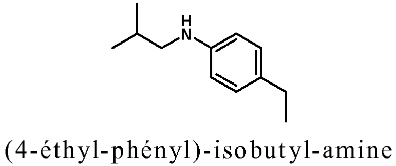

A la 4-éthylaniline (9.48 ml; 0.08 mol) est ajouté l'isobutyraldehyde (6.33 ml; 0.07 mol.) dans le tétrahydrofurane (100ml). Le mélange est agité pendant 2 heures à température ambiante. Puis le triacétoxy-borohydrure de sodium (22.04 g; 0.10 mol) est ajouté. Le mélange est agité une nuit à température ambiante, additionné d'eau (100ml) et extrait à l'acétate d'éthyle (2 x 100ml). Les phases organiques sont rassemblées, lavées à la saumure (100ml), séchées (Na₂SO₄) et concentrées.

Le produit brut est chromatographié sur gel de silice (éluant heptane/dichlorométhane de 0 à 50% de dichlorométhane). La (4-éthyl-phényl)-isobutyl-amine est obtenu sous la forme d'une huile orange avec une RMN¹H conforme.
MS : [M+H] = 179

### 2. Synthèse de l'intermédiaire 1.2

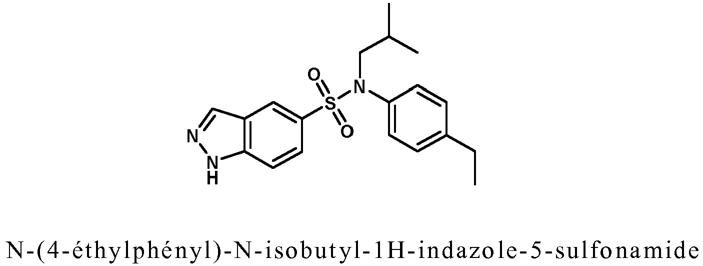

Le chlorure de 1H-indazole-5-sulfonyle (502 mg; 2.20 mmol) est additionné sur la (4-éthyl-phényl)-iso-butylamine (300 mg; 1.69 mmol) et la pyridine (820 µl; 10.15 mmol) dans le tétrahydrofurane (6ml). Le milieu réactionnel est agité pendant 7 heures à température ambiante, hydrolysé, extrait à l'acétate d'éthyle. Les phases organiques sont rassemblées, lavées avec de la saumure, séchées (Na₂SO₄) et concentrées.

Le produit brut est chromatographié sur gel de silice (éluant heptane acétate d'éthyle 20 à 50% d'acétate d'éthyle). Le N-(4-éthylphényl)-N-isobutyl-1H-indazole-5-sulfonamide (357 mg; 59%) est obtenu sous la forme d'un solide crème avec une RMN¹H conforme.
MS : [M+H] = 358

### 3. Synthèse du composé 39 selon l'invention

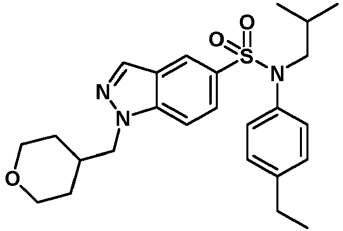

Sur le N-(4-éthylphényl)-N-isobutyl-1H-indazole-5-sulfonamide (150 mg; 0.42 mmol) et le césium carbonate (137 mg; 0.42 mmol) dans le N,N-diméthylformamide (12 ml) est ajouté le 4-(bromo-méthyl)-tétrahydropyrane (90 mg; 0.50 mmol). Le milieu réactionnel est agité pendant 30 minutes sous micro-onde à une température de 100°C, hydrolysé et extrait à l'acétate d'éthyle. Les phases organiques sont rassemblées, lavées à la saumure, séchées (Na₂SO₄) et concentrées.

Le produit brut est chromatographié sur gel de silice (éluant : heptane -acétate d'ethyle:40% d'acétate d'éthyle). Le N-(4-éthylphényl)-N-isobutyl-1-((tetrahydro-2H-pyran-4-yl)méthyl)-1H-indazole-5-sulfonamide (97 mg; 50%) est obtenu sous la forme d'une poudre blanche.
¹H NMR (400 MHz, DMSO-d6) δ 0.85 (d, J = 6.6 Hz, 7H), 1.17 (t, J = 7.6 Hz, 4H), 1.22 - 1.53 (m, 6H), 2.16 - 2.31 (m, 1H), 2.59 (q, J = 7.6 Hz, 2H), 3.15 - 3.44 (m, 6H), 3.83 (ddd, J = 11.5, 4.4, 2.0 Hz, 3H), 4.39 (d, J = 7.2 Hz, 3H), 6.92 - 7.05 (m, 3H), 7.10 - 7.21 (m, 3H), 7.26 (dd, J = 9.1, 1.8 Hz, 1H), 7.74 (d, J = 9.3 Hz, 1H), 8.10 (d, J = 1.7 Hz, 1H), 8.62 (s, 1H).
MS : [M+H] = 456

Avec un mode opératoire analogue à celui décrit pour la synthèse de l'exemple 1, on obtient les composés du tableau ci-après :

| | | |
|---|---|---|
| **Exemple 2** | 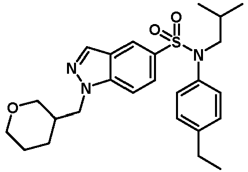 | **N-(4-éthylphényl)-N-isobutyl-1- ((tétrahydro-2H-pyran-3-yl)méthyl)-1H- indazole-5-sulfonamide** |
| | | 1H NMR (DMSO-d6) δ: 0.85 (d, J = 6.6 Hz, 6H), 1.17 (t, J = 7.6 Hz, 3H), 1.23 - 1.35 (m, 1H), 1.35 - 1.53 (m, 2H), 1.54 - 1.72 (m, 2H), 2.08 - 2.23 (m, 1H), 2.59 (q, J = 7.6 Hz, 2H), 3.21 (dd, J = 11.2, 8.9 Hz, 1H), 332 - 3.40 (m, 3H), 3.57 - 3.65 (m, 1H), 3.70 (dt, J = 10.9, 3.9 Hz, 1H), 4.31 - 4.48 (m, 2H), 6.96 (d, J = 8.3 Hz, 2H), 7.16 (d, J = 8.4 Hz, 2H), 7.45 (dd, J = 9.0, 1.8 Hz, 1H), 7.84 - 7.89 (m, 1H), 8.09 (d, J = 1.8 Hz, 1H), 8.29 (d, J = 0.8 Hz, 1H) |
| | Composé 22 | MS : [M+H] = 456 |
| **Exemple 3** | 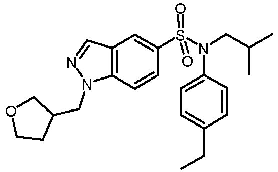 | **N-(4-éthylphényl)-N-isobutyl-1- ((tétrahydro-furan-3-yl)méthyl)-1H- indazole-5-sulfonamide** |
| | | 1H NMR (DMSO-d6) δ: 0.85 (d, J = 6.7 Hz, 6H), 1.17 (t, J = 7.6 Hz, 3H), 1.42 (hept, J = 6.8 Hz, 1H), 1.57 - 1.73 (m, 1H), 1.83 - 2.00 (m, 1H), 2.60 (q, J = 7.6 Hz, 2H), 2.81 (hept, J = 7.1 Hz, 1H), 3.31 - 3.35 (m, 2H), 3.50 (dd, J = 8.6, 5.6 Hz, 1H), 3.60 - 3.71 (m, 2H), 3.81 (td, J = 8.1, 5.5 Hz, 1H), 4.36 - 4.55 (m, 2H), 6.96 (d, J = 8.4 Hz, 2H), 7.17 (d, J = 8.3 Hz, 2H), 7.46 (dd, J = 8.8, 1.8 Hz, 1H), 7.90 (d, J = 9.0 Hz, 1H), 8.09 (d, J = 1.7 Hz, 1H), 8.30 (d, J = 0.9 Hz, 1H) |
| | Composé 21 | MS : [M+H] = 442 |
| **Exemple 4** | 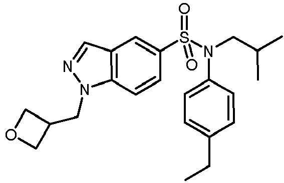 | **N-(4-éthylphényl)-N-isobutyl-1-(oxetan-3-ylmethyl)-1H-indazole-5-sulfonamide** |
| | | 1H NMR (DMSO-d6) δ: 0.85 (d, J = 6.6 Hz, 6H), 1.17 (t, J = 7.6 Hz, 3H), 1.41 (non, J = 6.8 Hz, 1H), 2.60 (q, J = 7.6 Hz, 2H), 3.32 (m,2H), 3.52 (tt, J = 7.6, 6.1 Hz, 1H), 4.49 (t, J = 6.2 Hz, 2H), 4.67 (dd, J = 7.8, 6.1 Hz, 2H), 4.77 (d, J = 7.2 Hz, 2H), 6.96 (d, J = 8.3 Hz, 2H), 7.17 (d, J = 8.3 Hz, 2H), 7.47 (dd, J = 8.9, 1.7 Hz, 1H), 7.91 (d, J = 8.9 Hz, 1H), 8.09 (d, J = 1.8 Hz, 1H), 8.29 (d, J = 0.9 Hz, 1H) |
| | Composé 37 | MS : [M+H] = 428 |
| **Exemple 5** | 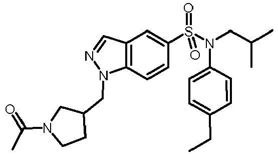 | **1-((1-acétylpyrrolidin-3-yl)méthyl)-N-(4- éthylphényl)-N-isobutyl-1H-indazole-5- sulfonamide** |
| | | 1H NMR (DMSO-d6, 80°C) δ: 0.86 (d, J = 6.6 Hz, 6H), 1.19 (t, J = 7.6 Hz, 3H), 1.44 - 1.60 (m, 1H), 1.60 - 1.83 (m, 1H), 1.83 - 2.07 (m, 4H), 2.61 (q, J = 7.6 Hz, 2H), 2.72 - 2.94 (m, 1H), 3.09 - 3.61 (m, 6H), 4.51 (d, J = 7.1 Hz, 2H), 6.99 (d, J = 8.5 Hz, 2H), 7.16 (d, J = 8.1 Hz, 2H), 7.49 (d, J = 8.7 Hz, 1H), 7.85 (d, J = 8.9 Hz, 1H), 8.10 (s, 1H), 8.28 (s, 1H) |
| | Composé 20 | MS : [M+H] = 483 |
| **Exemple 6** | 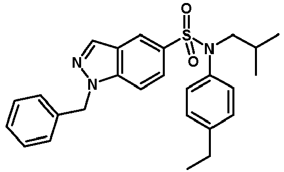 | **1-benzyl-N-(4-éthylphényl)-N-isobutyl-1H- indazole-5-sulfonamide** |
| | | 1H NMR (DMSO-d6) δ: 0.84 (d, J = 6.7 Hz, 6H), 1.16 (t, J = 7.6 Hz, 3H), 1.41 (hept, J = 6.9 Hz, 1H), 2.59 (q, J = 7.6 Hz, 2H), 3.32 (m, 2H), 5.71 (s, 2H), 6.98 (d, J = 8.4 Hz, 2H), 7.16 (d, J = 8.4 Hz, 2H), 7.25 (dd, J = 9.2, 1.8 Hz, 1H), 7.31 - 7.43 (m, 5H), 7.73 (d, J = 9.1 Hz, 1H), 8.12 (dd, J = 1.9, 0.8 Hz, 1H), 8.75 (d, J = 1.0 Hz, 1H) |
| | Composé 23 | MS : [M+H] = 448 |
| **Exemple 7** | 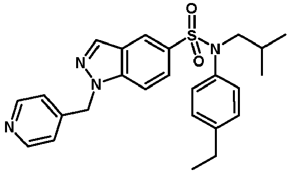 | **N-(4-éthylphényl)-N-isobutyl-1-(pyridin-4- ylmethyl)-1H-indazole-5-sulfonamide** |
| | | 1H NMR (DMSO-d6) δ: 0.85 (d, J = 6.6 Hz, 6H), 1.17 (t, J = 7.6 Hz, 3H), 1.32 - 1.48 (m, 1H), 2.59 (q, J = 7.7 Hz, 2H), 3.33 (m, 2H), 5.82 (s, 2H), 6.96 (d, J = 7.9 Hz, 2H), 7.15 (dd, J = 11.8, 6.5 Hz, 4H), 7.48 (d, J = 8.9 Hz, 1H), 7.90 (d, J = 8.9 Hz, 1H), 8.14 (s, 1H), 8.39 (s, 1H), 8.52 (d, J = 5.1 Hz, 2H) |
| | Composé 36 | MS : [M+H] = 449 |
| **Exemple 8** | 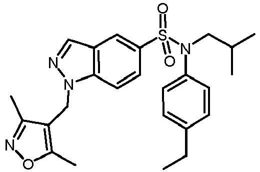 | **1-((3,5-diméthylisoxazol-4-yl)méthyl)-N- (4-éthylphényl)-N-isobutyl-1H-indazole-5- sulfonamide** |
| | | 1H NMR (DMSO-d6) δ: 0.85 (d, J = 6.6 Hz, 6H), 1.17 (t, J = 7.6 Hz, 3H), 1.41 (hept, J = 6.8 Hz, 1H), 2.11 (s, 3H), 2.44 (s, 3H), 2.59 (q, J = 7.6 Hz, 2H), 3.33 (s, 2H), 5.54 (s, 2H), 6.88 - 7.00 (m, 2H), 7.12 - 7.20 (m, 2H), 7.50 (dd, J = 8.9, 1.8 Hz, 1H), 7.88 - 8.01 (m, 1H), 8.10 (d, J = 2.0 Hz, 1H), 8.30 (d, J = 0.9 Hz, 1H) |
| | Composé 6 | MS : [M+H] = 467 |

### Exemple 9 : Synthèse de l'acide 1-((1-acétylazetidin-3-yl)méthyl)-1H-indazole-5-sulfonique (4-éthyl-phényl)-isobutyl-amide

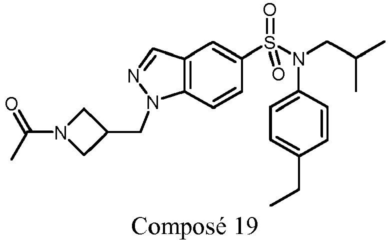

A un mélange de N-(4-ethylphenyl)-N-isobutyl-1H-indazole-5-sulfonamide (0.20 g; 0.56 mmol) et de (triphenyl-λ⁵-phosphanylidene)-acetonitrile (0.51 g; 1.68 mmol) dans du toluène anhydre (3ml) sous argon est ajouté du 1-(3-hydroxymethyl-azetidin-1-yl)-ethanone (0.27 g; 2.09 mmol) dans du toluène (1ml). Le milieu réactionnel est agité pendant 3 jours à une température de 95°C, hydrolysé et extrait à l'acétate d'éthyle. La phase organique est lavée, séchée (Na₂SO₄), filtrée et concentrée.

Le produit brut est purifié par HPLC préparative (colonne C18, éluant : acétonitrile dans eau/0.1% d'acide formique). L'acide 1-(1-acetyl-azetidin-3-ylméthyl)-1H-indazole-5-sulfonique (4-éthyl-phenyl)-isobutyl-amide (136 mg; 51%) est obtenu sous la forme d'un solide jaune pâle.
¹H NMR (DMSO-d6) δ: 0.85 (d, J = 6.6 Hz, 6H), 1.17 (t, J = 7.6 Hz, 3H), 1.42 (hept, J = 6.8 Hz, 1H), 1.73 (s, 3H), 2.59 (q, J = 7.6 Hz, 2H), 3.07 - 3.20 (m, 1H), 3.30 - 3.36 (m, 2H), 3.69 (dd, J = 9.6, 5.6 Hz, 1H), 3.89 (t, J = 9.0 Hz, 1H), 3.98 (dd, J = 8.5, 5.5 Hz, 1H), 4.18 (t, J = 8.4 Hz, 1H), 4.71 (d, J = 7.3 Hz, 2H), 6.96 (d, J = 8.4 Hz, 2H), 7.17 (d, J = 8.4 Hz, 2H), 7.48 (dd, J = 9.0, 1.7 Hz, 1H), 7.89 - 7.97 (m, 1H), 8.09 (d, J = 1.7 Hz, 1H), 8.31 (d, J = 0.9 Hz, 1H)
MS : [M+H] = 469

### Exemple 10 : Synthèse de l'acide 1-(tétrahydro-pyran-4-yl)-1H-indazole-5-sulfonique (4-éthyl-phényl)-isobutyl-amide

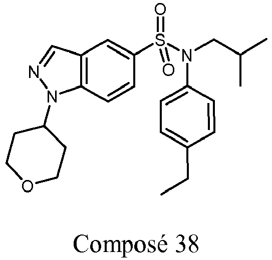

En suivant le même mode opératoire que pour l'exemple 9, l'acide 1-(tétrahydro-pyran-4-yl)-1H-indazole-5-sulfonique (4-é&thyl-phenyl)-isobutyl-amide (22 mg; 36 %) est obtenu sous la forme d'un solide blanc.
¹H NMR (DMSO-d6) δ: 0.85 (d, J = 6.7 Hz, 6H), 1.18 (t, J = 7.6 Hz, 3H), 1.41 (non, J = 6.5 Hz, 1H), 1.85 - 1.97 (m, 2H), 2.15 (qd, J = 12.3, 4.6 Hz, 2H), 2.60 (q, J = 7.6 Hz, 2H), 3.30 - 3.32 (m, 2H), 3.58 (td, J = 11.9, 2.0 Hz, 2H), 3.98 - 4.07 (m, 2H), 4.99 (td, J = 11.3, 5.7 Hz, 1H), 6.98 (d, J = 8.4 Hz, 2H), 7.18 (d, J = 8.4 Hz, 2H), 7.44 (dd, J = 8.9, 1.7 Hz, 1H), 7.94 (d, J = 8.9 Hz, 1H), 8.11 (d, J = 1.6 Hz, 1H), 8.32 (s, 1H)
MS : [M+H] = 442

### Exemple 11 : Synthèse de l'acide 1-(1-acétyl-pyrrolidin-3-yl)-1H-indazole-5-sulfonique (4-éthyl-phényl)-isobutyl-amide

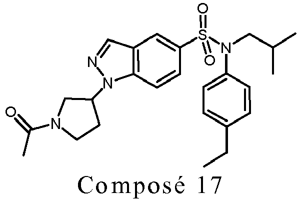

Un mélange de N-(4-éthylphényl)-N-isobutyl-1H-indazole-5-sulfonamide (0.20 g; 0.56 mmol), de carbonate de césium (0.27 g; 0.84 mmol) et de 1-(3-bromo-pyrrolidin-1-yl)-ethanone (0.13 g; 0.67 mmol) dans de la 1-méthyl-2-pyrrolidone (3ml) est agité pendant 5 heures à une température de 80°C, hydrolysé et extrait à l'acétate d'éthyle. La phase organique est lavée à l'eau, séchée (Na₂SO₄), filtrée et concentrée.

Le produit brut est purifié par HPLC préparative (colonne C18, éluant : acétonitrile dans eau/0.1% d'acide formique). L'acide 1-(1-acetyl-pyrrolidin-3-yl)-1H-indazole-5-sulfonique (4-éthyl-phényl)-isobutyl-amide (29 mg; 11%) est obtenu sous la forme d'un solide blanc.
mélange de 2 conformères: ¹H NMR (DMSO-d6) δ: 0.85 (dd, J = 6.7, 1.1 Hz, 6H), 1.18 (t, J = 7.6 Hz, 3H), 1.33 - 1.50 (m, 1H), 2.01 (s, 3H), 2.60 (q, J = 7.6 Hz, 2H), 3.34 (d, J = 2.4 Hz, 2H), 3.46 - 3.54 (m, 1H), 3.59 - 3.65 (m, 1H), 3.65 - 3.72 (m, 1H), 3.76 - 3.82 (m, 1H), 3.85 (dd, J = 12.3, 6.8 Hz, 1H), 4.05 (dd, J = 10.8, 7.0 Hz, 1H), 5.57 (ddt, J = 27.8, 11.5, 6.2 Hz, 1H), 6.90 - 7.03 (m, 2H), 7.16 - 7.21 (m, 2H), 7.49 (ddd, J = 8.8, 5.4, 1.7 Hz, 1H), 7.92 (t, J = 9.0 Hz, 1H), 8.11 (dd, J = 3.4, 1.6 Hz, 1H), 8.32 - 8.41 (m, 1H)
MS : [M+H] = 469

Avec un mode opératoire analogue à celui de l'intermédiaire 1.1 correspondant à une amination réductrice entre 1 équivalent d'aldéhyde et 1.15 équivalent d'aniline dans du tétrahydrofurane en présence de 1.45 équivalent de triacétoxyborohydrure de sodium on obtient les anilines du tableau ci-après :

| | | |
|---|---|---|
| Intermédiaire 12.1 | 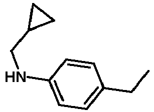 | Cyclopropylméthyl-(4-éthyl-phényl)-amine |
| | | (300 mg; 46%) obtenu sous la forme d'une huile orange avec une RMN ¹H conforme. |
| | | MS : [M+H] =176 |
| Intermédiaire 13.1 | 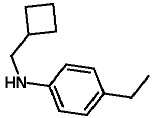 | Cyclobutylméthyl-(4-éthyl-phényl)-amine |
| | | (400 mg; 56%) obtenu sous la forme d'une huile orange avec une RMN ¹H conforme. |
| | | MS : [M+H] = 190 |
| Intermédiaire 14.1 | 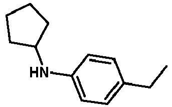 | Cyclopentylméthyl-(4-éthyl-phényl)-amine |
| | | (19.3 g; 56%) obtenu sous la forme d'une huile ambrée avec une RMN¹H conforme. |
| | | MS : [M+H] = 190 |
| Intermédiaire 15.1 | 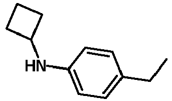 | Cyclobutylméthyl-(4-éthyl-phényl)-amine |
| | | (700 mg; 97%) obtenu sous la forme d'une huile avec une RMN ¹H conforme. |
| | | MS : [M+H] = 176 |
| Intermédiaire 16.1 | 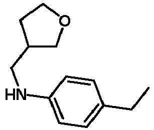 | (Tétrahydro-furan-3-ylméthyl)-(4-éthyl-phényl)-amine |
| | | (600 mg; 78%) obtenu sous la forme d'une huile orange avec une RMN ¹H conforme. |
| | | MS : [M+H] = 206 |
| Intermédiaire 17.1 | 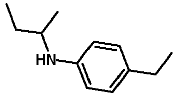 | sec-Butyl-(4-éthyl-phényl)-amine |
| | | (600 mg; 90%) obtenu sous la forme d'une huile orange avec une RMN ¹H conforme. |
| | | MS : [M+H] = 178 |
| Intermédiaire 18.1 | 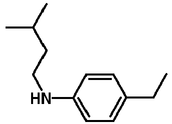 | (4-éthyl-phényl)-(3-méthyl-butyl)-amine |
| | | (250 mg; 32%) obtenu sous la forme d'une huile orange avec une RMN ¹H conforme. |
| | | MS : [M+H] = 192 |
| Intermédiaire 19.1 | 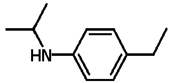 | 4-éthyl-N-isopropylaniline |
| | | (300 mg; 49%) obtenu sous la forme d'une huile orange avec une RMN ¹H conforme. |
| | | MS : [M+H] = 165 |
| Intermédiaire 20.1 | 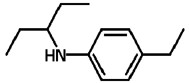 | (4-éthyl-phényl)-(1-éthyl-propyl)-amine |
| | | (700 mg; 98%) obtenu sous la forme d'une huile avec une RMN ¹H conforme. |
| | | MS : [M+H] = 192 |
| Intermédiaire 21.1 | 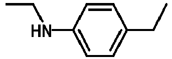 | Ethyl-(4-éthyl-phényl)-amine |
| | | Commercial |
| Intermédiaire 22.1 | 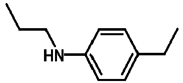 | Propyl-(4-éthyl-phényl)-amine |
| | | (450 mg; 73%) obtenu sous la forme d'une huile avec une RMN¹H conforme. |
| | | MS : [M+H] = 165 |
| Intermédiaire 23.1 | 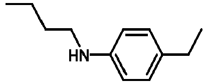 | Butyl-(4-éthyl-phényl)-amine |
| | | (650 mg; 98%) obtenu sous la forme d'une huile avec une RMN ¹H conforme. |
| | | MS : [M+H] = 178 |
| Intermédiaire 24.1 | 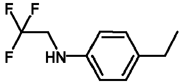 | (2,2,2-trifluoro-éthyl)-(4-éthyl-phényl)-amine |
| | | Commercial |
| Intermédiaire 25.1 | 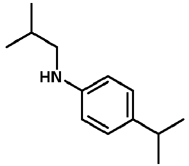 | Isobutyl-(4-isopropyl-phényl)-amine |
| | | (600 mg; 93%) obtenu sous la forme d'une huile avec une RMN ¹H conforme. |
| | | MS : [M+H] = 193 |
| Intermédiaire 26.1 | 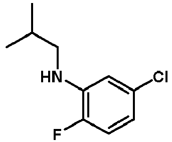 | (5-chloro-2-fluoro-phényl)-isobutyl-amine |
| | | (350 mg; 56%) obtenu sous la forme d'une huile avec une RMN ¹H conforme. |
| | | MS : [M+H] = 202 |
| Intermédiaire 27.1 | 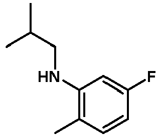 | (5-fluoro-2-methyl-phenyl)-isobutyl-amine |
| | | (802 mg; 61%) obtenu sous la forme d'une huile avec une RMN ¹H conforme. |
| | | MS : [M+H] = 182 |
| Intermédiaire 28.1 | 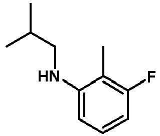 | (3-fluoro-2-méthyl-phényl)-isobutyl-amine |
| | | (408 mg; 31%) obtenu sous la forme d'une huile avec une RMN¹H conforme. |
| | | MS : [M+H] = 182 |
| Intermédiaire 29.1 | 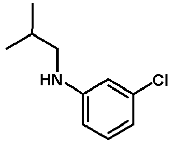 | (5-chloro-phényl)-isobutyl-amine |
| | | (655 mg; 100%) obtenu sous la forme d'une huile avec une RMN¹H conforme. |
| | | MS : [M+H] = 185 |
| Intermédiaire 30.1 | 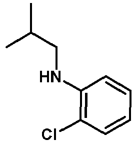 | (2-chloro-phényl)-isobutyl-amine |
| | | (655 mg; 100%) obtenu sous la forme d'une huile avec une RMN¹H conforme. |
| | | MS : [M+H] = 184 |
| Intermédiaire 31.1 | 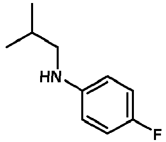 | (4-fluoro-phényl)-isobutyl-amine |
| | | (684 mg; 100%) obtenu sous la forme d'une huile avec une RMN¹H conforme. MS : [M+H] = 169 |
| Intermédiaire 32.1 | 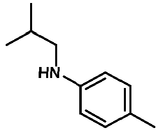 | (4-méthyl-phényl)-isobutyl-amine |
| | | (876 mg; 63%) obtenu sous la forme d'une huile incolore avec une RMN ¹H conforme. |
| | | MS : [M+H] = 164 |
| Intermédiaire 33.1 | 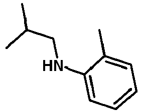 | Isobutyl-o-tolyl-amine |
| | | (972.8 mg; 70%) obtenu sous forme d'huile avec une RMN¹H conforme |
| | | MS : [M+H] =164 |
| Intermédiaire 34.1 | 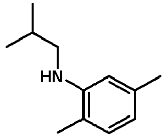 | (2,5-diméthyl-phenyl)-isobutyl-amine |
| | | (700 mg; 97%) obtenu sous la forme d'une huile avec une RMN¹H conforme |
| | | MS : [M+H] = 179 |
| Intermédiaire 35.1 | 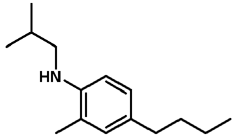 | (4-butyl-2-méthyl-phényl)-isobutyl-amine |
| | | (520 mg; 85%) obtenu sous la forme d'une huile jaune claire avec une RMN ¹H conforme |
| | | MS : [M+H] = 220 |
| Intermédiaire 36.1 | 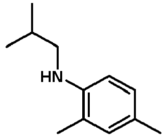 | (2,4-diméthyl-phényl)-isobutyl-amine |
| | | (1.15 g; 83%) obtenu sous la forme d'une huile avec une RMN ¹H conforme MS : |
| | | [M+H] = 179 |
| Intermédiaire 37.1 | 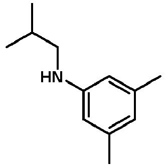 | (3,5-diméthyl-phényl)-isobutyl-amine |
| | | (600 mg; 82%) obtenu sous la forme d'une huile incolore avec une RMN ¹H conforme. |
| | | MS : [M+H] = 179 |
| Intermédiaire 38.1 | 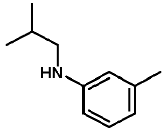 | (3 -méthyl-phényl)-isobutyl-amine |
| | | (600 mg; 79%) obtenu sous la forme d'une huile incolore avec une RMN ¹H conforme. |
| | | MS : [M+H] = 164 |
| Intermédiaire 39.1 | 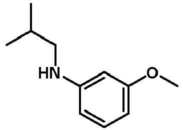 | (3 -méthoxy-phényl)-isobutyl-amine |
| | | (600 mg; 82%) obtenu sous la forme d'une huile incolore avec une RMN ¹H conforme |
| | | MS : [M+H] = 181 |
| Intermédiaire 40.1 | 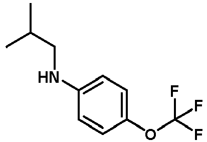 | (4-trifluoromethoxy-phényl)-isobutyl-amine |
| | | (600 mg; 100%) obtenu sous la forme d'une huile avec une RMN ¹H conforme |
| | | MS : [M+H] =235 |
| Intermédiaire 41.1 | 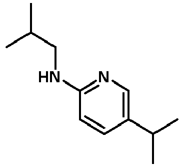 | Isobutyl-(5 -isopropyl-pyridin-2-yl)-amine |
| | | (600 mg; 93%) obtenu sous la forme d'une huile avec une RMN ¹H conforme |
| | | MS : [M+H] =193 |
| Intermédiaire 42.1 | 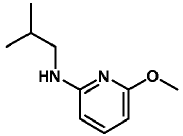 | (3-méthoxy-pyridin-2-yl)-isobutyl-amine |
| | | (450 mg; 68%) obtenu sous la forme d'une huile avec une RMN ¹H conforme |
| | | MS : [M+H] = 181 |
| Intermédiaire 43.1 | 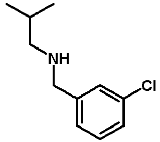 | (3-chloro-benzyl)-isobutyl-amine |
| | | (500 mg; 68%) obtenu sous la forme d'une huile avec une RMN ¹H conforme |
| | | MS : [M+H] =198 |
| Intermédiaire 44.1 | 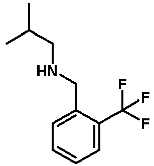 | (3-méthoxy-pyridin-2-yl)-isobutyl-amine |
| | | (600 mg; 100%) obtenu sous la forme d'une huile avec une RMN ¹H conforme |
| | | MS : [M+H] =232 |
| Intermédiaire 45.1 | 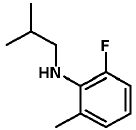 | (2-fluoro-6-méthyl-phényl)-isobutyl-amine |
| | | (560 mg; 44%) obtenu sous la forme d'une huile avec une RMN ¹H conforme |
| | | MS : [M+H] =182 |
| Intermédiaire 46.1 | 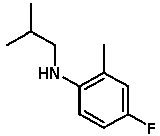 | (4-chloro-2-méthyl-phényl)-isobutyl-amine |
| | | (892 mg; 71%) obtenu sous la forme d'une huile avec une RMN ¹H conforme |
| | | MS : [M+H] =182 |
| Intermédiaire 47.1 | 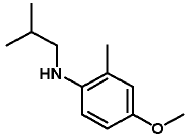 | Isobutyl-(4-méthoxy-2-méthyl-phényl)-amine |
| | | (980 mg; 77%) obtenu sous la forme d'une huile avec une RMN ¹H conforme |
| | | MS : [M+H] =193 |

Avec un mode opératoire analogue à celui de l'intermédiaire 1.2 en faisant réagir 1 équivalent d'anilines N substituées (issues du tableau ci-avant ou commerciales correspondantes) avec 1.3 équivalent de chlorure de 1H-indazole-5-sulfonyle dans du tétrahydrofurane (20V) en présence de 6 équivalents de pyridine on obtient les intermédiaires du tableau ci-dessous

| | | |
|---|---|---|
| Intermédiaire 12.2 | 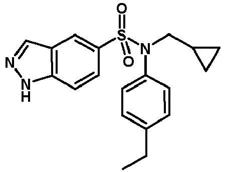 | acide 1H-indazole-5-sulfonique cyclopropylmethyl-(4-éthyl-phényl)-amide |
| | | (450 mg; 81%) est obtenue sous la forme d'une huile incolore avec une RMN ¹H conforme. |
| | | MS : [M+H] = 356 |
| Intermédiaire 13.2 | 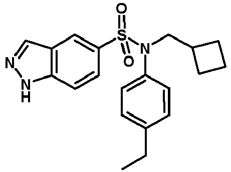 | acide 1H-indazole-5-sulfonique cyclobutylmethyl-(4-éthyl-phényl)-amide |
| | | (590 mg; 55%) obtenu sous la forme d'une huile incolore avec une RMN ¹H conforme |
| | | MS : [M+H] =370 |
| Intermédiaire 14.2 | 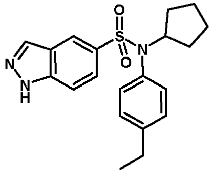 | acide 1H-indazole-5-sulfonique cyclopentyl-(4-éthyl-phényl)-amide |
| | | (250 mg; 47%) obtenu sous la forme d'une huile incolore avec une RMN ¹H conforme |
| | | MS : [M+H] =370 |
| Intermédiaire 15.2 | 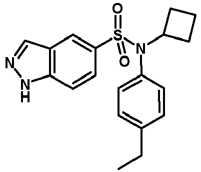 | acide 1H-indazole-5-sulfonique cyclobutyl-(4-éthyl-phényl)-amide |
| | | (80 mg; 51%) obtenu sous la forme d'une huile incolore avec une RMN ¹H conforme |
| | | MS : [M+H] =356 |
| Intermédiaire 16.2 | 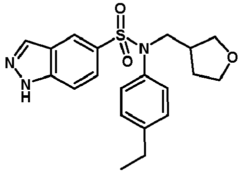 | acide 1H-indazole-5-sulfonique (4-éthyl-phenyl)-(tétrahydro-furan-3-ylméthyl)-amide |
| | | (800 mg; 78%) obtenu sous la forme d'une huile incolore avec une RMN ¹H conforme |
| | | MS : [M+H] =386 |
| Intermédiaire 17.2 | 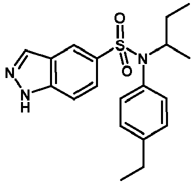 | acide 1H-indazole-5-sulfonique sec-butyl-(4-éthyl-phényl)-amide |
| | | (310 mg; 56%) obtenu sous la forme d'une huile incolore avec une RMN ¹H conforme |
| | | MS : [M+H] =358 |
| Intermédiaire 18.2 | 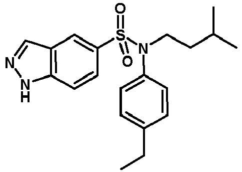 | acide 1H-indazole-5-sulfonique (4-éthyl-phényl)-(3-méthyl-butyl)-amide |
| | | (220 mg; 50%) obtenu sous la forme d'une huile incolore avec une RMN ¹H conforme |
| | | MS : [M+H] =372 |
| Intermédiaire 19.2 | 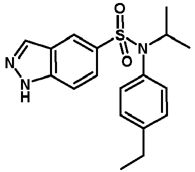 | acide 1H-indazole-5-sulfonique (4-éthyl-phényl)-isopropyl-amide |
| | | (400 mg; 70%) obtenu sous la forme d'une huile incolore avec une RMN ¹H conforme |
| | | MS : [M+H] =344 |
| Intermédiaire 20.2 | 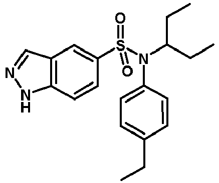 | acide 1H-indazole-5-sulfonique (4-éthyl-phenyl)-(1-ethyl-propyl)-amide |
| | | (40 mg; 25%) obtenu sous la forme d'une huile incolore avec une RMN ¹H conforme |
| | | MS : [M+H] =372 |
| Intermédiaire 21.2 | 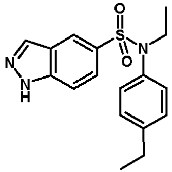 | acide 1H-indazole-5-sulfonique éthyl-(4-éthyl-phényl)-amide |
| | | (160 mg; 55%) obtenu sous la forme d'une huile incolore avec une RMN ¹H conforme |
| | | MS : [M+H] =330 |
| Intermédiaire 22.2 | 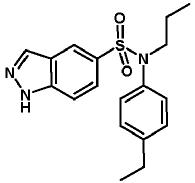 | acide 1H-indazole-5-sulfonique (4-éthyl-phényl)-propyl-amide |
| | | (400 mg; 46%) obtenu sous la forme d'une huile incolore avec une RMN ¹H conforme |
| | | MS : [M+H] =344 |
| Intermédiaire 23.2 | 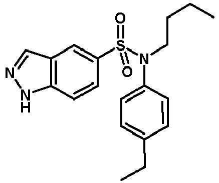 | acide 1H-indazole-5-sulfonique (4-éthyl-phényl)-butyl-amide |
| | | (800 mg; 67%) obtenu sous la forme d'une huile incolore avec une RMN ¹H conforme |
| | | MS : [M+H] =358 |
| Intermédiaire 25.2 | 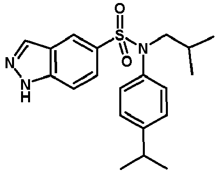 | acide 1H-indazole-5-sulfonique isobutyl-(4-isopropyl-phényl)-amide |
| | | (800 mg; 76%) obtenu sous la forme d'une huile incolore avec une RMN ¹H conforme |
| | | MS : [M+H] =372 |
| Intermédiaire 26.2 | 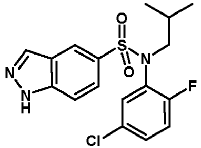 | acide 1H-indazole-5-sulfonique (5-chloro-2-fluoro-phényl)-isobutyl-amide |
| | | (120 mg; 20%) obtenu sous la forme d'une huile incolore avec une RMN ¹H conforme |
| | | MS : [M+H] =381 |
| Intermédiaire 27.2 | 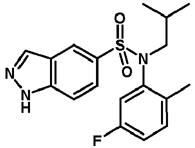 | acide 1H-indazole-5-sulfonique (5-fluoro-2-méthyl-phényl)-isobutyl-amide |
| | | (267 mg; 53%) obtenu sous la forme d'une huile incolore avec une RMN ¹H conforme |
| | | MS : [M+H] =362 |
| Intermédiaire 28.2 | 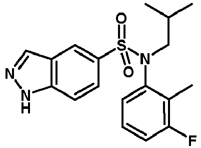 | acide 1H-indazole-5-sulfonique (3-fluoro-2-methyl-phényl)-isobutyl-amide |
| | | (137 mg; 38%) obtenu sous la forme d'une huile incolore avec une RMN ¹H conforme |
| | | MS : [M+H] =362 |
| Intermédiaire 29.2 | 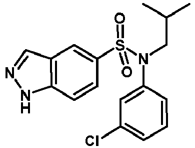 | acide 1H-indazole-5-sulfonique (5-chloro-phényl)-isobutyl-amide |
| | | (90 mg; 56%) obtenu sous la forme d'un solide blanc avec une RMN¹H conforme |
| | | MS : [M+H] =364 |
| Intermédiaire 31.2 | 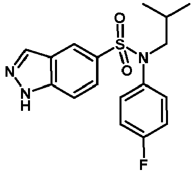 | acide 1H-indazole-5-sulfonique (4-fluoro-phényl)-isobutyl-amide |
| | | (100 mg; 66%) obtenu sous la forme d'une huile incolore avec une RMN ¹H conforme |
| | | MS : [M+H] =348 |
| Intermédiaire 32.2 | 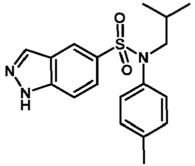 | acide 1H-indazole-5-sulfonique isobutyl-p-tolyl-amide |
| | | (770 mg; 81%) obtenu sous la forme d'une huile incolore avec une RMN¹H conforme |
| | | MS : [M+H] =344 |
| Intermédiaire 33.2 | 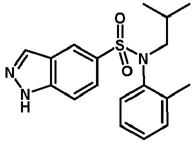 | acide 1H-indazole-5-sulfonique isobutyl-o-tolyl-amide |
| | | (481 mg; 46%) obtenu sous la forme d'un solide blanc avec une RMN¹H conforme |
| | | MS : [M+H] =344 |
| Intermédiaire 34.2 | 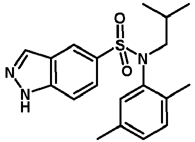 | 1H-indazole-5-sulfonique acide (2,5-diméthyl-phényl)-isobutyl-amide |
| | | (540 mg; 59%) obtenu sous la forme d'une huile incolore avec une RMN ¹H conforme |
| | | MS : [M+H] =358 |
| Intermédiaire 35.2 | 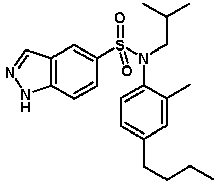 | acide 1H-indazole-5-sulfonique (4-butyl-2-méthyl-phényl)-isobutyl-amide |
| | | (650 mg; 75%) obtenu sous la forme d'une huile incolore avec une RMN ¹H conforme |
| | | MS : [M+H] =400 |
| Intermédiaire 36.2 | 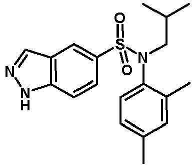 | acide 1H-indazole-5-sulfonique (2,4-diméthyl-phényl)-isobutyl-amide |
| | | (500 mg; 55%) obtenu sous la forme d'une huile incolore avec une RMN ¹H conforme |
| | | MS : [M+H] =381 |
| Intermédiaire 37.2 | 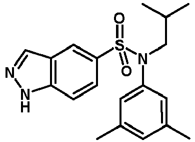 | acide 1H-indazole-5-sulfonique (3,5-diméthyl-phényl)-isobutyl-amide |
| | | (140 mg; 13%) obtenu sous la forme d'une huile incolore avec une RMN ¹H conforme |
| | | MS : [M+H] =358 |
| Intermédiaire 38.2 | 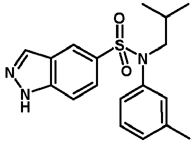 | acide 1H-indazole-5-sulfonique (3-méthyl-phényl)-isobutyl-amide |
| | | (310 mg; 27%) obtenu sous la forme d'une huile incolore avec une RMN ¹H conforme |
| | | MS : [M+H] =344 |
| Intermédiaire 39.2 | 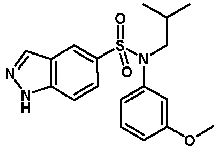 | acide 1H-indazole-5-sulfonique (3-méthoxy-phényl)-isobutyl-amide |
| | | (450 mg; 62%) obtenu sous la forme d'une huile incolore avec une RMN ¹H conforme |
| | | MS : [M+H] =360 |
| Intermédiaire 40.2 | 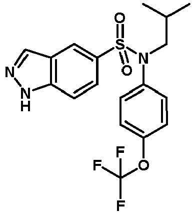 | acide 1H-indazole-5-sulfonique (4-trifluorométhoxy-phényl)-isobutyl-amide |
| | | (100 mg; 55%) obtenu sous la forme d'une huile incolore avec une RMN ¹H conforme |
| | | MS : [M+H] =414 |
| Intermédiaire 42.2 | 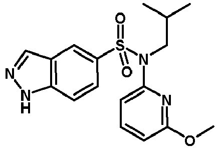 | acide 1H-indazole-5-sulfonique (3-méthoxy-pyridin-2-yl)-isobutyl-amide |
| | | (300 mg; 37%) obtenu sous la forme d'une huile incolore avec une RMN ¹H conforme |
| | | MS : [M+H] =361 |
| Intermédiaire 43.2 | 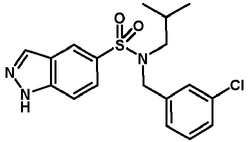 | acide 1H-indazole-5-sulfonique (3-chloro-benzyl)-isobutyl-amide |
| | | (50 mg; 30%) obtenu sous la forme d'une huile incolore avec une RMN ¹H conforme |
| | | MS : [M+H] =378 |
| Intermédiaire 44.2 | 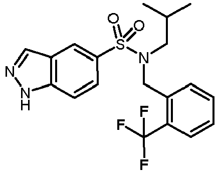 | acide 1H-indazole-5-sulfonique isobutyl-(2-trifluorométhyl-benzyl)-amide |
| | | (70 mg; 39%) obtenu sous la forme d'une huile incolore avec une RMN ¹H conforme |
| | | MS : [M+H] =412 |
| Intermédiaire 45.2 | 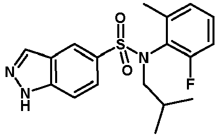 | acide 1H-indazole-5-sulfonique (2-fluoro-6-méthyl-phényl)-isobutyl-amide |
| | | (305 mg; 65%) obtenu sous la forme d'un solide blanc avec une RMN¹H conforme. |
| | | MS : [M+H] = 362 |
| Intermédiaire 46.2 | 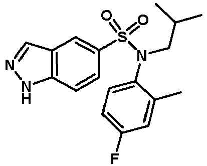 | acide 1H-indazole-5-sulfonique (4-fluoro-2-méthyl-phényl)-isobutyl-amide |
| | | (307 mg; 65%) est obtenu sous la forme d'un solide jaunâtre avec une RMN ¹H conforme. |
| | | MS : [M-H] = 360 |
| Intermédiaire 47.2 | 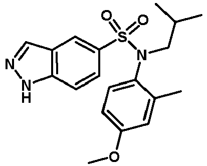 | acide 1H-indazole-5-sulfonique isobutyl-(4-methoxy-2-méthyl-phényl)-amide |
| | | (437 mg; 89%) obtenu sous la forme d'une huile incolore avec une RMN ¹H conforme. |
| | | MS : [M+H] = 374 |
| Intermédiaire 48.1 | 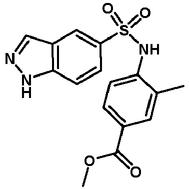 | 4-(1H-indazo le-5 -sulfonylamino)-3-méthyl-benzoate de méthyle |
| | | Obtenu à partir de l'amine commerciale (480 mg; 63%) obtenu sous forme d'un solide blanc avec une RMN¹H conforme. |
| | | MS : [M-H] = 346 |
| Intermédiaire 49.1 | 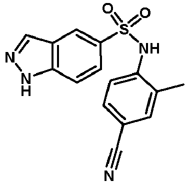 | acide 1H-indazole-5-sulfonique (4-cyano-2-méthyl-phényl)-amide |
| | | Obtenu à partir de l'amine commerciale (330 mg; 80%) obtenu sous forme d'un solide beige avec une RMN¹H conforme. |
| | | MS : [M-H] = 313 |

### 1. Synthèse de l'intermédiaire 24.2

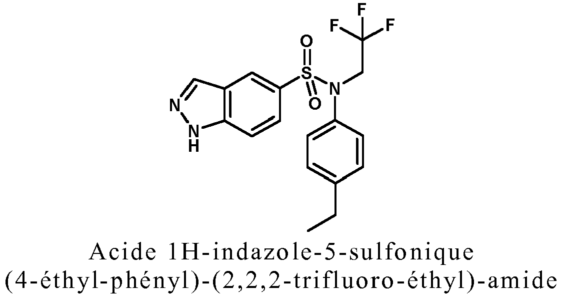

Un mélange de chlorure de 1H-indazole-5-sulfonyle (200 mg; 0.88 mmol.) de pyridine (3ml) d'iodure de potassium (7.3 mg; 0.04 mmol) de 4-diméthylaminopyridine (5.4 mg; 0.04 mmol) et N-(4-ethyl-phenyl)-N-(2,2,2-trifluoroéthyl)amine, HCl (231 mg; 0.96 mmol) est agité pendant 16 heures à une température de 100°C.

Du fluorure d'argent (I) (5.6 mg; 0.04 mmol) est ajouté au milieu réactionnel est agité pendant 3 jours à une température de 80°C.

Le produit brut est purifié par HPLC préparative (colonne C18, éluant : acétonitrile dans eau/0.1% d'acide formique). L'acide 1H-indazole-5-sulfonique (4-éthyl-phényl)-(2,2,2-trifluoro-éthyl)-amide (20mg; 6 %) est obtenu sous la forme d'une huile jaune claire.
MS : [M+H] =384

### 2. Synthèse de l'intermédiaire 30.2

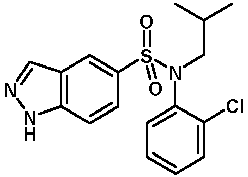

### Acide 1H-indazole-5-sulfonique (2-chloro-phényl)-isobutyl-amide

Un mélange de chlorure de 1H-indazole-5-sulfonyle (200 mg; 0.88 mmol), de pyridine (3.0 ml), d'iodure de potassium (14 mg; 0.08 mmol) de 4-diméthylaminopyridine (5.4 mg; 0.04 mmol) et de (2-chloro-phenyl)-isobutyl-amine (600 mg; 3.27 mmol) est agité pendant 3 jours à une température de 100°C.

Le produit brut est purifié par HPLC préparative (colonne C18, éluant : acétonitrile dans eau/0.1% d'acide formique). L'acide 1H-indazole-5-sulfonique (2-chloro-phényl)-isobutyl-amide (10 mg; 3%) obtenu sous la forme d'une huile jaune avec une RMN¹H conforme
MS : [M+H] =364

### 3. Synthèse de l'intermédiaire 41.2

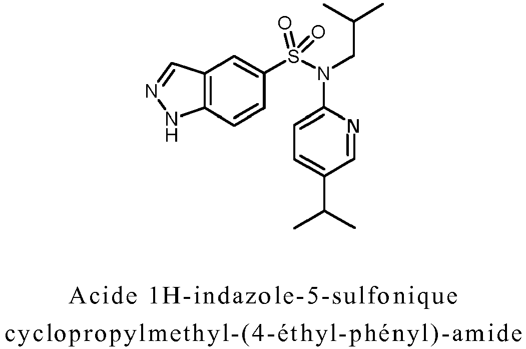

Avec le même opératoire que celui utilisé pour l'intermédiaire 30.2, l'acide 1H-indazole-5-sulfonique cyclo-propylmethyl-(4-éthyl-phényl)-amide (40mg; 12 %) est obtenu sous la forme d'une huile jaune avec une RMN¹H conforme
MS : [M+H] =373

### 4. Synthèse de l'intermédiaire 45.2

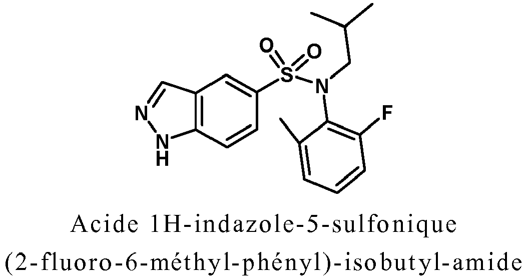

Le (2-fluoro-6-méthyl-phényl)-isobutyl-amine (520 mg; 2.87 mmol) est ajouté à une solution de chlorure de 1h-indazole-5-sulfonyle (297.4 mg; 1.30 mmol) dans de l'acetonitrile (1.25 ml) et le milieu réactionnel est agité pendant 40 minutes sous micro-ondes à une température de 100°C. Le milieu réactionnel est traité avec une solution d'acide chlorhydrique 1N et extrait à l'acétate d'éthyle. Les phases organiques sont rassemblées, lavées à l'eau, séchées (MgSO₄), filtrées et concentrées.

Le produit brut est chromatographié sur gel de silice (éluant heptane/acétate d'éthyle de 0 à 60% d'acétate d'éthyle). L'acide 1H-Indazole-5-sulfonique (2-fluoro-6-methyl-phényl)-isobutyl-amide (305 mg; 65 %) est obtenu sous forme d'un solide blanc avec une RMN¹H conforme
MS : [M+H] =362

Avec un mode opératoire analogue à celui décrit pour synthétiser l'exemple 1, l'addition de 1.2 équivalent de 4-(bromo-méthyl)-tétrahydropyrane sur 1 équivalent de 1H-indazole-5-sulfonamide N-substitué (précédemment préparé) dans du N,N-diméthylformamide en présence de 1 équivalent de carbonate de césium sous micro-onde à 100°C, conduit aux composés du tableau ci-dessous :

| | | |
|---|---|---|
| **Exemple 12** | 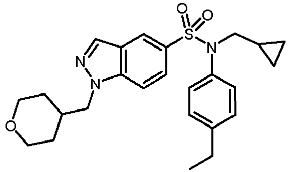 | **N-(cyclopropylméthyl)-N-(4- éthylphényl)-1-((tétrahydro-2H-pyran- 4-yl)methyl)-1H-indazole-5- sulfonamide** |
| | | ¹H NMR (DMSO-d6) δ: 0.23 - 0.35 (m, 2H), 0.64 - 0.77 (m, 1H), 1.12 (t, J = 7.6 Hz, 3H), 1.19 - 1.37 (m, 4H), 2.10 (dtd, J = 11.1, 6.9, 3.4 Hz, 1H), 2.54 (q, J = 7.6 Hz, 2H), 3.17 (td, J = 11.3, 3.0 Hz, 2H), 3.38 (d, J = 7.0 Hz, 2H), 3.76 (ddd, J = 11.5, 4.4, 2.2 Hz, 2H), 4.32 (d, J = 7.1 Hz, 2H), 6.89 - 6.97 (m, 2H), 7.11 (d, J = 8.3 Hz, 2H), 7.45 (dd, J = 8.9, 1.7 Hz, 1H), 7.85 (dt, J = 8.8, 0.9 Hz, 1H), 8.06 (d, J = 1.6 Hz, 1H), 8.23 (d, J = 0.9 Hz, 1H). |
| | Composé 31 | MS : [M+H] =454 |
| **Exemple 13** | 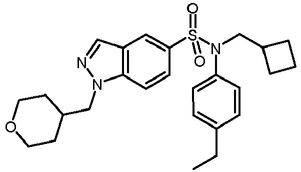 | **N-(cyclobutylméthyl)-N-(4- éthylphényl)-1-((tétrahydro-2H-pyran- 4-yl)méthyl)-1H-indazole-5- sulfonamide** |
| | | ¹H NMR (DMSO-d6) δ: 1.17 (t, J = 7.6 Hz, 3H), 1.24 - 1.45 (m, 4H), 1.54 - 1.66 (m, 2H), 1.69 - 1.89 (m, 4H), 2.10 - 2.30 (m, 2H), 2.59 (q, J = 7.6 Hz, 2H), 3.27 (td, J = 11.6, 2.4 Hz, 2H), 3.56 (d, J = 7.5 Hz, 2H), 3.83 (ddd, 2H), 4.40 (d, J = 7.1 Hz, 2H), 6.88 - 6.96 (m, 2H), 7.15 (d, J = 8.3 Hz, 2H), 7.31 (dd, J = 9.1, 1.8 Hz, 1H), 7.75 (d, J = 9.1 Hz, 1H), 8.12 - 8.14 (m, 1H), 8.63 (d, J = 0.9 Hz, 1H) |
| | Composé 24 | MS : [M+H] =468 |
| **Exemple 14** | 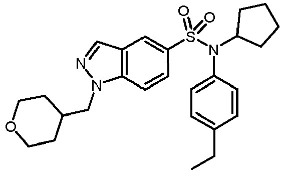 | **acide 1-(tétrahydro-pyran-4-ylméthyl)- 1H-indazole-5-sulfonique cyclopentyl- (4-éthyl-phényl)-amide** |
| | | ¹H NMR (DMSO-d6) δ: 1.12 - 1.26 (m, 5H), 1.26 - 1.49 (m, 9H), 1.74 (dd, J = 12.2, 6.9 Hz, 2H), 2.61 (q, J = 7.6 Hz, 2H), 3.24 (td, J = 11.3, 2.9 Hz, 2H), 3.82 (ddd, J = 11.5, 4.3, 2.2 Hz, 2H), 4.39 (d, J = 7.0 Hz, 2H), 4.46 - 4.57 (m, 1H), 6.87 - 6.92 (m, 2H), 7.17 - 7.23 (m, 2H), 7.69 (dd, J = 9.0, 1.8 Hz, 1H), 7.91 - 7.96 (m, 1H), 8.21 (d, J = 1.6 Hz, 1H), 8.30 (d, J = 0.9 Hz, 1H) |
| | Composé 32 | MS : [M+H] =468 |
| **Exemple 15** | 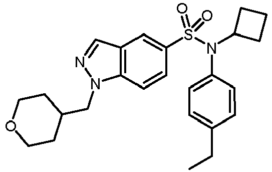 | **acide 1-(tétrahydro-pyran-4-ylmethyl)- 1H-indazole-5-sulfonique cyclobutyl-(444 éthyl-phényl)-amide** |
| | | ¹H NMR (DMSO-d6) δ: 1.18 (t, J = 7.6 Hz, 3H), 1.23 - 1.62 (m, 6H), 1.76 (dq, J = 12.0, 9.7 Hz, 2H), 2.05 (dddd, J = 9.4, 7.4, 5.1, 2.5 Hz, 2H), 2.10 - 2.26 (m, 1H), 2.61 (q, J = 7.6 Hz, 2H), 3.24 (td, J = 11.3, 2.9 Hz, 2H), 3.82 (ddd, J = 11.5, 4.3, 2.1 Hz, 2H), 4.38 (dd, J = 8.5, 6.5 Hz, 3H), 6.80 (d, J = 8.3 Hz, 2H), 7.17 (d, J = 8.3 Hz, 2H), 7.48 (dd, J = 9.0, 1.8 Hz, 1H), 7.90 (d, J = 9.1 Hz, 1H), 8.11 (d, J = 1.6 Hz, 1H), 8.31 (s, 1H) |
| | Composé 41 | MS : [M+H] =454 |
| **Exemple 16** | 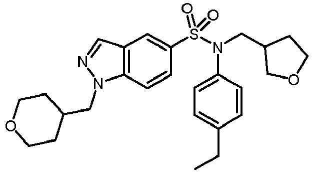 | **acide 1-(tétrahydro-pyran-4-ylméthyl)- 1H-indazole-5-sulfonique (4-éthyl- phényl)-(tétrahydro-furan-3-ylméthyl)- amide** |
| | | ¹H NMR (DMSO-d6) δ: 1.18 (t, J = 7.6 Hz, 3H), 1.25 - 1.43 (m, 4H), 1.58 (dq, J = 13.2, 6.8 Hz, 1H), 1.76 - 1.90 (m, 1H), 2.06 (p, J = 5.9 Hz, 1H), 2.11 - 2.22 (m, 1H), 2.61 (q, J = 7.6 Hz, 2H), 3.24 (td, J = 11.3, 3.0 Hz, 2H), 3.42 (dd, J = 8.6, 5.2 Hz, 1H), 3.48 - 3.62 (m, 4H), 3.71 (td, J = 8.1, 5.4 Hz, 1H), 3.76 - 3.89 (m, 2H), 4.39 (d, J = 7.0 Hz, 2H), 6.91 - 7.00 (m, 2H), 7.14 - 7.24 (m, 2H), 7.48 (dd, J = 9.0, 1.7 Hz, 1H), 7.87 - 7.95 (m, 1H), 8.12 (d, J = 1.6 Hz, 1H), 8.30 (d, J = 1.0 Hz, 1H) |
| | Composé 33 | MS : [M+H] =484 |
| **Exemple 17** | 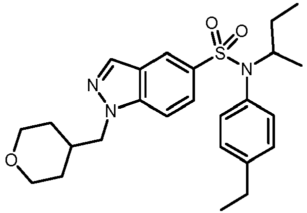 | **acide 1-(tétrahydro-pyran-4-ylméthyl)- 1H-indazole-5-sulfonique sec-butyl-(4- éthyl-phényl)-amide** |
| | | ¹H NMR (DMSO-d6) δ: 0.85 (t, J = 7.3 Hz, 3H), 0.93 (d, J = 6.7 Hz, 3H), 1.12 - 1.25 (m, 5H), 1.26 - 1.44 (m, 4H), 2.62 (q, J = 7.6 Hz, 2H), 3.24 (td, J = 11.3, 3.2 Hz, 2H), 3.82 (ddd, J = 11.5, 4.3, 2.3 Hz, 2H), 4.19 (dt, J = 7.8, 6.4 Hz, 1H), 4.39 (d, J = 7.1 Hz, 2H), 6.91 (d, J = 8.3 Hz, 2H), 7.21 (d, J = 8.3 Hz, 2H), 7.68 (dd, J = 8.9, 1.8 Hz, 1H), 7.94 (dt, J = 9.0, 0.9 Hz, 1H), 8.20 (d, J = 1.6 Hz, 1H), 8.30 (d, J = 0.9 Hz, 1H) |
| | Composé 30 | MS : [M+H] =456 |
| **Exemple 18** | 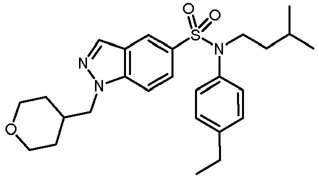 | **N-(4-éthylphényl)-N-isopentyl-1- ((tétrahydro-2H-pyran-4-yl)méthyl)- 1H-indazole-5-sulfonamide** |
| | | ¹H NMR (DMSO-d6) δ: 0.80 (d, J = 6.6 Hz, 6H), 1.17 (t, J = 7.6 Hz, 5H), 1.25 - 1.42 (m, 4H), 1.61 (hept, J = 6.7 Hz, 1H), 2.09 - 2.24 (m, 1H), 2.60 (q, J = 7.6 Hz, 2H), 3.23 (td, J = 11.2, 3.1 Hz, 2H), 3.55 (t, J = 7.1 Hz, 2H), 3.81 (dt, J = 11.5, 3.3 Hz, 2H), 4.38 (d, J = 7.1 Hz, 2H), 6.90 - 6.99 (m, 2H), 7.12 - 7.22 (m, 2H), 7.47 (dd, J = 9.0, 1.7 Hz, 1H), 7.87 - 7.96 (m, 1H), 8.11 (d, J = 2.0 Hz, 1H), 8.30 (d, J = 1.1 Hz, 1H) |
| | Composé 35 | MS : [M+H] =470 |
| **Exemple 19** | 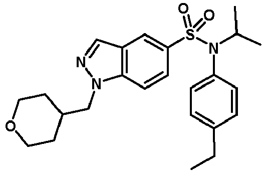 | **N-(4-ethylphenyl)-N-isopropyl-1- ((tetrahydro-2H-pyran-4-yl)methyl)- 1H-indazole-5-sulfonamide** |
| | | ¹H NMR (DMSO-d6) δ: 0.95 (d, J = 6.7 Hz, 6H), 1.19 (t, J = 7.6 Hz, 3H), 1.26 - 1.45 (m, 4H), 2.63 (q, J = 7.6 Hz, 2H), 3.24 (td, J = 11.3, 3.0 Hz, 2H), 3.82 (ddd, J = 11.5, 4.3, 2.2 Hz, 2H), 4.39 (d, J = 7.1 Hz, 2H), 4.47 (p, J = 6.7 Hz, 1H), 6.90 - 6.98 (m, 2H), 7.22 (d, J = 8.3 Hz, 2H), 7.70 (dd, J = 8.9, 1.8 Hz, 1H), 7.90 - 7.99 (m, 1H), 8.22 (d, J = 1.6 Hz, 1H), 8.30 (d, J = 0.9 Hz, 1H). |
| | Composé 29 | MS : [M+H] =442 |
| **Exemple 20** | 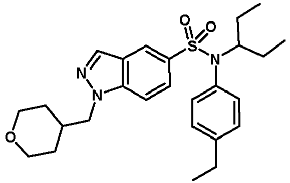 | **acide 1-(tetrahydro-pyran-4-ylmethyl)- 1H-indazole-5-sulfonique (4-ethyl- phenyl)-(1-ethyl-propyl)-amide** |
| | | ¹H NMR (DMSO-d6) δ: 0.85 (t, J = 7.3 Hz, 6H), 1.07 - 1.22 (m, 5H), 1.30 (ddd, J = 20.0, 9.0, 5.7 Hz, 6H), 2.16 (s, 1H), 2.61 (q, J = 7.6 Hz, 2H), 3.23 (td, J = 11.2, 3.4 Hz, 2H), 3.81 (d, J = 11.1 Hz, 2H), 3.91 (q, J = 6.5 Hz, 1H), 4.39 (d, J = 7.2 Hz, 2H), 6.86 - 6.93 (m, 2H), 7.21 (d, J = 8.3 Hz, 2H), 7.93 (d, J = 9.0 Hz, 1H), 8.15 (d, J = 1.5 Hz, 1H), 8.29 (d, J = 0.9 Hz, 1H). |
| | Composé 1 | MS : [M+H] =470 |
| **Exemple 22** | 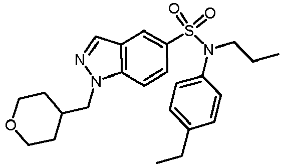 | **acide 1-(tetrahydro-pyran-4-ylmethyl)- 1H-indazole-5-sulfonique (4-ethyl- phenyl)-propyl-amide** |
| | | ¹H NMR (DMSO-d6) δ: 0.83 (t, J = 7.3 Hz, 3H), 1.17 (t, J = 7.6 Hz, 3H), 1.25 - 1.44 (m, 6H), 2.24 (ddt, J = 10.9, 7.5, 3.8 Hz, 1H), 2.60 (q, J = 7.5 Hz, 2H), 3.26 (td, J = 11.6, 2.5 Hz, 2H), 3.50 (t, J = 6.9 Hz, 2H), 3.83 (ddd, J = 11.5, 4.5, 2.0 Hz, 2H), 4.40 (d, J = 7.1 Hz, 2H), 6.93 - 7.02 (m, 2H), 7.17 (d, J = 8.3 Hz, 2H), 7.29 (dd, J = 9.1, 1.8 Hz, 1H), 7.70 - 7.80 (m, 1H), 8.12 (dd, J = 1.9, 0.8 Hz, 1H), 8.62 (d, J = 1.0 Hz, 1H) |
| | Composé 8 | MS : [M+H] =442 |
| **Exemple 23** | 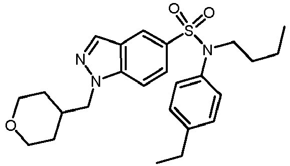 | **acide 1-(tetrahydro-pyran-4-ylmethyl)- 1H-indazole-5-sulfonique butyl-(4- ethyl-phenyl)-amide** |
| | | ¹H NMR (DMSO-d6) δ: 0.74 - 0.88 (m, 3H), 1.17 (t, J = 7.6 Hz, 3H), 1.23 - 1.45 (m, 8H), 2.24 (ddt, J = 11.0, 7.6, 3.8 Hz, 1H), 2.60 (q, J = 7.6 Hz, 2H), 3.26 (td, J = 11.5, 2.5 Hz, 2H), 3.54 (q, J = 4.4 Hz, 2H), 3.83 (ddd, J = 11.5, 4.3, 2.0 Hz, 2H), 4.40 (d, J = 7.2 Hz, 2H), 6.93 - 7.01 (m, 2H), 7.17 (d, J = 8.3 Hz, 2H), 7.28 (dd, J = 9.1, 1.8 Hz, 1H), 7.74 (dd, J = 9.0, 1.0 Hz, 1H), 8.11 - 8.14 (m, 1H), 8.62 (d, J = 0.9 Hz, 1H). |
| | Composé 9 | MS : [M+H] =456 |
| **Exemple 25** | 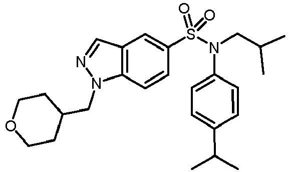 | **acide 1-(tetrahydro-pyran-4-ylméthyl)- 1H-indazole-5-sulfonique butyl-(4- isopropyl-phenyl)-amide** |
| | | ¹H NMR (DMSO-d6) δ: 0.85 (d, J = 6.7 Hz, 6H), 1.19 (d, J = 6.9 Hz, 6H), 1.25 - 1.50 (m, 5H), 2.11 - 2.22 (m, 1H), 2.88 (hept, J = 6.8 Hz, 1H), 3.23 (td, J = 11.3, 2.9 Hz, 2H), 3.82 (ddd, J = 11.6, 4.4, 2.2 Hz, 2H), 4.38 (d, J = 7.1 Hz, 2H), 6.94 - 7.03 (m, 2H), 7.20 (d, J = 8.4 Hz, 2H), 7.44 (dd, J = 8.9, 1.8 Hz, 1H), 7.85 - 7.94 (m, 1H), 8.09 (d, J = 1.7 Hz, 1H), 8.29 (d, J = 0.9 Hz, 1H) |
| | Composé 7 | MS : [M+H] =470 |
| **Exemple 26** | 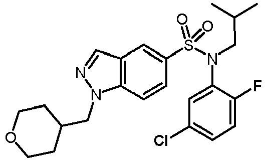 | **acide 1-(tétrahydro-pyran-4-ylméthyl)- 1H-indazole-5-sulfonique (5-chloro-2- fluoro-phényl)-isobutyl-amide** |
| | | ¹H NMR (DMSO-*d*₆) δ: 0.85 (d, J = 6.6 Hz, 6H), 1.21 - 1.49 (m, 5H), 2.17 (dd, J = 10.5, 5.9 Hz, 1H), 3.23 (td, J = 11.1, 3.3 Hz, 2H), 3.76 - 3.87 (m, 2H), 4.40 (d, J = 7.0 Hz, 2H), 7.15 (dd, J = 6.5, 2.7 Hz, 1H), 7.35 (dd, J = 10.1, 8.9 Hz, 1H), 7.50 (ddd, J = 8.8, 4.1, 2.6 Hz, 1H), 7.56 (dd, J = 9.0, 1.8 Hz, 1H), 7.92 - 7.99 (m, 1H), 8.20 (d, J = 1.7 Hz, 1H), 8.33 (d, J = 1.0 Hz, 1H). |
| | Composé 10 | MS : [M+H] =480 |
| **Exemple 27** | 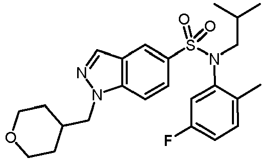 | **acide 1-(tetrahydro-pyran-4-ylmethyl)- 1H-indazole-5-sulfonique (5-fluoro-2- methyl-phenyl)-isobutyl-amide** |
| | | ¹H NMR (DMSO-d6) δ: 0.77 (d, J = 6.7 Hz, 3H), 0.95 (d, J = 6.6 Hz, 3H), 1.22 - 1.49 (m, 5H), 2,07 - 2.25 (m, 1H), 2.27 (s, 3H), 3.13 (dd, J = 13.2, 4.7 Hz, 1H), 3.23 (td, J = 11.2, 3.4 Hz, 2H), 3.43 (dd, J = 13.2, 9.1 Hz, 1H), 3.81 (dt, J = 11.5, 3.2 Hz, 2H), 4.40 (d, J = 7.1 Hz, 2H), 6.45 (dd, J = 10.1, 2.7 Hz, 1H), 7.12 (td, J = 8.3, 2.7 Hz, 1H), 7.35 (dd, J = 8.6, 6.6 Hz, 1H), 7.51 (dd, J = 8.9, 1.8 Hz, 1H), 7.91 - 7.99 (m, 1H), 8.16 (d, J = 1.6 Hz, 1H), 8.34 (d, J = 0.9 Hz, 1H). |
| | Composé 42 | MS : [M+H] =460 |
| **Exemple 28** | 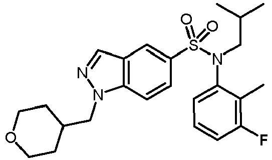 | **acide 1-(tetrahydro-pyran-4-ylmethyl)- 2H-indazole-5-sulfonique (3-fluoro-2- methyl-phenyl)-isobutyl-amide** |
| | | ¹H NMR (DMSO-d6) δ: 0.77 (d, J = 6.7 Hz, 3H), 0.94 (d, J = 6.5 Hz, 3H), 1.18 - 1.52 (m, 5H), 2.08 - 2.27 (m, 4H), 3.13 (dd, J = 13.1, 4.8 Hz, 1H), 3.23 (td, J = 11.2, 3.1 Hz, 2H), 3.46 (dd, J = 13.2, 9.0 Hz, 1H), 3.73 - 3.88 (m, 2H), 4.39 (d, J = 7.1 Hz, 2H), 6.52 (d, J = 8.1 Hz, 1H), 7.06 - 7.26 (m, 2H), 7.52 (dd, J = 8.9, 1.8 Hz, 1H), 7.93 (d, J = 8.8 Hz, 1H), 8.14 (d, J = 1.7 Hz, 1H), 8.32 (d, J = 1.0 Hz, 1H). |
| | Composé 43 | MS : [M+H] =460 |
| **Exemple 29** | 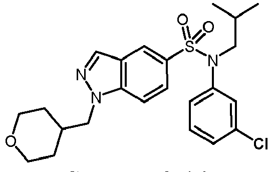 | **acide 1-(tetrahydro-pyran-4-ylmethyl)- 1H-indazole-5-sulfonique (5-chloro- phenyl)-isobutyl-amide** |
| | | ¹H NMR (DMSO-d6) δ: 0.85 (d, J = 6.6 Hz, 6H), 1.21 - 1.50 (m, 5H), 3.23 (td, J = 11.2, 3.2 Hz, 2H), 3.38 (d, J = 7.3 Hz, 2H), 3.80 (s, 2H), 4.38 (d, J = 7.1 Hz, 2H), 7.08 (dt, J = 7.0, 2.0 Hz, 1H), 7.13 (t, J = 1.9 Hz, 1H), 7.34 - 7.40 (m, 2H), 7.41 - 7.44 (m, 1H), 7.91 (d, J = 8.9 Hz, 1H), 8.11 (d, J = 1.7 Hz, 1H), 8.31 (d, J = 1.0 Hz, 1H). |
| | Composé 44 | MS : [M+H] =462 |
| **Exemple 30** | 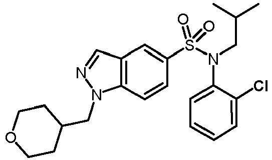 | **acide 1-(tétrahydro-pyran-4-ylméthyl)- 1H-indazole-5-sulfonique (2-chloro- phényl)-isobutyl-amide** |
| | | ¹H NMR (400 MHz, CDCl3) δ 0.86 (t, J = 6.2 Hz, 6H), 1.37 (td, J = 10.9, 9.9, 4.0 Hz, 4H), 1.55 (dt, J = 13.5, 6.8 Hz, 2H), 2.22 (s, 1H), 3.28 (td, J = 11.1, 4.0 Hz, 2H), 3.36 (dd, J = 7.2, 1.8 Hz, 2H), 3.89 (dt, J = 11.6, 3.3 Hz, 2H), 4.22 (d, J = 7.1 Hz, 2H), 7.19 (m, 3H), 7.25 - 7.30 (m, 1H), 7.36 (d, J = 8.8 Hz, 1H), 7.61 (dd, J = 8.9, 1.7 Hz, 1H), 8.03 (s, 1H), 8.07 (d, J = 1.6 Hz, 1H). |
| | Composé 45 | MS : [M+H] =462 |
| **Exemple 31** | 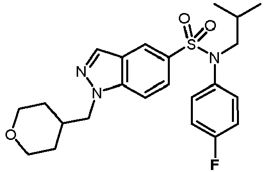 | **acide 1-(tétrahydro-pyran-4-ylméthyl)- 1H-indazole-5-sulfonique (4-fluoro- phényl)-isobutyl-amide** |
| | | ¹H NMR (DMSO-d6) δ: 0.85 (d, J = 6.6 Hz, 6H), 1.23 - 1.48 (m, 5H), 2.16 (ddd, J = 11.0, 6.9, 4.2 Hz, 1H), 3.23 (td, J = 11.3, 2.9 Hz, 2H), 3.35 (s, 2H), 3.82 (ddd, J = 11.5, 4.5, 2.2 Hz, 2H), 4.38 (d, J = 7.0 Hz, 2H), 7.05 - 7.22 (m, 4H), 7.45 (dd, J = 8.9, 1.7 Hz, 1H), 7.90 (d, J = 8.9 Hz, 1H), 8.07 (d, J = 1.7 Hz, 1H), 8.30 (s, 1H). |
| | Composé 46 | MS : [M+H] =446 |
| **Exemple 32** | 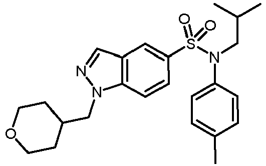 | **acide 1-(tétrahydro-pyran-4-ylméthyl)- 1H-indazole-5-sulfonique isobutyl-p- tolyl-amide** |
| | | ¹H NMR (DMSO-d6) δ: 0.85 (d, J = 6.7 Hz, 6H), 1.23 - 1.48 (m, 5H), 2.05 - 2.25 (m, 1H), 2.29 (s, 3H), 3.24 (td, J = 11.3, 2.8 Hz, 3H), 3.32 (td, 2H), 3.82 (dd, J = 11.3, 3.7 Hz, 2H), 4.38 (d, J = 7.1 Hz, 2H), 6.89 - 6.95 (m, 2H), 7.13 (d, J = 8.1 Hz, 2H), 7.45 (dd, J = 8.9, 1.8 Hz, 1H), 7.89 (d, J = 8.8 Hz, 1H), 8.07 (d, J = 1.6 Hz, 1H), 8.30 (s, 1H). |
| | Composé 47 | MS : [M+H] =442 |
| **Exemple 33** | 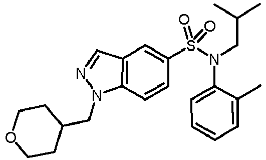 | **acide 1-(tétrahydro-pyran-4-ylméthyl)- 1H-indazole-5-sulfonique isobutyl-o- tolyl-amide** |
| | | ¹H NMR (DMSO-d6) δ: 0.76 (d, J = 6.7 Hz, 3H), 0.96 (d, J = 6.6 Hz, 3H), 1.24 - 1.50 (m, 5H), 2.07 - 2.26 (m, 1H), 2.30 (s, 3H), 3.13 (dd, J = 13.2, 4.9 Hz, 1H), 3.24 (td, J = 11.0, 2.9 Hz, 2H), 3.45 (dd, J = 13.1, 8.9 Hz, 1H), 3.82 (dd, J = 13.4, 2.4 Hz, 2H), 4.40 (d, J = 7.1 Hz, 2H), 6.62 (dd, J = 8.1, 1.3 Hz, 1H), 7.08 (td, J = 7.7, 1.7 Hz, 1H), 7.24 (td, J = 7.5, 1.3 Hz, 1H), 7.31 (dd, J = 7.8, 1.6 Hz, 1H), 7.53 (dd, J = 8.9, 1.8 Hz, 1H), 7.93 (d, J = 8.9 Hz, 1H), 8.13 (s, 1H), 8.32 (s, 1H). |
| | Composé 48 | MS : [M+H] =442 |
| **Exemple 34** | 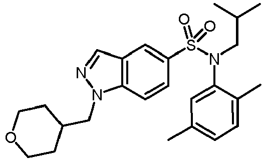 | **acide 1-(tetrahydro-pyran-4-ylmethyl)- 1H-indazole-5-sulfonique (2.5- diméthyl-phenyl)-isobutyl-amide** |
| | | ¹H NMR (DMSO-d6) δ: 0.77 (d, J = 6.7 Hz, 3H), 0.96 (d, J = 6.6 Hz, 3H), 1.26 - 1.38 (m, 4H), 1.44 (dtd, J = 8.9, 6.7, 4.8 Hz, 1H), 2.03 (s, 3H), 2.12 - 2.22 (m, 1H), 2.23 (s, 3H), 3.07 (dd, J = 13.1, 4.8 Hz, 1H), 3.19 - 3.27 (m, 2H), 3.43 (dd, J = 13.1, 8.9 Hz, 1H), 3.81 (dq, J = 10.8, 3.4 Hz, 2H), 4.41 (d, J = 7.0 Hz, 2H), 6.29 (d, J = 1.7 Hz, 1H), 7.04 (dd, J = 7.8, 1.8 Hz, 1H), 7.17 (d, J = 7.7 Hz, 1H), 7.49 (dd, J = 9.0, 1.7 Hz, 1H), 7.93 (dt, J = 9.0, 0.9 Hz, 1H), 8.13 (d, J = 1.6 Hz, 1H), 8.32 (d, J = 0.9 Hz, 1H) |
| | Composé 11 | MS : [M+H] = 456 |
| **Exemple 35** | 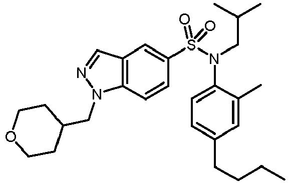 | **acide 1-(tétrahydro-pyran-4-ylméthyl)- 1H-indazole-5-sulfonique (4-butyl-2- méthyl-phényl)-isobutyl-amide** |
| | | ¹H NMR (DMSO-d6) δ: 0.75 (d, J = 6.6 Hz, 3H), 0.90 (t, J = 7.3 Hz, 3H), 0.95 (d, J = 6.6 Hz, 3H), 1.24 - 1.47 (m, 8H), 1.48 - 1.59 (m, 2H), 2.16 (s, 1H), 2.25 (s, 3H), 3.09 (dd, J = 13.1, 4.9 Hz, 1H), 3.24 (td, J = 11.1, 3.2 Hz, 2H), 3.37 - 3.46 (m, 1H), 3.77 - 3.88 (m, 2H), 4.39 (d, J = 7.0 Hz, 2H), 6.52 (d, J = 8.2 Hz, 1H), 6.88 (dd, J = 8.2, 2.2 Hz, 1H), 7.11 (d, J = 2.1 Hz, 1H), 7.52 (dd, J = 9.0, 1.7 Hz, 1H), 7.85 - 7.98 (m, 1H), 8.13 (d, J= 1.6 Hz, 1H), 8.31 (d, J = 0.9 Hz, 1H) |
| | Composé 13 | MS : [M+H] = 498 |
| **Exemple 36** | 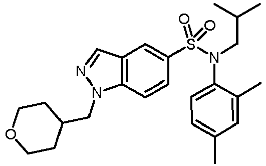 | **acide 1-(tetrahydro-pyran-4-ylmethyl)- 1H-indazole-5-sulfonique (2.4- diméthyl-phényl)-isobutyl-amide** |
| | | ¹H NMR (DMSO-d6) δ: 0.76 (d, J = 6.7 Hz, 3H), 0.96 (d, J = 6.5 Hz, 3H), 1.22 - 1.48 (m, 5H), 2.09 (d, J = 4.8 Hz, 1H), 2.11 - 2.21 (m, 1H), 2.25 (d, J = 4.1 Hz, 6H), 3.08 (dd, J = 13.1, 4.8 Hz, 1H), 3.24 (td, J = 11.2, 3.2 Hz, 2H), 3.43 (dd, J = 13.1, 8.9 Hz, 1H), 3.76 - 3.87 (m, 2H), 4.40 (d, J = 7.0 Hz, 2H), 6.48 (d, J = 8.1 Hz, 1H), 6.87 (dd, J = 8.1, 2.1 Hz, 1H), 7.11 (d, J = 2.1 Hz, 1H), 7.53 (dd, J = 9.0, 1.7 Hz, 1H), 7.90 - 7.96 (m, 1H), 8.13 (d, J = 1.7 Hz, 1H), 8.32 (d, J = 0.9 Hz, 1H) |
| | Composé 27 | MS : [M+H] = 456 |
| **Exemple 37** | 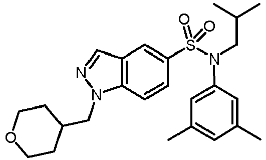 | **acide 1-(tétrahydro-pyran-4-ylméthyl)- 1H-indazole-5-sulfonique (3,5- diméthyl-phényl)-isobutyl-amide** |
| | | ¹H NMR (DMSO-d6) δ: 0.85 (d, J = 6.6 Hz, 6H), 1.22 - 1.38 (m, 4H), 1.44 (hept, J = 6.8 Hz, 1H), 2.16 (s, 7H), 3.23 (td, J = 11.1, 3.6 Hz, 2H), 3.29 (d, J = 7.3 Hz, 2H), 3.76 - 3.85 (m, 2H), 4.39 (d, J = 7.0 Hz, 2H), 6.58 - 6.63 (m, 2H), 6.94 (s, 1H), 7.42 (dd, J = 8.8, 1.7 Hz, 1H), 7.87 - 7.92 (m, 1H), 8.09 (d, J = 1.6 Hz, 1H), 8.31 (d, J = 0.9 Hz, 1H) |
| | Composé 28 | MS : [M+H] = 456 |
| **Exemple 38** | 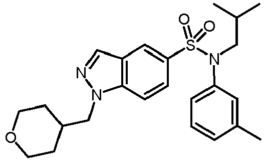 | **acide 1-(tétrahydro-pyran-4-ylméthyl)- 1H-indazole-5-sulfonique (3-méthyl- phényl)-isobutyl-amide** |
| | | ¹H NMR (DMSO-d6) δ: 0.85 (d, J = 6.6 Hz, 6H), 1.23 - 1.52 (m, 5H), 2.22 (s, 4H), 3.23 (td, J = 11.3, 3.2 Hz, 2H), 3.77 - 3.87 (m, 2H), 4.38 (d, J = 7.1 Hz, 2H), 6.78 - 6.89 (m, 2H), 7.10 - 7.15 (m, 1H), 7.20 (t, J = 7.7 Hz, 1H), 7.85 - 7.93 (m, 1H), 8.08 (d, J = 1.6 Hz, 1H), 8.30 (d, J = 0.9 Hz, 1H) |
| | Composé 26 | MS : [M+H] =442 |
| **Exemple 39** | 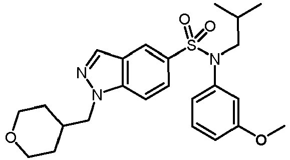 | **acide 1-(tétrahydro-pyran-4-ylméthyl)- 1H-indazole-5-sulfonique (3-méthoxy- phényl)-isobutyl-amide** |
| | | ¹H NMR (DMSO-d6) δ: 0.86 (d, J = 6.6 Hz, 6H), 1.21 - 1.38 (m, 4H), 1.45 (hept, J = 6.8 Hz, 1H), 3.23 (td, J = 11.3, 3.2 Hz, 2H), 3.61 (s, 3H), 3.81 (dt, J = 11.7, 2.7 Hz, 2H), 4.38 (d, J = 7.0 Hz, 2H), 6.53 (t, J = 2.3 Hz, 1H), 6.66 (ddd, J = 7.9, 2.0, 0.9 Hz, 1H), 6.89 (ddd, J = 8.3, 2.5, 0.9 Hz, 1H), 7.24 (t, J = 8.1 Hz, 1H), 7.46 (dd, J = 8.9, 1.7 Hz, 1H), 7.88 - 7.94 (m, 1H), 8.10 (d, J= 1.6 Hz, 1H), 8.30 (d, J = 0.8 Hz, 1H) |
| | Composé 25 | MS : [M+H] = 458 |
| **Exemple 40** | 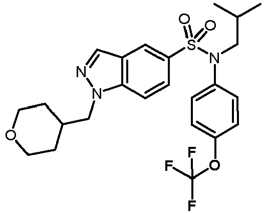 | **acide 1-(tétrahydro-pyran-4-ylméthyl)- 1H-indazole-5-sulfonique (4- trifluorométhoxy-phenyl)-isobutyl- amide** |
| | | ¹H NMR (DMSO-d6) δ: 0.85 (d, J = 6.6 Hz, 6H), 1.24 - 1.47 (m, 5H), 2.10 - 2.20 (m, 1H), 3.23 (td, J = 11.3, 2.9 Hz, 2H), 3.37 (d, J = 7.4 Hz, 2H), 3.80 (s, 2H), 4.38 (d, J = 7.1 Hz, 2H), 7.20 - 7.26 (m, 2H), 7.32 - 7.37 (m, 2H), 7.43 (dd, J = 9.0, 1.8 Hz, 1H), 7.91 (d, J = 9.0 Hz, 1H), 8.10 (d, J = 1.7 Hz, 1H), 8.30 (d, J = 0.9 Hz, 1H) ; |
| | Composé 49 | MS : [M+H] = 512 |
| **Exemple 41** | 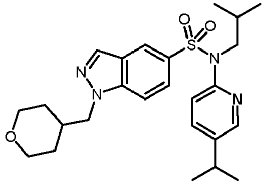 | **acide 1-(tétrahydro-pyran-4-ylméthyl)- 1H-indazole-5-sulfonique isobutyl-(5- isopropyl-pyridin-2-yl)-amide** |
| | | ¹H NMR (Chloroform-d) δ: 0.82 (d, J = 6.7 Hz, 6H), 1.20 (s, 6H), 1.30 - 1.44 (m, 4H), 2.15 - 2.28 (m, 1H), 2.87 (p, J = 7.0 Hz, 1H), 3.28 (td, J = 11.5, 3.0 Hz, 2H), 3.42 (d, J = 7.3 Hz, 2H), 3.86 - 3.93 (m, 2H), 4.18 (d, J = 7.2 Hz, 2H), 7.29 - 7.33 (m, 1H), 7.40 (dd, J = 9.0, 1.7 Hz, 1H), 7.49 (d, J = 8.1 Hz, 1H), 7.55 (dd, J = 8.3, 2.5 Hz, 1H), 8.01 (d, J = 0.8 Hz, 1H), 8.04 (d, J = 1.8 Hz, 1H), 8.08 (d, J = 2.5 Hz, 1H). |
| | Composé 50 | MS : [M+H] = 471 |
| **Exemple 42** | 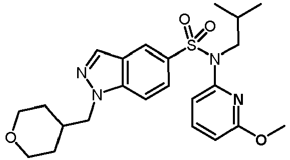 | **acide 1-(tétrahydro-pyran-4-ylméthyl)- 1H-indazole-5-sulfonique (3-méthoxy- pyridin-2-yl)-isobutyl-amide** |
| | | ¹H NMR (DMSO-d6) δ: 0.86 (d, J = 6.6 Hz, 6H), 1.20 - 1.37 (m, 4H), 1.57 (hept, J = 6.9 Hz, 1H), 2.03 - 2.24 (m, 1H), 3.12 - 3.26 (m, 3H), 3.42 (s, 3H), 3.53 (d, J = 7.1 Hz, 2H), 3.71 - 3.87 (m, 2H), 4.37 (d, J = 7.1 Hz, 2H), 6.69 (d, J = 8.1 Hz, 1H), 7.12 (d, J = 7.5 Hz, 1H), 7.46 (dd, J = 9.0, 1.8 Hz, 1H), 7.73 - 7.82 (m, 1H), 7.88 (dd, J = 9.0, 1.0 Hz, 1H), 8.19 (d, J = 1.6 Hz, 1H), 8.29 (d, J = 0.9 Hz, 1H) |
| | Composé 12 | MS : [M+H] =459 |
| **Exemple 43** | 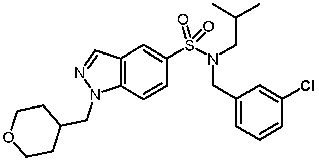 | **acide 1-(tétrahydro-pyran-4-ylméthyl)- 1H-indazole-5-sulfonique (3-chloro- benzyl)-isobutyl-amide** |
| | | ¹H NMR (DMSO-d6) δ: 0.70 (d, J = 6.6 Hz, 6H), 1.25 - 1.42 (m, 4H), 1.58 (dt, J = 13.6, 6.9 Hz, 1H), 2.92 (d, J = 7.6 Hz, 2H), 3.24 (td, J = 11.3, 3.2 Hz, 2H), 3.82 (d, J = 11.2 Hz, 2H), 4.33 (s, 2H), 4.40 (d, J = 7.1 Hz, 2H), 7.24 - 7.37 (m, 4H), 7.80 (dd, J = 9.0, 1.7 Hz, 1H), 7.96 (d, J = 8.9 Hz, 1H), 8.31 (d, J = 0.9 Hz, 1H), 8.37 (d, J = 1.7 Hz, 1H) |
| | Composé 51 | MS : [M+H] = 476 |
| **Exemple 44** | 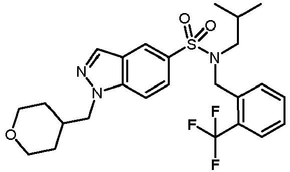 | **acide 1-(tétrahydro-pyran-4-ylméthyl)- 1H-indazole-5-sulfonique isobutyl-(2- trifluorométhyl-benzyl)-amide** |
| | | ¹H NMR (DMSO-d6) δ: 0.70 (d, J = 6.6 Hz, 6H), 1.23 - 1.43 (m, 5H), 2.97 (d, J = 7.2 Hz, 2H), 3.24 (td, J = 11.3, 3.0 Hz, 2H), 3.82 (d, J = 10.7 Hz, 2H), 4.40 (d, J = 7.1 Hz, 2H), 4.48 (s, 2H), 7.50 (t, J = 7.6 Hz, 1H), 7.70 (q, J = 7.6 Hz, 2H), 7.78 (d, J = 7.9 Hz, 1H), 7.83 (dd, J = 8.9, 1.8 Hz, 1H), 7.98 (d, J = 8.9 Hz, 1H), 8.39 (d, J = 1.7 Hz, 1H) |
| | Composé 52 | MS : [M+H] = 510 |
| **Exemple 45** | 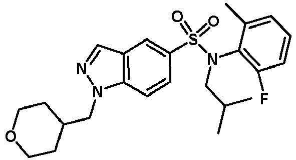 | **acide 1-(tétrahydro-pyran-4-ylméthyl)- 1H-indazole-5-sulfonique (2-fluoro-6- méthyl-phényl)-isobutyl-amide** |
| | | ¹H NMR (DMSO-d6) δ: 0.77 (d, J = 6.7 Hz, 3H), 0.93 (d, J = 6.6 Hz, 3H), 1.21 - 1.39 (m, 4H), 1.41 - 1.57 (m, 1H), 2.08 - 2.25 (m, 1H), 2.28 (s, 3H), 3.15 (dd, J = 13.7, 5.8 Hz, 1H), 3.23 (td, J = 11.0, 3.7 Hz, 2H), 3.45 (dd, J = 13.6, 7.9 Hz, 1H), 3.81 (dt, J = 11.4, 3.3 Hz, 2H), 4.39 (d, J = 7.0 Hz, 2H), 7.00 (ddd, J = 10.2, 8.3, 1.6 Hz, 1H), 7.15 (d, J = 7.6 Hz, 1H), 7.23 - 7.36 (m, 1H), 7.63 (dd, J = 8.9, 1.8 Hz, 1H), 7.93 (d, J = 9.1 Hz, 1H), 8.21 (d, J = 1.6 Hz, 1H), 8.31 (d, J = 1.0 Hz, 1H) |
| | Composé 53 | MS : [M+H] = 460 |
| **Exemple 46** | 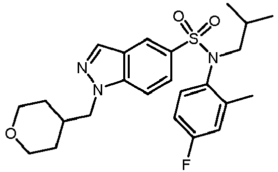 | **acide 1-(tétrahydro-pyran-4-ylméthyl)- 1H-indazole-5-sulfonique (4-fluoro-2- methyl-phenyl)-isobutyl-amide** |
| | | ¹H NMR (DMSO-d6) δ: 0.77 (d, J = 6.7 Hz, 3H), 0.95 (d, J = 6.5 Hz, 3H), 1.24 - 1.50 (m, 5H), 2.11 - 2.24 (m, 1H), 2.29 (s, 3H), 3.10 (dd, J = 13.1, 4.8 Hz, 1H), 3.23 (td, J = 11.2, 3.2 Hz, 2H), 3.45 (dd, J = 13.2, 8.9 Hz, 1H), 3.82 (dt, J = 11.6, 2.7 Hz, 2H), 4.39 (d, J = 7.1 Hz, 2H), 6.66 (dd, J = 8.9, 5.5 Hz, 1H), 6.92 (td, J = 8.4, 3.1 Hz, 1H), 7.18 (dd, J = 9.8, 3.0 Hz, 1H), 7.53 (dd, J = 8.9, 1.7 Hz, 1H), 7.93 (dd, J = 9.0, 0.9 Hz, 1H), 8.13 (d, J = 1.6 Hz, 1H), 8.32 (d, J = 0.9 Hz, 1H) |
| | Composé 54 | MS : [M+H] = 460 |
| **Exemple 47** | 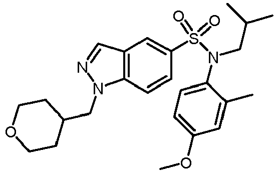 | **acide 1-(tétrahydro-pyran-4-ylméthyl)- 1H-indazole-5-sulfonique isobutyl-(4- méthoxy-2-méthyl-phényl)-amide** |
| | | 1H NMR (DMSO-d6) δ: 0.76 (d, J = 6.7 Hz, 3H), 0.96 (d, J = 6.6 Hz, 3H), 1.23 - 1.53 (m, 5H), 2.08 - 2.21 (m, 1H), 2.24 (s.3H), 3.24 (td, J = 11.2, 3.2 13.0, 4.8 3.43 (dd, J = 13.1, 8.9 Hz, 1H), 3.73 (s, 3H), 3.82 (dt, J = 11.4, 3.3 Hz, 2H), 4.39 (d, J = 7.1 Hz, 2H), 6.51 (d, J = 8.8 Hz, 1H), 6.61 (dd, J = 8.8, 3.0 Hz, 1H), 6.85 (d, J = 2.9 Hz, 1H), 7.53 (dd, J = 8.9, 1.7 Hz, 1H), 7.87 - 7.96 (m, 1H), 8.12 (d, J = 1.6 Hz, 1H), 8.31 (d, J = 0.9 Hz, 1H) |
| | Composé 55 | MS : [M+H] = 472 |
| **Exemple 48** | 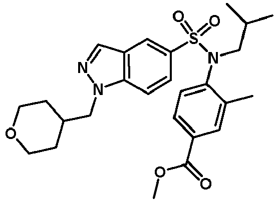 | **4-{1sobutyl-[1-(tétrahydro-pyran-4- ylméthyl)-1H-indazole-5-sulfonyl] - amino}-3-méthyl-benzoate de méthyle** |
| | | ¹H NMR (DMSO-d6) δ: 0.76 (d, J = 6.7 Hz, 3H), 0.94 (d, J = 6.6 Hz, 3H), 1.23 - 1.49 (m, 5H), 2.09 - 2.23 (m, 1H), 2.37 (s, 3H), 3.15 (dd, J = 13.2, 4.8 Hz, 1H), 3.24 (td, J = 11.2, 3.0 Hz, 2H), 3.46 (dd, J = 13.3, 8.9 Hz, 1H), 3.75 - 3.88 (m, 5H), 4.40 (d, J = 7.1 Hz, 2H), 6.80 (d, J = 8.3 Hz, 1H), 7.51 (dd, J = 8.9, 1.7 Hz, 1H), 7.65 (dd, J = 8.3, 2.2 Hz, 1H), 7.91 (d, J = 2.2 Hz, 1H), 7.93 (d, J = 8.9 Hz, 1H), 8.15 (d, J = 1.7 Hz, 1H), 8.32 (d, J = 0.9 Hz, 1H) |
| | Composé 56 | MS : [M+H] = 500 |
| **Exemple 49** | 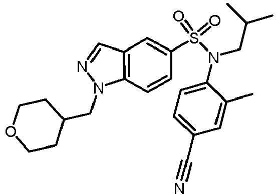 | **acide 1-(tetrahydro-pyran-4-ylméthyl)- 1H-indazole-5-sulfonique (4-cyano-2- méthyl-phenyl)-isobutyl-amide** |
| | | ¹H NMR (DMSO-d6) δ: 0.76 (d, J = 6.6 Hz, 3H), 0.93 (d, J = 6.5 Hz, 3H), 1.21 - 1.48 (m, 5H), 2.06 - 2.25 (m, 1H), 2.35 (s, 3H), 3.07 - 3.29 (m, 3H), 3.45 (dd, J = 13.3, 9.0 Hz, 1H), 3.82 (dd, J = 10.1, 3.2 Hz, 2H), 4.40 (d, J = 7.0 Hz, 2H), 6.88 (d, J = 8.3 Hz, 1H), 7.52 (dd, J = 9.0, 1.8 Hz, 1H), 7.59 (dd, J = 8.2, 2.1 Hz, 1H), 7.86 (d, J = 2.0 Hz, 1H), 7.94 (dd, J = 9.0, 0.9 Hz, 1H), 8.15 (d, J = 1.6 Hz, 1H), 8.33 (d, J = 1.0 Hz, 1H). |
| | Composé 57 | MS : [M+H] = 467 |

### Exemple 50 : Synthèse de l'acide 1-(tétrahydro-pyran-4-ylméthyl)-1H-indazole-5-sulfonique (4-éthyl-ohényl)-(tétrahydro-pyran-4-ylméthyl)-amide

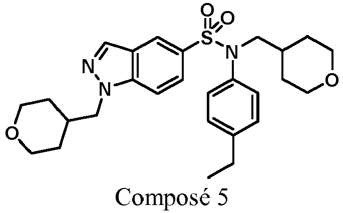

### 1. Synthèse de l'intermédiaire 50.1

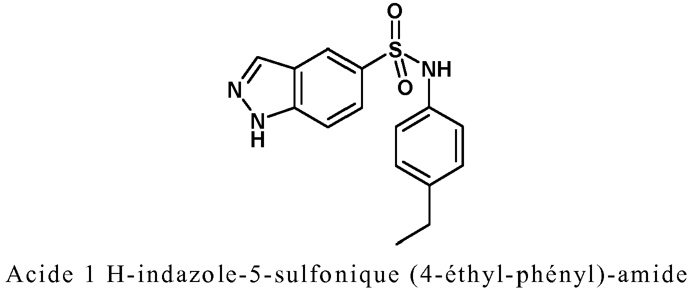

Un mélange de chlorure de 1H-indazole-5-sulfonyle (1.00 g; 4.39 mmol) de pyridine (5.0 ml) et de 4-éthylaniline (603 µl; 4.82 mmol) est agité 4heures à 50°C. Le milieu réactionnel est dilué à l'acétate d'éthyle et extrait. La phase organique est lavée avec une solution saturée de chlorure d'ammonium, une solution saturée d'hydrogénocarbonate de sodium et de l'eau. Elle est séchée (MgSO₄) filtrée et concentrée à sec.

Le produit brut est chromatographié sur gel de silice (éluant heptane/acétate d'éthyle de 0 à 40% d'acétate d'éthyle). L'acide 1H-Indazole-5-sulfonique (4-éthyl-phényl)-amide (1.32 g; 100%) obtenu sous la forme d'une huile orange avec une RMN¹H conforme
MS : [M+H] = 302

### 2. Synthèse du composé 5 selon l'invention

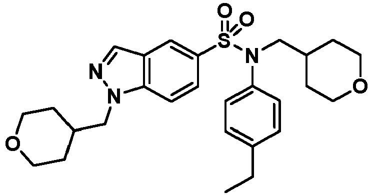

Un mélange d'acide 1H-indazole-5-sulfonique (4-éthyl-phényl)-amide (1.30 g; 4.31 mmol), de carbonate de césium (2.11 g; 6.47 mmol) et de 4-(bromomethyl)tétrahydropyrane (680 µl; 5.18 mmol; 1.20 eq.) dans la N-méthyl-2-pyrrolidone (10ml) est agité pendant 16 heures à une température de 50°C. Le milieu réactionnel est dilué à l'acétate d'éthyle (30ml). La phase organique est lavée avec une solution saturée de NH₄Cl (20ml), une solution saturée de NaHCO₃ (20ml), et de l'eau (20ml). La phase organique est séchée (MgSO₄), filtrée et concentrée.

Le produit brut est purifié par HPLC préparative (colonne C18, éluant : d'acétonitrile dans eau/0.1% d'acide formique). L'acide 1-(tétrahydro-pyran-4-ylmethyl)-1H-indazole-5-sulfonique (4-éthyl-phényl)-(tétrahydro-pyran-4-ylméthyl)-amide (360 mg; 17 %) est obtenu sous la forme d'un solide blanc.
¹H NMR (DMSO-d6) δ: 1.08 - 1.23 (m, 5H), 1.25 - 1.47 (m, 5H), 1.52 - 1.62 (m, 2H), 2.60 (q, J = 7.5 Hz, 2H), 3.13 (td, J = 11.6, 2.2 Hz, 2H), 3.23 (td, J = 11.3, 3.0 Hz, 2H), 3.43 (d, J = 7.2 Hz, 2H), 3.72 - 3.87 (m, 4H), 4.38 (d, J = 7.1 Hz, 2H), 6.97 (d, J = 8.4 Hz, 2H), 7.17 (d, J = 8.4 Hz, 2H), 7.46 (dd, J = 9.0, 1.7 Hz, 1H), 7.84 - 7.98 (m, 1H), 8.10 (d, J = 1.7 Hz, 1H), 8.29 (d, J = 0.9 Hz, 1H)
MS : [M+H] =498
- Une autre fraction est obtenue correspondant au mélange ci-après :

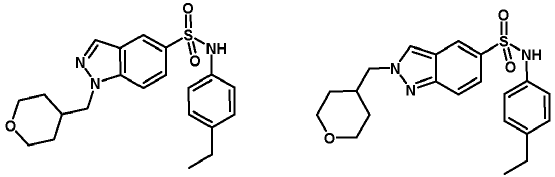

Intermédiaire 51.1 : Mélange de N-(4-éthylphényl)-1-((tétrahydro-2H-pyran-4-yl)méthyl)-1H-indazole-5-sulfonamide et de N-(4-éthylphényl)-2-((tétrahydro-2H-pyran-4-yl)méthyl)-2H-indazole-5-sulfonamide.

Le mélange de N-(4-éthylphényl)-1-((tétrahydro-2H-pyran-4-yl)méthyl)-1H-indazole-5-sulfonamide et de N-(4-éthylphényl)-2-((tétrahydro-2H-pyran-4-yl)methyl)-2H-indazole-5-sulfonamide (170.00 mg ; 10%) est obtenu sous la forme d'un solide avec une RMN¹H conforme
MS : [M+H] = 400

### Exemple 51 : Synthèse de l'acide 1-(tetrahydro-pyran-4-ylmethyl)-1H-indazole-5-sulfonique cyclopropyl-(4-éthyl-phényl)-amide

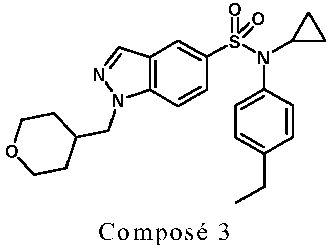

A un mélange de N-(4-éthylphényl)-1-((tétrahydro-2H-pyran-4-yl)méthyl)-1H-indazole-5-sulfonamide et de N-(4-éthylphényl)-2-((tétrahydro-2H-pyran-4-yl)méthyl)-2H-indazole-5-sulfonamide (170 mg; 0.43 mmol) de triéthylamine (180 µl; 1.28 mmol), d'acétate de cuivre (II) (232 mg; 1.28 mmol), d'acide cyclopropylboronique (220 mg; 2.55 mmol) dans du dichlorométhane (3ml) est ajouté une spatule de tamis moléculaire.

Le milieu réactionnel est agité 16 heures à température ambiante sous 1 atmosphère d'oxygène, filtré sur célite avec rinçage au dichlorométhane (50ml) et à l'eau (20ml). La phase organique est extraite, lavée avec un mélange (1/1) ammoniaque et solution saturée (NH₄Cl) (2X50ml) puis à l'eau (50ml), séchée (MgSO₄) filtrée et concentrée.

Le produit brut est purifié par HPLC préparative (colonne C18, éluant : d'acétonitrile dans eau/0.1% d'acide formique). L'acide 1-(tétrahydro-pyran-4-ylméthyl)-1H-indazole-5-sulfonique cyclopropyl-(4-éthyl-phényl)-amide002 (40mg; 21%) est obtenu sous la forme d'un solide blanc
¹H NMR (DMSO-d6) δ: 0.62 (t, J = 3.2 Hz, 2H), 0.76 (dt, J = 7.1, 3.6 Hz, 2H), 1.14 - 1.22 (m, 4H), 1.23 - 1.42 (m, 4H), 2.16 (dd, J = 10.8, 5.1 Hz, 1H), 2.58 - 2.71 (m, 3H), 3.17 - 3.29 (m, 3H), 3.76 - 3.89 (m, 2H), 4.38 (d, J = 7.1 Hz, 2H), 6.87 - 7.02 (m, 2H), 7.15 (d, J = 8.4 Hz, 2H), 7.46 (dd, J = 8.8, 1.7 Hz, 1H), 7.91 (d, J = 8.9 Hz, 1H), 8.15 (d, J = 1.6 Hz, 1H), 8.33 (d, J = 0.9 Hz, 1H)
MS : [M+H] = 440

### Exemple 52 : Synthèse de l'acide 1-(tétrahydro-pyran-4-ylméthyl)-1H-indazole-5-sulfonique isobutyl-(2-trifluorométhyl-phényl)-amide

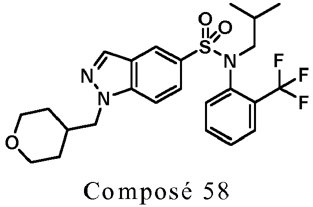

### 1. Synthèse de l'intermédiaire 52.1

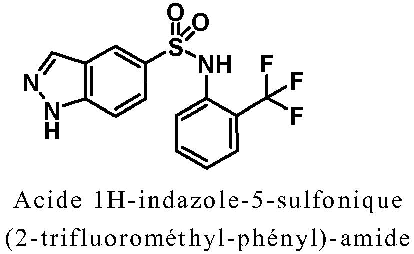

Un mélange de chlorure de 1H-indazole-5-sulfonyle (500.0 mg; 2.19 mmol) de pyridine (3.0 ml) et de 2-(trifluoromethyl)-aniline (306.18 µl; 2.41 mmol) est agité pendant 16 heures à une température de 40°C. Le milieu réactionnel est dilué à l'acétate d'éthyle et extrait. La phase organique est lavée avec une solution saturée de chlorure d'ammonium, une solution saturée d'hydrogénocarbonate de sodium et de l'eau. Elle est séchée (MgSO₄) filtrée et concentrée à sec.

Le produit brut est chromatographié sur gel de silice (éluant heptane/acétate d'éthyle de 0 à 40% d'acétate d'éthyle).

L'acide 1H-indazole-5-sulfonique (2-trifluorométhyl-phényl)-amide (300 mg; 40%) obtenu sous la forme d'un solide blanc avec une RMN¹H conforme
MS : [M+H] = 342

### 2. Synthèse du composé 58 selon l'invention

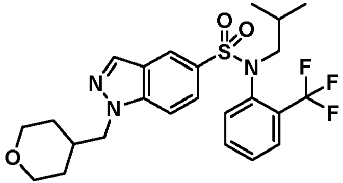

A une solution d'acide 1H-indazole-5-sulfonique (2-trifluorométhyl-phényl)-amide (300mg; 0.88 mmol) dans la 1-méthyl-2-pyrrolidone (3ml) sont ajoutés le carbonate de césium (30 mg; 1.32 mmol) et le 4-(bromométhyl)tétrahydropyrane (127µl; 0.97 mmol). Le milieu réactionnel est agité pendant 16 heures à température ambiante. Du 1-bromo-2-méthylpropane (287µl; 2.64 mmol) est ajouté et le milieu réactionnel est agité 6 heures à 80°C. Le milieu réactionnel est dilué avec de l'acétate d'éthyle, lavé avec une solution saturée de chlorure d'ammonium puis une solution saturée d'hydrogénocarbonate de sodium et à l'eau. La phase organique est séchée (MgSO₄), filtrée et concentrée à sec.

Le produit brut est purifié par HPLC préparative (colonne C18, éluant : d'acétonitrile dans eau/0.1% d'acide formique).L'acide 1-(tetrahydro-pyran-4-ylmethyl)-1H-indazole-5-sulfonique isobutyl-(2-trifluoromethyl-phenyl)- amide (80 mg; 16%) est obtenu sous la forme d'un solide beige.
¹H NMR (DMSO-d6) δ: 0.71 (d, J = 6.7 Hz, 3H), 0.85 - 0.93 (m, 3H), 1.25 - 1.43 (m, 4H), 1.48 (dtd, J = 8.8, 6.6, 4.6 Hz, 1H), 2.12 - 2.25 (m, 1H), 3.15 (dd, J = 13.4, 4.6 Hz, 1H), 3.24 (td, J = 10.8, 10.4, 2.2 Hz, 2H), 3.46 (dd, J = 13.4, 8.8 Hz, 1H), 3.78 - 3.86 (m, 2H), 4.41 (d, J = 7.1 Hz, 2H), 6.99 (dd, J = 5.7, 3.7 Hz, 1H), 7.56 (dd, J = 9.0, 1.7 Hz, 1H), 7.58 - 7.64 (m, 2H), 7.85 (dt, J = 5.6, 3.7 Hz, 1H), 7.96 (d, J = 8.9 Hz, 1H), 8.18 - 8.24 (m, 1H), 8.35 (s, 1H).
MS : [M+H] =496

### Exemple 53 : Synthèse de l'acide 1-(tétrahydro-pyran-4-ylméthyl)-1H-indazole-5-sulfonique isobutyl-(4-trifluorométhyl-phényl)-amide

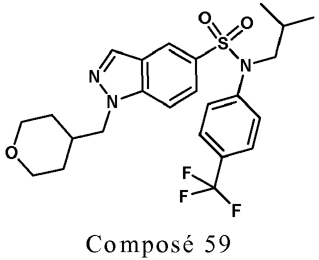

### 1. Synthèse de l'intermédiaire 53.1

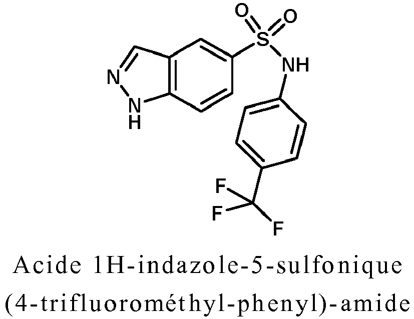

Un mélange de chlorure de 1H-indazole-5-sulfonyle (250.0 mg; 1.10 mmol) et de -4-(trifluoromethyl)-aniline (388.6 µl; 2.41 mmol) dans l'acétonitrile (1.7ml) est agité pendant 40 minutes à une température de 100°C sous micro-ondes. Le milieu réactionnel est dilué à l'acétate d'éthyle et extrait.

La phase organique est lavée avec une solution saturée de chlorure d'ammonium, une solution saturée d'hydrogénocarbonate de sodium et de l'eau. Elle est séchée (MgSO₄) filtrée et concentrée à sec.).

L'acide 1H-Indazole-5-sulfonique (4-trifluoromethyl-phenyl)-amide (297 mg; 79%) est obtenu sous la forme d'un solide jaunâtre avec une RMN¹H conforme
MS : [M+H] = 342

### 2. Synthèse du composé 59 selon l'invention

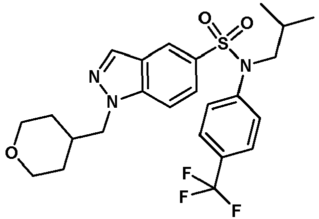

Avec un mode opératoire analogue à celui décrit pour l'exemple 52, on obtient l'acide 1-(tétrahydro-pyran-4-ylméthyl)-1H-indazole-5-sulfonique isobutyl-(4-trifluorométhyl-phényl)-amide (178 mg; 40%) est obtenu sous la forme d'un solide blanc.
¹H NMR (DMSO-d6) δ: 0.84 (d, J = 6.6 Hz, 6H), 1.22 - 1.51 (m, 5H), 2.07 - 2.24 (m, 1H), 3.23 (td, J = 11.4, 2.9 Hz, 2H), 3.42 (d, J = 7.3 Hz, 2H), 3.81 (ddd, J = 11.6, 4.2, 2.1 Hz, 2H), 4.37 (d, J = 7.1 Hz, 2H), 7.37 (d, J = 8.2 Hz, 2H), 7.43 (dd, J = 8.9, 1.8 Hz, 1H), 7.73 (d, J = 8.6 Hz, 2H), 7.91 (d, 1H), 8.13 (d, J = 1.6 Hz, 1H), 8.31 (d, J = 1.0 Hz, 1H).
MS : [M+H] =496

### Exemple 54 : Synthèse de l'acide 1-(tétrahydro-pyran-4-ylméthyl)-1H-indazole-5-sulfonique (2-cyano-4-méthyl-phenyl)-isobutyl-amide

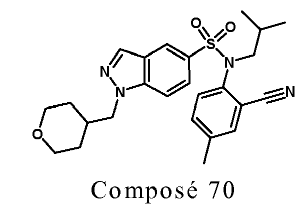

### 1. Synthèse de l'intermédiaire 54.1

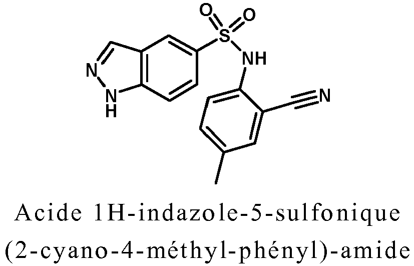

Avec un mode opératoire analogue à celui décrit pour l'intermédiaire 53.1, on obtient l'acide 1H-Indazole-5-sulfonique (2-cyano-4-méthyl-phényl)-amide (342.4 mg; 100 %) est obtenu sous forme d'un solide jaunâtre avec une RMN¹H conforme
MS : [M+H] = 313

### 2. Synthèse de l'acide 1-(tétrahydro-pyran-4-ylméthyl)-1H-indazole-5-sulfonique(2-cyano-4-méthyl-phenyl)-isobutyl-amide (composé 70)

Avec un mode opératoire analogue à celui décrit pour l'exemple 52, on obtient l'acide 1-(tétrahydro-pyran-4-ylméthyl)-2H-indazole-5-sulfonique (2-cyano-4-méthyl-phényl)-isobutyl-amide (129.7 mg; 20%) est obtenu sous la forme d'un solide blanc.
¹H NMR (Chloroforme-d) δ: 0.93 (d, J = 6.7 Hz, 6H), 1.42 - 1.53 (m, 4H), 1.54 - 1.67 (m, 1H), 2.21 - 2.39 (m, 1H), 2.42 (s, 3H), 3.37 (td, J = 11.3, 3.5 Hz, 2H), 3.45 (d, J = 7.2 Hz, 2H), 3.98 (dt, J = 11.7, 2.6 Hz, 2H), 4.32 (d, J = 7.2 Hz, 2H), 7.25 (d, J = 8.2 Hz, 1H), 7.41 (dd, J = 8.2, 2.1 Hz, 1H), 7.45 (d, J = 2.1 Hz, 1H), 7.52 (d, J = 8.9 Hz, 1H), 7.78 (dd, J = 8.9, 1.6 Hz, 1H), 8.15 (d, J = 1.0 Hz, 1H), 8.18 (d, J = 1.6 Hz, 1H).
MS : [M+H] =467

### Exemple 55 : Synthèse de l'acide 1-(tétrahydro-pyran-4-ylméthyl)-1H-indazole-5-sulfonique (4,6-diméthyl-pyridin-3-yl)-isobutyl-amide

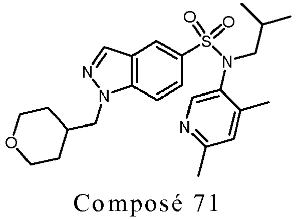

### 1. Synthèse de l'intermédiaire 55.1

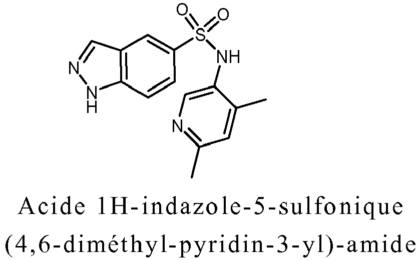

Avec un mode opératoire analogue à celui décrit pour l'intermédiaire 53.1, on obtient l'acide 1H-indazole-5-sulfonique (4,6-diméthyl-pyridin-3-yl)-amide (151 mg; 46 %) est obtenu sous forme d'un solide jaunâtre avec une RMN¹H conforme.
MS : [M+H] = 303

### 2. Synthèse de l'acide 1-(tetrahydro-pyran-4-ylmethyl)-1H-indazole-5-sulfonique (4,6-dimethyl-pyridin-3-yl)-isobutyl-amide

Avec un mode opératoire analogue à celui décrit l'exemple 52, on obtient l'acide 1-(tétrahydro-pyran-4-ylméthyl)-1H-indazole-5-sulfonique (4,6-diméthyl-pyridin-3-yl)-isobutyl-amide (17 mg; 7%) est obtenu sous la forme d'un solide blanc.
¹H NMR (DMSO-d6) δ: 0.78 (d, J = 6.7 Hz, 3H), 0.95 (d, J = 6.6 Hz, 3H), 1.18 - 1.56 (m, 5H), 2.13 - 2.27 (m, 4H), 2.41 (s, 3H), 3.19 - 3.29 (m, 3H), 3.44 (dd, J = 13.3, 8.6 Hz, 1H), 3.78 - 3.89 (m, 2H), 4.40 (d, J = 7.0 Hz, 2H), 7.21 (s, 1H), 7.56 (dd, J = 8.9, 1.8 Hz, 1H), 7.68 (s, 1H), 7.96 (d, J = 8.8 Hz, 1H), 8.16 (d, J = 1.7 Hz, 1H), 8.33 (s, 1H).
MS : [M+H] =457

### Exemple 57: Synthèse de l'acide 1-(tétrahydro-pyran-4-ylméthyl)-1H-indazole-5-sulfonique (2-fluoro-4-méthyl-phényl)-isobutyl-amide

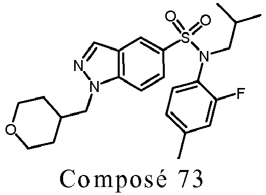

### 1. Synthèse de l'intermédiaire 57.1

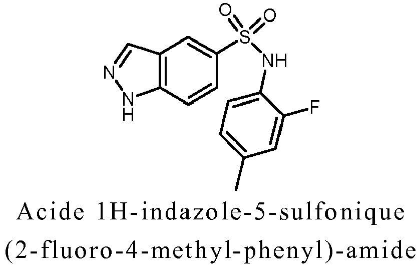

Avec un mode opératoire analogue à celui décrit pour l'intermédiaire 53.1, on obtient l'acide 1H-indazole-5-sulfonique (2-fluoro-4-méthyl-phényl)-amide (189 mg; 94 %) est obtenu sous forme d'un solide jaunâtre avec une RMN¹H conforme.
MS : [M+H] = 306

### 2. Synthèse de l'acide 1-(tetrahydro-pyran-4-ylmethyl)-1H-indazole-5-sulfonique (2-fluoro-4-methyl-phenyl)-isobutyl-amide.

Avec un mode opératoire analogue à celui décrit pour l'exemple 52, on obtient l'acide 1-(tétrahydro-pyran-4-ylméthyl)-1H-indazole-5-sulfonique (2-fluoro-4-méthyl-phényl)-isobutyl-amide (19 mg; 4%) est obtenu sous la forme d'un solide blanc.
¹H 1H NMR (Chloroforme-d) δ: 0.93 (d, J = 6.6 Hz, 6H), 1.47 (td, J = 11.2, 10.7, 4.2 Hz, 3H), 1.54 - 1.69 (m, 2H), 2.30 (dd, J = 10.7, 4.6 Hz, 1H), 2.36 (s, 3H), 3.24 - 3.47 (m, 5H), 3.95 - 4.03 (m, 2H), 4.31 (d, J = 7.1 Hz, 2H), 6.82 (dd, J = 11.5, 1.8 Hz, 1H), 6.94 (dd, J = 8.2, 1.8 Hz, 1H), 7.16 (t, J = 8.1 Hz, 1H), 7.45 (dt, J = 9.0, 0.9 Hz, 1H), 7.67 (dd, J = 8.9, 1.6 Hz, 1H), 8.13 (dd, J = 9.3, 1.2 Hz, 2H).
MS : [M+H] =460

### Partie II : Synthèse des sulfonamides bicycliques à partir du schéma réactionnel 2

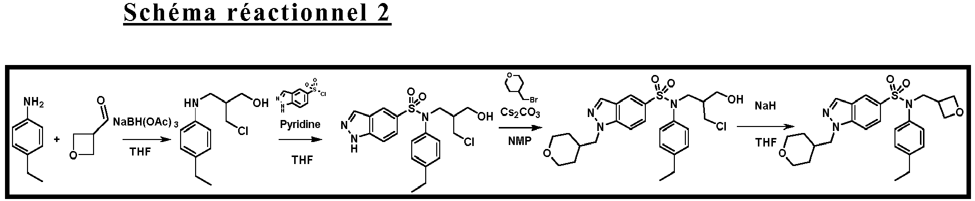

### Exemple 58 : Synthèse de l'acide 1-(tetrahydro-pyran-4-ylmethyl)-1H-indazole-5-sulfonique (4-ethyl-phenyl)-oxetan-3-ylmethyl-amide

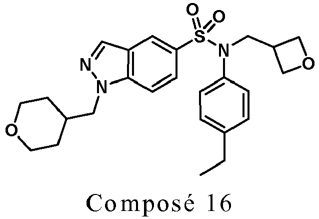

### 1. Synthèse de l'intermédiaire 58.1

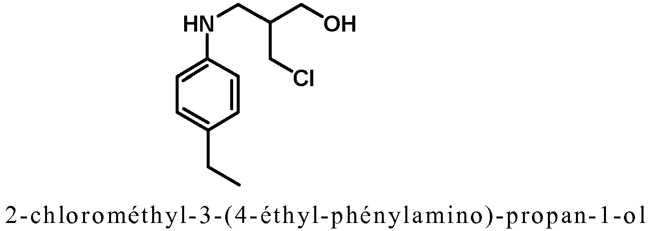

Un mélange de 4-éthylaniline (513 µl; 4.13 mmol), d'oxetane-3-carbaldéhyde (807.3 mg; 3.75 mmol) et de tétrahydrofurane (2.5 ml) est agité pendant 2 heures à température ambiante. Le triacétoxyborohydrure de sodium (1.19 g; 5.63 mmol) est ajouté et le milieu réactionnel est agité pendant 16 heures à température ambiante, hydrolysé et extrait à l'acétate d'éthyle.

Les phases organiques sont rassemblées, lavées à la saumure, séchées (Na₂SO₄), concentrées.

Le produit brut est chromatographié sur gel de silice (éluant Heptane/Acétate d'éthyle de 0 à 40% d'acétate d'éthyle). Le 2-chlorométhyl-3-(4-éthyl-phénylamino)-propan-1-ol (340mg; 40%) est obtenu sous la forme d'une huile orange avec une RMN¹H montrant l'ouverture de l'oxétane.
MS : [M+H] = 228

### 2. Synthèse de l'intermédiaire 58.2

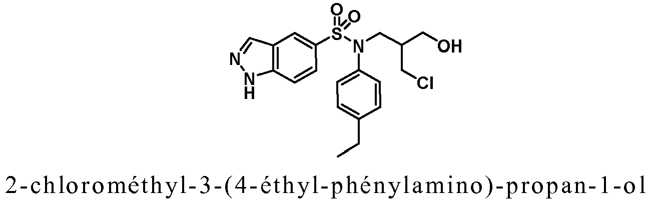

Le mélange de chlorure de 1H-indazole-5-sulfonyle (368.5 mg; 1.62 mmol) de pyridine (3.0 ml; 37.17 mmol) et de (4-éthyl-phényl)-oxetan-3-ylméthyl-amine (340 mg; 1.78 mmol), est agité pendant 30 minutes à une température de 100°C sous micro-onde.

Le milieu réactionnel est dilué à l'acétate d'éthyle. La phase organique est lavée avec une solution saturée de chlorure d'ammonium, une solution saturée d'hydrogénocarbonate de sodium et de l'eau. Elle est séchée (MgSO₄) filtrée et concentrée à sec.

Le produit brut est chromatographié sur gel de silice (éluant heptane/acétate d'éthyle de 0 à 40% d'acétate d'éthyle). L'acide 1H-indazole-5-sulfonique (2-chlorométhyl-3-hydroxy-propyl)-(4-éthyl-phényl)-amide (260mg; 39%) est obtenu sous la forme d'une huile incolore avec une RMN¹H conforme.
MS : [M+H] = 408

### 3. Synthèse de l'intermédiaire 58.3

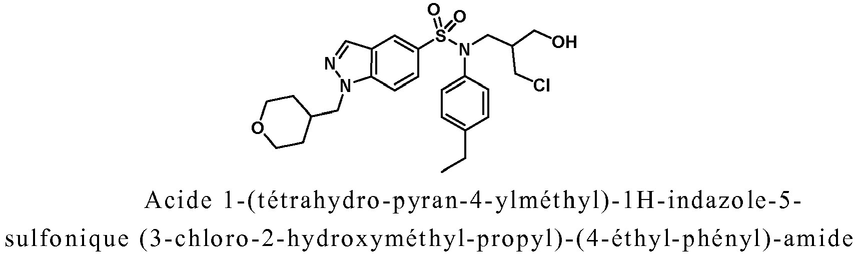

Un mélange d'acide 1H-indazole-5-sulfonique (2-chlorométhyl-3-hydroxy-propyl)-(4-éthyl-phényl)-amide (1.26g; 0.64mmol), de carbonate de césium (0.31mg; 0.96mmol) et de 4-(bromomethyl)-tétrahydropyrane (100 µl; 0.76 mmol) dans la N-méthyl-2-pyrrolidone (4 ml) est agité pendant 1 heure à une température de 80°C. Le milieu réactionnel est dilué à l'acétate d'éthyle (20ml).

La phase organique est lavée avec une solution saturée de NH₄Cl, une solution saturée de NaHCO₃, et de l'eau. La phase organique est séchée (MgSO₄), filtrée et concentrée.

Le produit brut est purifié par HPLC préparative (colonne C18, éluant : d'acétonitrile dans eau/0.1% d'acide formique). L'acide 1-(tétrahydro-pyran-4-ylméthyl)-1H-indazole-5-sulfonique (3-chloro-2-hydroxyméthyl-propyl)-(4-éthyl-phényl)-amide (100 mg; 31%) est obtenu sous la forme d'une huile incolore.
¹H NMR (DMSO-d6) δ: 1.18 (t, J = 7.6 Hz, 3H), 1.25 - 1.43 (m, 4H), 1.73 (p, J = 6.4 Hz, 1H), 2.61 (q, J = 7.6 Hz, 2H), 3.23 (td, J = 11.3, 3.0 Hz, 2H), 3.35 - 3.41 (m, 1H), 3.46 - 3.52 (m, 1H), 3.52 - 3.63 (m, 2H), 3.69 (qd, J = 10.8, 4.9 Hz, 2H), 3.82 (ddd, J = 11.4, 4.3, 2.2 Hz, 2H), 4.39 (d, J = 7.0 Hz, 2H), 4.69 (t, J = 5.1 Hz, 1H), 6.95 - 7.02 (m, 2H), 7.16 - 7.22 (m, 2H), 7.44 (dd, J = 9.0, 1.8 Hz, 1H), 7.92 (d, J = 8.9 Hz, 1H), 8.10 (d, J = 1.6 Hz, 1H), 8.31 (s, 1H)
MS : [M+H] = 506

### 4. Synthèse du composé 16 selon l'invention

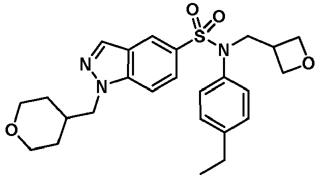

A une solution d'acide 1-(tétrahydro-pyran-4-ylméthyl)-1H-indazole-5-sulfonique (3-chloro-2-hydroxy-méthyl-propyl)-(4-éthyl-phényl)-amide (100 mg; 0.20 mmol) dans du tétrahydrofurane (3ml) est ajouté de l'hydrure de sodium 60% (17.4 mg; 0.43 mmol). Le milieu réactionnel est agité pendant 1 heure à une température de 80°C puis pendant 16 heures à une température de 30°C.

Le milieu réactionnel est dilué à l'acétate d'éthyle (20ml). La phase organique est lavée avec une solution saturée de NH₄Cl (20ml), une solution saturée de NaHCO₃ (20ml), et de l'eau (20ml). La phase organique est séchée (MgSO₄), filtrée et concentrée.

Le produit brut est purifié par HPLC préparative (colonne C18, éluant : d'acétonitrile dans eau/0.1% d'acide formique). L'acide 1-(tétrahydro-pyran-4-ylméthyl)-1H-indazole-5-sulfonique (4-éthyl-phényl)-oxetan-3-ylméthyl-amide (35.0 mg; 33%) sous la forme d'un solide blanc.
¹H NMR (Méthanol-d4) δ: 1.20 (t, J = 7.6 Hz, 3H), 1.31 - 1.51 (m, 5H), 2.27 (tq, J = 10.6, 6.2, 5.6 Hz, 1H), 2.62 (q, J = 7.6 Hz, 2H), 3.01 (hept, J = 7.3 Hz, 1H), 3.31 - 3.43 (m, 2H), 3.92 (d, J = 7.7 Hz, 3H), 4.33 - 4.42 (m, 3H), 4.56 - 4.66 (m, 2H), 6.88 (d, J = 8.2 Hz, 2H), 6.93 - 7.03 (m, 1H), 7.13 (d, J = 8.1 Hz, 2H), 7.58 (dd, J = 8.9, 1.7 Hz, 1H), 7.74 (d, J = 8.8 Hz, 1H), 8.13 (s, 1H)
MS : [M+H] = 470

### Partie III : Synthèse des sulfonamides bicycliques à partir du schéma réactionnel 3

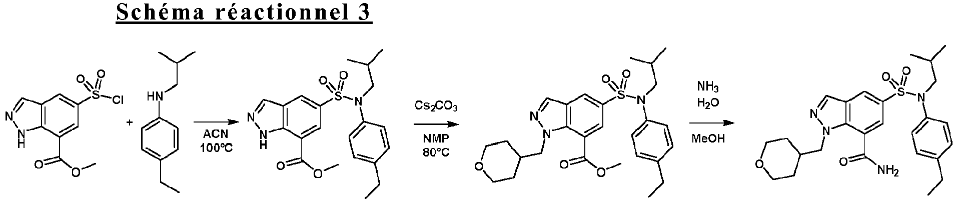

### Exemple 59 : Synthèse du 5-[(4-éthyl-phényl)-isobutyl-sulfamoyl]-1-(tétrahydro-pyran-4-ylméthyl)-1H-indazole-7-carboxylate de méthyle (intermédiaire ne faisant pas partie de l'invention)

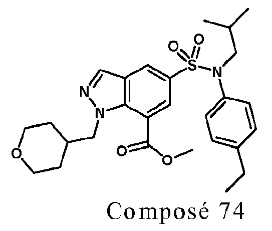

### 1. Synthèse de l'intermédiaire 59.1

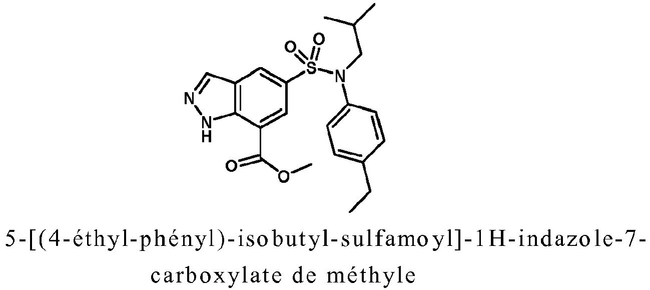

La (4-éthyl-phényl)-isobutyl-amine (417.5 mg; 2.35 mmol) est ajouté à du 5-chlorosulfonyl-1H-indazole-7-carboxylate de méthyle (300 mg; 1.07 mmol) dans de l'acétonitrile (4ml). Le milieu réactionnel est agité pendant 1 heure et 20 minutes à une température de 100°C sous micro-ondes.

Le milieu réactionnel est dilué à l'acétate d'éthyle, lavé avec une solution d'acide chlorhydrique 1N, puis une solution saturée d'hydrogénocarbonate de sodium et à l'eau, séché (MgSO₄), filtré et concentré.

La 5-[(4-éthyl-phényl)-isobutyl-sulfamoyl]-1H-indazole-7-carboxylate de méthyle (429.8 mg; 97 %) est obtenu sous forme de solide blanc avec une RMN¹H conforme.
MS : [M+H] = 416

### 2. Synthèse du 5-[(4-éthyl-phényl)-isobutyl-sulfamoyl]-1-(tetrahydro-pyran-4-ylmethyl)-1H-indazole-7-carboxylate de méthyle (composé 74)

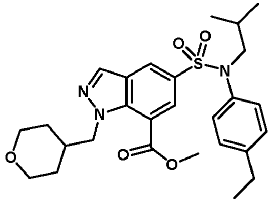

Le 4-(bromomethyl)tétrahydropyrane (97.6 µl; 0.74 mmol) est ajouté à un mélange de 5-[(4-éthyl-phényl)-isobutyl-sulfamoyl]-1H-indazole-7-carboxylate de méthyle (280.0 mg; 0.67 mmol), de carbonate de césium (329 mg; 1 mmol) dans de la 1-méthyl-2-pyrrolidone (2.8 ml). Le milieu réactionnel est agité à une température de 80°C pendant une nuit.

Le produit brut est filtré puis purifié par HPLC préparative (colonne C18, éluant : acétonitrile dans eau/0.1% d'acide formique). Le 5-[(4-éhyl-phényl)-isobutyl-sulfamoyl]-1-(tétrahydro-pyran-4-ylmethyl)-1H-indazole-7-carboxylate de méthyle (223.4 mg; 63%) est obtenue sous forme d'un solide blanc.
¹H NMR (DMSO-d6) δ: 0.85 (d, J = 6.6 Hz, 6H), 1.18 (t, J = 7.6 Hz, 3H), 1.25 (dt, J = 11.1, 5.3 Hz, 4H), 1.33 - 1.55 (m, 1H), 1.86 - 2.12 (m, 1H), 2.60 (q, J = 7.6 Hz, 2H), 3.18 (td, J = 11.1, 3.6 Hz, 2H), 3.35 (s, 2H), 3.79 (dt, J = 11.4, 3.3 Hz, 2H), 3.93 (s, 3H), 4.57 (d, J = 7.2 Hz, 2H), 6.93 - 7.06 (m, 2H), 7.12 - 7.23 (m, 2H), 7.80 (d, J = 1.7 Hz, 1H), 8.37 (d, J = 1.9 Hz, 1H), 8.48 (s, 1H)
MS : [M+H] = 514

### Exemple 60 : Synthèse de l'acide 5-[(4-Ethyl-phenyl)-isobutyl-sulfamoyl]-1-(tetrahydro-pyran-4-ylmethyl)-1H-indazole-7-carboxylique amide

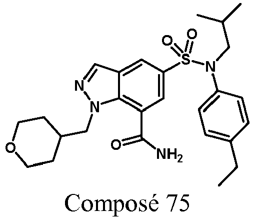

De l'ammoniaque (1.05 ml) est ajouté à une solution de 5-[(4-éthyl-phényl)-isobutyl-sulfamoyl]-1-(tétrahydro-pyran-4-ylméthyl)-1H-indazole-7-carboxylate de méthyle (210.0 mg; 0.41 mmol) dans du N,N-diméthylformamide (1.05 ml).

Le milieu réactionnel est agité pendant 3 jours à température ambiante et 2 jours à une température de 60°C. Le milieu réactionnel est dilué et extrait avec du dichlorométhane. Les phases organiques sont rassemblées et séchées (MgSO₄), filtrées et concentrée à sec.

Le produit brut est filtré puis purifié par HPLC préparative (colonne C18, éluant : acétonitrile dans eau/0.2% de carbonate d'ammonium). L'amide 5-[(4-Ethyl-phenyl)-isobutyl-sulfamoyl]-1-(tetrahydro-pyran-4-ylmethyl)-1H-indazole-7-carboxylique est obtenu sous forme d'un solide blanc
¹H NMR (Chloroforme-d) δ: 0.94 (d, J = 6.7 Hz, 6H), 1.25 (t, J = 7.6 Hz, 3H), 1.38 - 1.51 (m, 4H), 1.59 - 1.69 (m, 1H), 2.08 - 2.21 (m, 1H), 2.67 (q, J = 7.6 Hz, 2H), 3.25 - 3.39 (m, 4H), 3.90 - 4.01 (m, 2H), 4.59 (d, J = 7.2 Hz, 2H), 5.75 (s, 1H), 6.00 (s, 1H), 6.94 - 7.00 (m, 2H), 7.11 - 7.18 (m, 2H), 7.62 (d, J = 1.7 Hz, 1H), 8.15 (d, J = 1.6 Hz, 1H), 8.19 (s, 1H)
MS : [M+H] = 499

### Partie IV : Synthèse des sulfonamides bicycliques à partir du schéma réactionnel 4

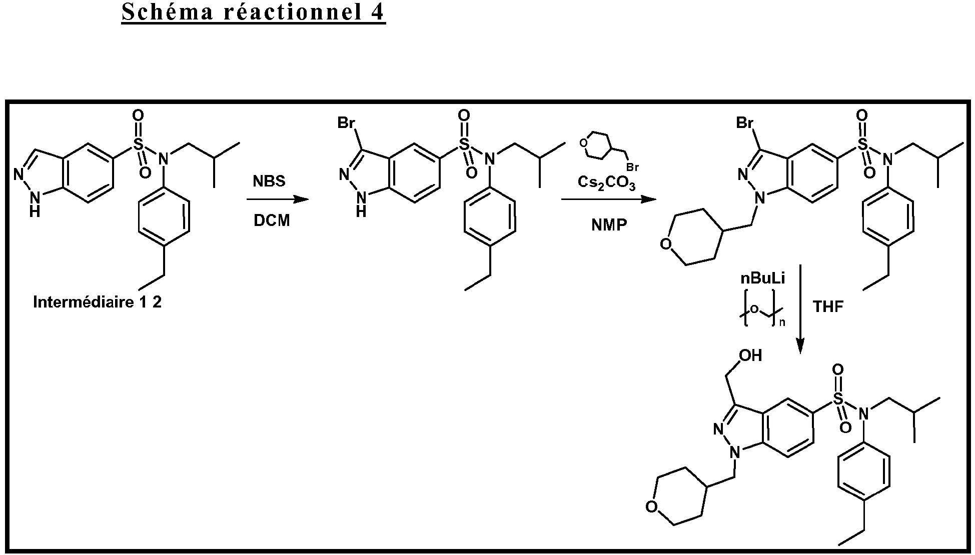

### Exemple 61 : Synthèse du N-(4-éthylphényl)-N-isobutyl-1-((tetrahydro-2H-pyran-4-yl)méthyl)-1H-indazole-5-sulfonamide

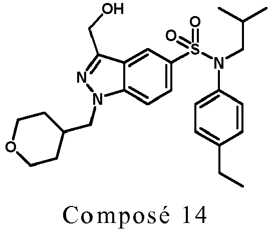

### 1. Synthèse de l'intermédiaire 60.1

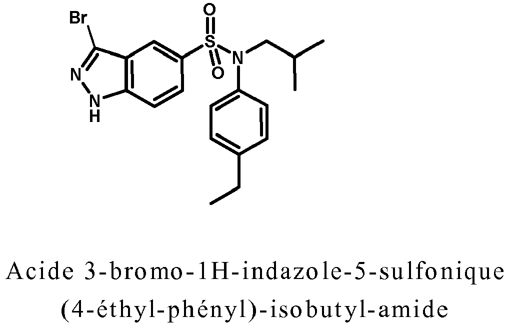

A une solution d'acide 1H-indazole-5-sulfonique (4-éthyl-phényl)-isobutyl-amide (200mg; 0.56 mmol) dans le dichlorométhane (5ml) est ajouté le N-bromosuccinimide (120 mg; 0.67 mmol). Le milieu réactionnel est agité pendant 16 heures à température ambiante, dilué avec du dichlorométhane et extrait.

La phase organique est lavée avec une solution saturée de NH₄Cl, une solution saturée de NaHCO₃ et de l'eau, séchée (MgSO₄), filtrée et concentrée à sec.

L'acide 3-bromo-1H-indazole-5-sulfonique (4-éthyl-phényl)-isobutyl-amide (260 mg; 100 %) sous la forme d'un solide jaune pâle avec une RMN¹H conforme.
MS : [M+H] = 436

### 2. Synthèse de l'intermédiaire 60.2

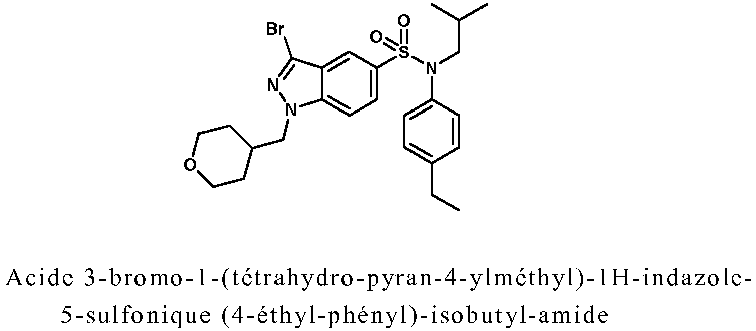

Un mélange d'acide 3-bromo-1H-indazole-5-sulfonique (4-éthyl-phényl)-isobutyl-amide (260 mg; 0.60 mmol.), de carbonate de césium (388 mg; 1.19 mmol) et de 4-(bromométhyl)-tétrahydropyrane (160 mg; 0.89 mmol) dans de la N-méthyl-2-pyrrolidone (3 ml) est agité pendant 1 heure à une température de 80°C.

Le milieu réactionnel est dilué à l'acétate d'éthyle (30ml). La phase organique est lavée avec une solution saturée de NH₄Cl (20ml), une solution saturée de NaHCO₃ (20ml) et de l'eau (20ml), séchée (MgSO₄), filtrée et concentrée à sec.

L'acide 3-bromo-1-(tétrahydro-pyran-4-ylméthyl)-1H-indazole-5-sulfonique (4-éthyl-phényl)-isobutyl-amide (350 mg; 100%) est obtenu sous la forme d'une huile jaune claire avec une RMN¹H conforme.
MS : [M+H] = 534

### 3. Synthèse du composé 14 selon l'invention

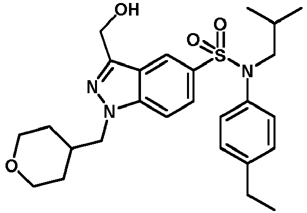

Sous argon, sur une solution d'acide 3-bromo-1-(tétrahydro-pyran-4-ylméthyl)-1H-indazole-5-sulfonique (4-éthyl-phényl)-isobutyl-amide (200 mg; 0.37 mmol) dans le tétrahydrofurane (3 ml) à une température de -78°C, est additionné le N-butyllithium 2.5 M (900 µl; 2.24 mmol). Le milieu réactionnel est agité pendant 30 minuntes avant d'ajouter le paraformaldehyde (980 mg; 2.24 mmol), de laisser sa température revenir à température ambiante puis d'agiter pendant 3 heures à une température de 60°C.

Le milieu réactionnel hydrolysé avec une solution d'acide chlorhydrique 1M (5ml) à température ambiante pendant 10 jours, dilué à l'acétate d'éthyle (50ml).

La phase organique est lavée avec une solution saturée de NH₄Cl (20ml), une solution saturée NaHCO₃ (20ml) et de l'eau (20ml), séchée (MgSO₄), filtrée et concentrée à sec.

Le produit brut est purifié par HPLC préparative (colonne C18, éluant : d'acétonitrile dans eau/0.1% d'acide formique). L'acide 3-hydroxymethyl-1-(tétrahydro-pyran-4-ylméthyl)-1H-indazole-5-sulfonique (4-éthyl-phényl)-isobutyl-amide (20mg; 11 %) est obtenu sous la forme d'une huile incolore
¹H NMR (CD30D-*d4*) δ: 0.90 (d, J = 6.7 Hz, 7H), 1.22 (t, J = 7.6 Hz, 3H), 1.27 - 1.58 (m, 4H), 1.71 (d, J = 12.7 Hz, 2H), 2.16 (s, 1H), 2.64 (q, J = 7.7 Hz, 2H), 3.35 (s, 2H), 3.41 (t, J = 11.6 Hz, 2H), 3.96 (dd, J = 11.9, 4.1 Hz, 2H), 4.66 (s, 2H), 6.92 (d, J = 9.0 Hz, 1H), 7.00 (d, J = 7.9 Hz, 2H), 7.16 (d, J = 8.0 Hz, 2H), 7.44 (d, J = 8.6 Hz, 1H), 8.03 (d, J = 2.4 Hz, 1H)
MS : [M+H] = 486

### Partie V : Synthèse des sulfonamides bicycliques à partir du schéma réactionnel 5

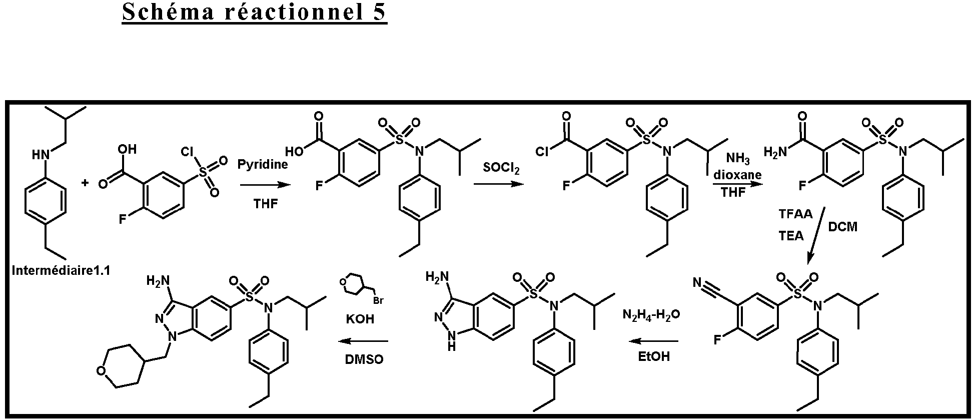

### Exemple 62 : Synthèse de l'acide 3-amino-1H-indazole-5-sulfonique (4-éthyl-ohényl)-isobutyl-amide

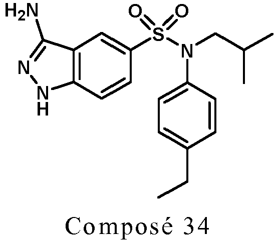

### 1. Synthèse de l'intermédiaire 61.1

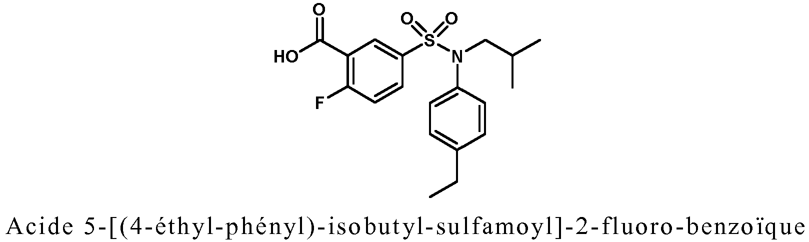

A une solution d'acide 5-chlorosulfonyl-2-fluoro-benzoïque (1.44 g; 5.87 mmol) dans du tétrahydrofurane (8 ml) est ajoutée une solution de (4-éthyl-phényl)-isobutyl-amine (0.80 g; 4.51 mmol) et de pyridine (0.36 ml; 4.51 mmol) dans du tétrahydrofurane (8 ml).

Le milieu réactionnel agité pendant 19 heures à température ambiante, hydrolysé avec une solution aqueuse de HCl 1N et dilué à l'acétate d'éthyle.

La phase organique est lavée avec une solution aqueuse de HCl 1N. La phase organique est séchée (Na₂SO₄), filtrée et concentrée.

Le produit brut est chromatographié sur gel de silice (éluant dichlorométhane/méthanol de 0 à 10% de méthanol) puis par HPLC préparative (colonne C18, éluant : de 60% à 70% d'acétonitrile dans eau/0.1% d'acide formique). Le 5-[(4-éthyl-phényl)-isobutyl-sulfamoyl]-2-fluoro-benzoic acide (0.56 g; 33%) est obtenu sous la forme d'un solide blanc cassé avec une RMN¹H conforme.
MS : [M-H] = 378

### 2. Synthèse de l'intermédiaire 61.2

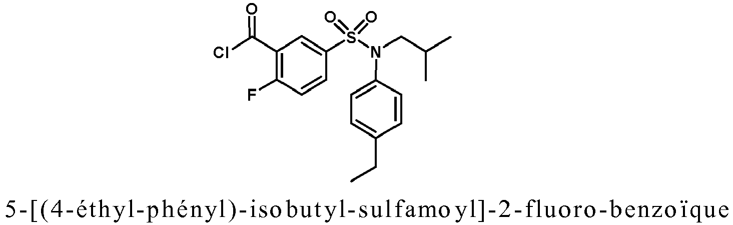

Une solution d'acide 5-[(4-éthyl-phényl)-isobutyl-sulfamoyl]-2-fluoro-benzoïque (0.59 g; 1.55 mmol) dans du chlorure de thionyle (5.0 ml) est agité pendant 3 heures au reflux puis est concentrée à sec. Le chlorure de 5-[(4-éthyl-phényl)-isobutyl-sulfamoyl]-2-fluoro-benzoyle (0.62 g; 100%) est obtenu sous la forme d'une huile brune.
MS : [M-H] = 397

### 3. Synthèse de l'intermédiaire 61.3

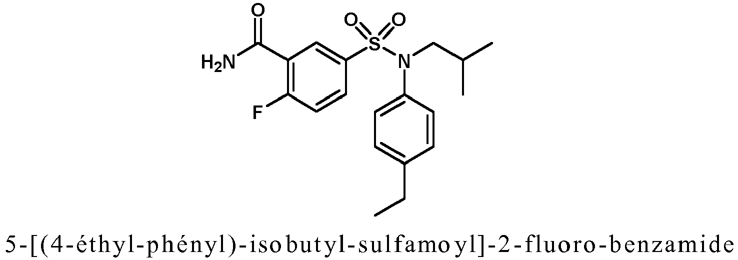

A une solution de chlorure de 5-[(4-éthyl-phényl)-isobutyl-sulfamoyl]-2-fluoro-benzoyle (0.31 g; 0.78 mmol) dans du tétrahydrofurane (3 ml) est ajoutée par fraction une solution d'ammoniac 0.5M dans du dioxanne (8.4 ml; 4.2 mmol) sur une période de 43 heures. Le milieu réactionnel est hydrolysé avec une solution aqueuse (NaHCO₃) et de l'acétate d'éthyle.

La phase organique extraite est lavée avec une solution aqueuse de soude 1N, séchée (Na₂SO₄), filtrée et concentrée. Le brut est chromatographié sur gel de silice (éluant heptane/acétate d'éthyle de 20 à 50% d'acétate d'éthyle). Le 5-[(4-éthyl-phényl)-isobutyl-sulfamoyl]-2-fluoro-benzamide (0.17 g; 58 %) est obtenu sous la forme d'un solide blanc avec une RMN¹H conforme.
MS : [M-H] = 379

### 4. Synthèse de l'intermédiaire 61.4

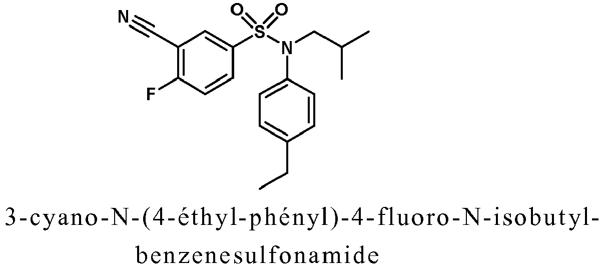

A une solution de 5-[(4-éthyl-phényl)-isobutyl-sulfamoyl]-2-fluoro-benzamide (0.17 g; 0.45 mmol) à 0°C sont ajoutés de la triethylamine (0.19 ml; 1.35 mmol) puis de l'anhydride trifluoroacétique (93 µl; 0.67 mmol.). Le milieu réactionnel est agité pendant 1 heure, hydrolysé pendant 15 minutes, extrait à l'acétate d'éthyle. Les phases organiques sont réunies, séchées (Na₂SO₄), filtrées et concentrées.

Le 3-cyano-N-(4-éthy-phényl)-4-fluoro-N-isobutyl-benzenesulfonamide (0.17 g; 89 %) est obtenu sous la forme d'un solide blanc (pureté HPLC = 85%).avec une RMN¹H conforme.
MS : [M-H] = 361

### 5. Synthèse du composé 34 selon l'invention

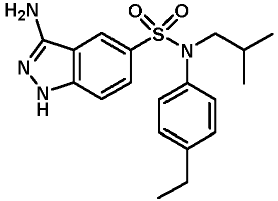

A une suspension de 3-cyano-N-(4-éthyl-phényl)-4-fluoro-N-isobutyl-benzenesulfonamide (0.19 g; 0.45 mmol) dans de l'éthanol (2.5 ml) est ajoutée de l'hydrazine hydrate (1.25 ml; 25.7 mmol). Le milieu réactionnel est agité pendant 50 minutes à une température de 100°C dans un tube. Le milieu réactionnel est concentré.

Le produit brut est purifié par HPLC préparative (colonne C18, éluant : acétonitrile dans eau/0.2% de carbonate d'ammonium). L'acide 3-amino-1H-indazole-5-sulfonique (4-éthyl-phényl)-isobutyl-amide (78mg; 53%) est obtenu sous la forme d'un solide.
¹H NMR (DMSO-d6) δ: 0.85 (d, J = 6.6 Hz, 6H), 1.17 (t, J = 7.5 Hz, 3H), 1.34 - 1.48 (m, 1H), 2.59 (q, J = 7.7 Hz, 2H), 3.28 - 3.32 (m, 2H), 5.73 (s, 2H), 6.96 (d, J = 7.8 Hz, 2H), 7.16 (d, J = 7.8 Hz, 2H), 7.26 (d, J = 8.8 Hz, 1H), 7.32 (d, J = 8.8 Hz, 1H), 8.19 (s, 1H), 11.91 (s, 1H).
MS : [M-H] = 373

### Exemple 63 : Synthèse de l'acide 3-amino-1H-indazole-5-sulfonique (4-ethyl-phenyl)-isobutyl-amide

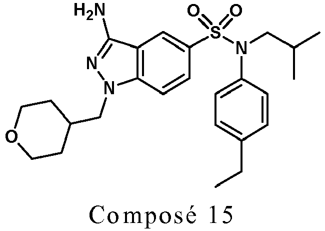

A de l'hydroxyde de potassium (29 mg; 0.44 mmol) à 85% dans du diméthylsufoxide (0.20 ml) est ajouté de l'acide 3-amino-1H-indazole-5-sulfonique (4-éthyl-phényl)-isobutyl-amide (54 mg; 0.14 mmol) dans du diméthylsufoxide (0.80 ml). Le milieu réactionnel est agité pendant 5 minutes avant d'ajouter le 4-(bromométhyl)tétrahydropyrane (27 mg; 0.15 mmol.) dans du diméthylsufoxide (0.50 ml). L'agitation est poursuivie pendant 3 heures et 20 minutes avant hydrolyse avec quelques gouttes d'eau et filtration sur filtre seringue. Le filtrat est purifié par HPLC préparative (colonne C18, éluant : acétonitrile dans eau/0.1% d'acide formique).

L'acide 3-amino-1-(tétrahydro-pyran-4-ylméthyl)-1H-indazole-5-sulfonique (4-éthyl-phényl)-isobutyl-amide (30mg; 44%) est obtenu sous la forme d'une huile incolore.
¹H NMR (DMSO-d6) δ: 0.85 (d, J = 6.6 Hz, 6H), 1.17 (t, J = 7.6 Hz, 3H), 1.21 - 1.47 (m, 5H), 2.59 (q, J = 7.6 Hz, 2H), 3.23 (td, J = 11.5, 2.4 Hz, 2H), 3.77 - 3.88 (m, 2H), 4.03 (d, J = 7.0 Hz, 2H), 6.92 - 7.01 (m, 2H), 7.16 (d, J = 8.3 Hz, 2H), 7.26 (dd, J = 8.9, 1.8 Hz, 1H), 7.51 (d, J = 8.9 Hz, 1H), 8.17 (d, J = 1.7 Hz, 1H).
MS : [M-H] = 471

### Partie VI : Synthèse des sulfonamides bicycliques à partir du schéma réactionnel 6

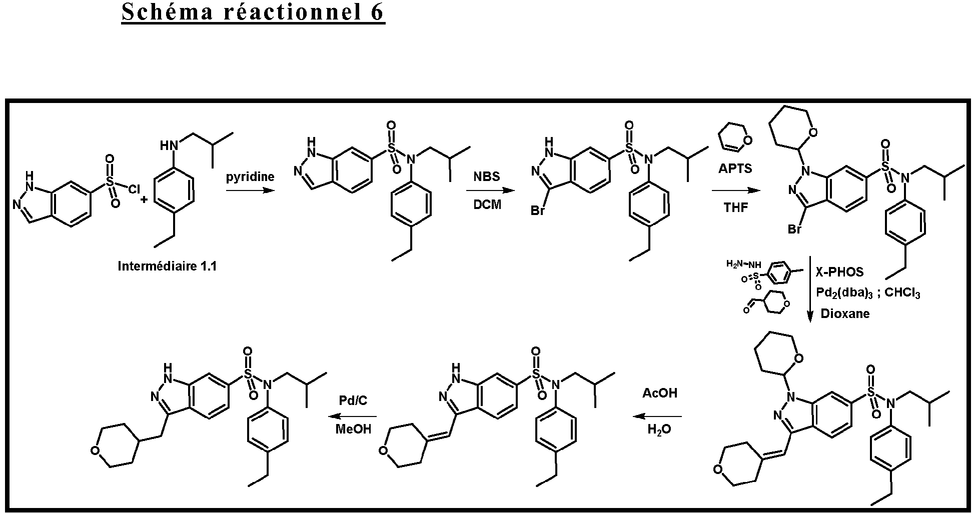

### Exemple 64 : Synthèse de l'acide 3-(tétrahydro-pyran-4-ylideneméthyl)-1H-indazole-6-sulfonique (4-éthyl-ohényl)-isobutyl-amide

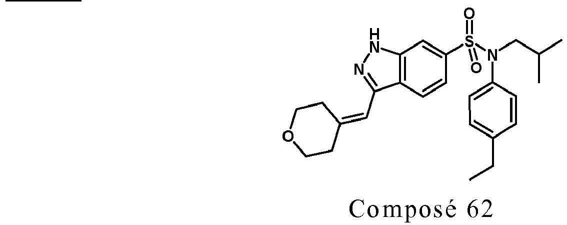

### 1. Synthèse de l'intermédiaire 63.1

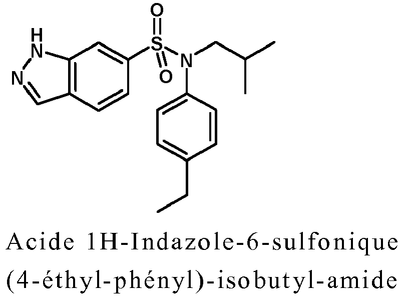

Un mélange de chlorure de 1H-indazole-6-sulfonyle (500mg; 2.31 mmol), de pyridine (3ml) et de (4-éthyl-phenyl)-oxetan-3-ylmethyl-amine (491mg; 2.77 mmol) est agité pendant 30 minutes à une température de 50°C. Le milieu réactionnel est dilué avec de l'acétate d'éthyle.

La phase organique est lavée avec une solution saturée de NH₄Cl, une solution saturée de NaHCO₃ et de l'eau. Elle est séchée sur sulfate de magnésium filtrée et concentrées à sec.

Le produit brut est chromatographié sur gel de silice (éluant heptane/acétate d'éthyle de 0 à 40% d'acétate d'éthyle). L'acide 1H-indazole-6-sulfonique (4-éthyl-phényl)-isobutyl-amide (640mg; 78%) est obtenu sous la forme d'une huile incolore est obtenue avec une RMN¹H conforme
MS : [M+H] = 358

### 2. Synthèse de l'intermédiaire 63.2

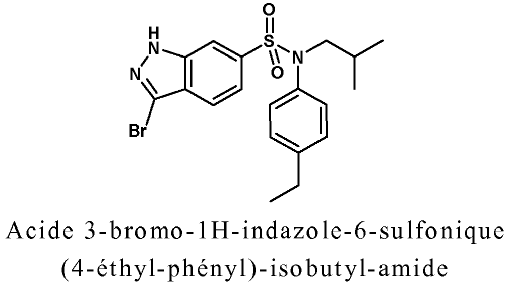

A une solution d'acide 1H-Indazole-6-sulfonique (4-ethyl-phenyl)-isobutyl-amide (1.60 g; 4.48 mmol) dans du dichlorométhane (10ml) est ajouté du N-bromosuccinimide (956 mg; 5.37 mmol). Le milieu réactionnel est agité pendant 16 heures à température ambiante, dilué avec du dichlorométhane.

La phase organique est extraite et lavé avec une solution de chlorure d'ammonium saturée, une solution d'hydrogénocarbonate de sodium saturée et de l'eau. Elle est séchée (MgSO₄), filtrée et concentrée à sec. L'acide 3-bromo-1H-indazole-6-sulfonique (4-éthyl-phényl)-isobutyl-amide (1.9 g; 97%) est obtenu sous la forme d'un solide jaune pâle avec une RMN¹H conforme.
MS : [M+H] =436

### 3. Synthèse de l'intermédiaire 63.3

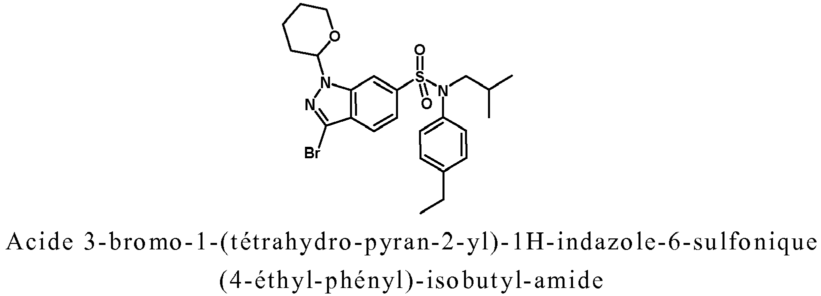

Un mélange d'acide 3-bromo-1H-indazole-6-sulfonique (4-ethyl-phenyl)-isobutyl-amide (700 mg; 1.60 mmol), de p-toluenesulfonate de pyridinium (202 mg; 0.80 mmol) et de 3,4-dihydro-2H-pyranne (0.58 ml; 6.42 mmol) dans du tétrahydrofurane (10ml) est agité 16 heures à 80°C. Le milieu réactionnel est dilué à d'acétate d'éthyle (20ml) et extrait.

La phase organique est lavée avec une solution saturée de NH₄Cl, une solution saturée de NaHCO₃ et de l'eau, séchée (MgSO₄), filtrée et concentrée à sec.

Le produit brut est purifié par HPLC préparative (colonne C18, éluant d'acétonitrile dans eau/0.1% d'acide formique). L'acide 3-bromo-1-(tétrahydro-pyran-2-yl)-1H-indazole-6-sulfonique (4-éthyl-phényl)-isobutyl-amide (500 mg; 57%) est obtenu sous la forme d'un solide blanc.
¹H NMR (DMSO-d6) δ: 0.86 (dd, J = 13.3, 6.7 Hz, 6H), 1.18 (t, J = 7.6 Hz, 3H), 1.38 - 1.63 (m, 3H), 1.68 - 1.81 (m, 1H), 1.98 (d, J = 10.7 Hz, 2H), 2.60 (q, J = 7.6 Hz, 2H), 3.36 - 3.41 (m, 2H), 3.67 - 3.76 (m, 1H), 6.00 (dd, J = 9.3, 2.4 Hz, 1H), 6.95 - 7.02 (m, 2H), 7.16 - 7.21 (m, 2H), 7.41 (dd, J = 8.5, 1.4 Hz, 1H), 7.83 (d, J = 8.8 Hz, 1H), 7.99 (s, 1H)
MS : [M+H] = 520

### 4. Synthèse de l'intermédiaire 63.4

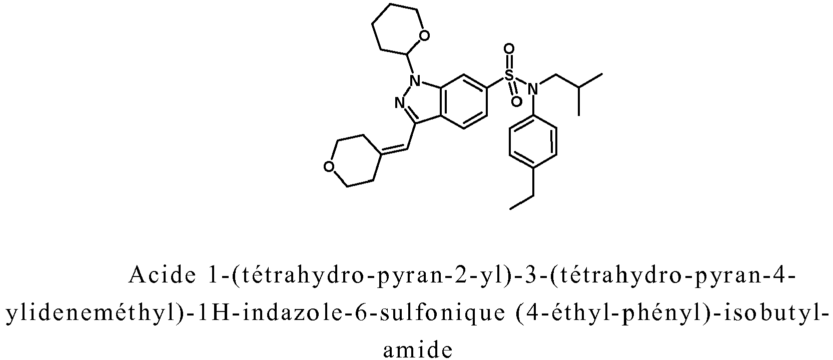

Sous argon, un mélange de p-toluenesulfonhydrazide (286mg; 1.54 mmol), de 4-formyltétrahydro-pyrane (175 mg; 1.54 mmol) et de 1,4-dioxanne (2ml) est agité 1 heure. Du ter-butoxide de lithium (125 mg; 1.54 mmol), du X-PHOS (18mg; 0.04 mmol), du tris(dibenzylideneacétone)-dipalladium (0) chloroforme adduit (40 mg; 0.04 mmol) et de l'acide 3-bromo-1-(tétrahydro-pyran-2-yl)-1H-indazole-6-sulfonique (4-éthyl-phényl)-isobutyl-amide (200mg; 0.38 mmol) sont ajouté sous argon, et le milieu réactionnel est agité 5heures à 100°C.

Le milieu réactionnel est dilué avec 20mL d'acétate d'éthyle et extrait. La phase organique est lavée avec une solution saturée de NH₄Cl (20ml), une solution saturée de NaHCO₃ (20ml) et de l'eau (20ml), séchée (MgSO₄), filtrée et concentrée.

Le produit brut est chromatographié sur gel de silice (éluant heptane/acétate d'éthyle de 0 à 100% d'acétate d'éthyle).

L'acide 1-(tétrahydro-pyran-2-yl)-3-(tétrahydro-pyran-4-ylideneméthyl)-1H-indazole-6-sulfonique (4-éthyl-phényl)-isobutyl-amide (100mg; 48%) est obtenu sous la forme d'une huile jaune avec une RMN¹H conforme.
MS : [M+H] = 538

### 5. Synthèse du composé 62 selon l'invention

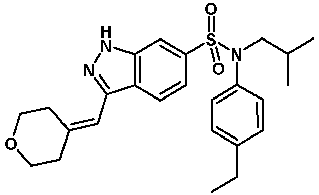

Un mélange d'acide 1-(tetrahydro-pyran-2-yl)-3-(tétrahydro-pyran-4-ylideneméthyl)-1H-indazole-6-sulfonique (4-éthyl-phényl)-isobutyl-amide (100 mg; 0.19 mmol) d'acide acétique (6ml) et d'eau (2ml) est agité pendant 5 heures à une température de 80°C. Le milieu réactionnel est dilué avec 20ml d'acétate d'éthyle et extrait. La phase organique est lavée avec une solution saturée de NH₄Cl (20ml), une solution saturée de NaHCO₃ (20ml), et de l'eau (20ml), séchée (MgSO₄), filtrée et concentrée.

Le produit brut est chromatographié sur gel de silice (éluant heptane/acétate d'éthyle de 0 à 100% d'acétate d'éthyle). L'acide 3-(tetrahydro-pyran-4-ylidenemethyl)-1H-indazole-6-sulfonique (4-éthyl-phényl)-isobutyl-amide (80 mg; 93 %) est obtenu sous la forme d'un solide blanc.
¹H NMR (DMSO-d6) δ: 0.85 (d, J = 6.7 Hz, 7H), 1.17 (t, J = 7.6 Hz, 3H), 1.41 (dt, J = 13.6, 6.8 Hz, 1H), 2.60 (q, J = 7.3 Hz, 2H), 2.93 (t, J = 5.4 Hz, 2H), 3.65 (t, J = 5.5 Hz, 2H), 3.73 (t, J = 5.4 Hz, 2H), 6.61 (s, 1H), 6.95 (d, J = 8.3 Hz, 2H), 7.13 - 7.21 (m, 2H), 7.22 (dd, J = 8.5, 1.5 Hz, 1H), 7.63 - 7.67 (m, 1H), 7.98 (d, J = 8.5 Hz, 1H).
MS : [M+H] = 454

### Exemple 65 : Synthèse de l'acide 3-(tétrahydro-pyran-4-ylméthyl)-1H-indazole-6-sulfonique (4-éthyl-phényl)-isobutyl-amide

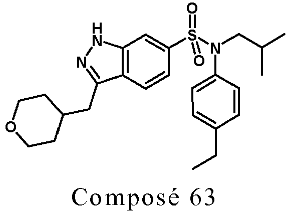

De l'acide 3-(tétrahydro-pyran-4-ylidenemethyl)-1H-indazole-6-sulfonique (4-éthyl-phényl)-isobutyl-amide (60 mg; 0.13 mmol) dans du méthanol (3ml) en présence de palladium (5% Pd sur charbon activé : type DEGUSSA) (14mg) est placé pendant 16 heures sous hydrogène (latm). Le milieu réactionnel est filtré sur célite. Le filtrat est concentré à sec. Le produit brut est chromatographié sur gel de silice (éluant heptane/acétate d'éthyle de 0 à 100% d'acétate d'éthyle). L'acide 3-(tétrahydro-pyran-4-ylméthyl)-1H-indazole-6-sulfonique (4-éthyl-phényl)-isobutyl-amide (30 mg; 50%) est obtenu sous la forme d'un solide blanc.
¹H NMR (DMSO-d6) δ: 0.85 (d, J = 6.4 Hz, 6H), 1.17 (t, J = 7.6 Hz, 3H), 1.23 - 1.48 (m, 3H), 1.56 (d, J = 13.4 Hz, 2H), 1.97 (d, J = 11.7 Hz, 1H), 2.56 - 2.65 (m, 2H), 2.90 (d, J = 7.0 Hz, 2H), 3.26 (t, J = 11.3 Hz, 2H), 3.82 (dd, J = 11.6, 3.8 Hz, 2H), 6.97 (d, J = 8.1 Hz, 2H), 7.19 (dd, J = 14.2, 8.2 Hz, 3H), 7.62 (s, 1H), 7.95 (d, J = 8.3 Hz, 1H), 13.15 (s, 1H).
MS : [M+H] =456

### Partie VII : Synthèse des sulfonamides bicycliques à partir du schéma réactionnel 7

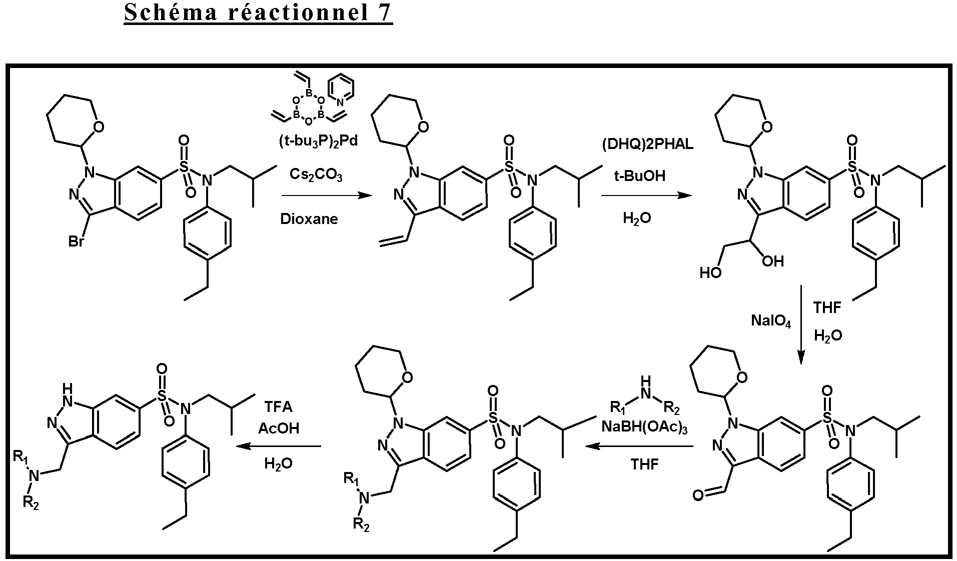

### Exemple 65 : Synthèse de l'acide 3-morpholin-4-ylméthyl-1H-indazole-6-sulfonique (4-éthyl-ohényl)-isobutyl-amide

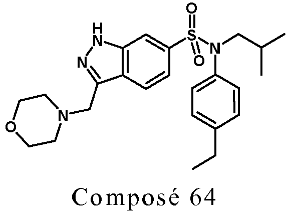

### 1. Synthèse de l'intermédiaire 65.1

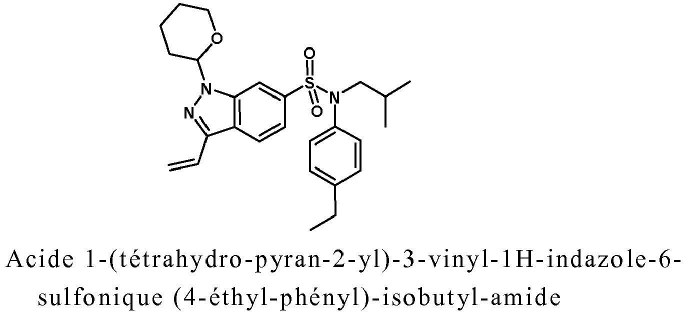

A un mélange, dégazé sous argon, d'acide 3-bromo-1-(tétrahydro-pyran-2-yl)-1H-indazole-6-sulfonique (4-éthyl-phényl)-isobutyl-amide (500 mg; 0.96 mmol), de carbonate de césium (940 mg; 2.88 mmol), de complexe anhydride vinylboronique, pyridine (462 mg; 1.92 mmol) dans du 1,4-dioxanne (3ml) et de l'eau (1ml) est ajouté du bis(tri-t-butylphosphine)palladium(0) (49mg; 0.10 mmol) est ajouté.

Le milieu réactionnel est agité pendant 3 heures à une température de 90°C et filtré sur célite. Le filtrat est dilué à l'acétate d'éthyle et extrait.

La phase organique est lavée avec 20mL d'une solution d'hydrogénocarbonate de sodium saturée et 20mL d'eau. La phase organique est lavée avec une solution saturée de NH₄Cl (20ml), une solution saturée de NaHCO₃ (20ml), et de l'eau (20ml), séchée (MgSO₄), filtrée et concentrée.

Le produit brut est chromatographié sur gel de silice (éluant heptane/acétate d'éthyle de 0 à 100% d'acétate d'éthyle). L'acide 1-(tetrahydro-pyran-2-yl)-3-vinyl-1H-indazole-6-sulfonique (4-éthyl-phenyl)-isobutyl-amide (400mg; 89%) est obtenu sous la forme d'un solide beige avec une RMN¹H conforme.
MS : [M+H] = 468

### 2. Synthèse de l'intermédiaire 65.2

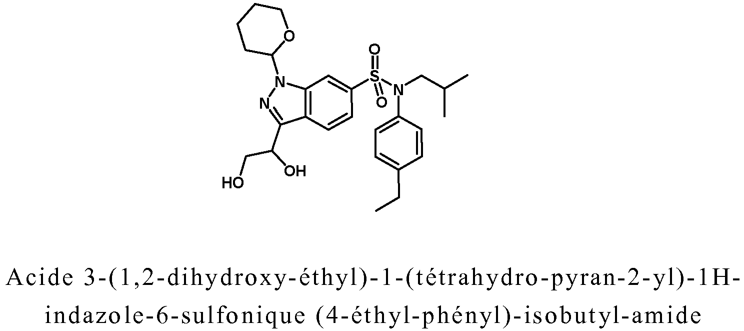

A un mélange de (DHQ)₂PHAL (1.20 g; 1.46 mmol), d'alcool ter-butylique (10 ml) et d'eau (10 ml) refroidit à une température de 0°C est ajouté l'acide 1-(tétrahydro-pyran-2-yl)-3-vinyl-1H-indazole-6-sulfonique (4-éthyl-phényl)-isobutyl-amide (400 mg; 0.86 mmol). Le milieu réactionnel est agité pendant 16 heures à une température de 40°C. Du sulfite de sodium (5g) est ajouté.

Le milieu réactionnel est agité pendant 45 minutes et dilué à l'acétate d'éthyle (30ml) et extrait. Les phases organiques sont rassemblées, lavées avec une de solution saturé (Na₂SO₃, 20ml), de l'eau (20ml), séchées (MgSO₄), filtrées et concentrées. Le produit brut est chromatographié sur gel de silice (éluant heptane/acétate d'éthyle de 0 à 100% d'acétate d'éthyle).

L'acide 3-(1,2-dihydroxy-éthyl)-1-(tétrahydro-pyran-2-yl)-1H-indazole-6-sulfonique (4-éthyl-phényl)-isobutyl-amide (380mg; 89%) est obtenu sous la forme d'une huile claire avec une RMN¹H conforme.
MS : [M+H] = 502

### 3. Synthèse de l'intermédiaire 65.3

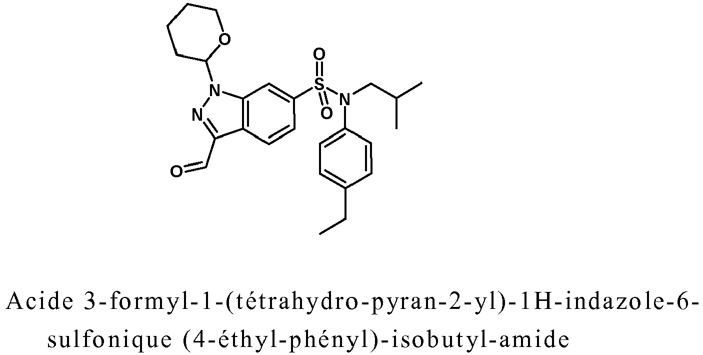

A un mélange d'acide 3-(1,2-dihydroxy-éthyl)-1-(tétrahydro-pyran-2-yl)-1H-indazole-6-sulfonique (4-éthyl-phényl)-isobutyl-amide (380mg; 0.76 mmol), d'acétone (2ml), de tétrahydrofurane (2ml) et d'eau (2ml) est ajouté du métapériodate de sodium (1.62 g; 7.58 mmol).

Le milieu réactionnel est agité pendant deux heures et demi à une température de 35°C, dilué avec de l'acétate d'éthyle (30ml) et de l'eau (20ml) et extrait.

Les phases organiques sont rassemblées, lavées avec une de solution saturé (Na₂SO₃, 20ml), de l'eau (20ml), séchées (MgSO₄), filtrées et concentrées. L'acide 3-formyl-1-(tétrahydro-pyran-2-yl)-1H-indazole-6-sulfonique (4-éthyl-phényl)-isobutyl-amide (120 mg; 34 %) est obtenu sous la forme d'un solide blanc avec une RMN¹H conforme.
MS : [M+H] = 470

### 4. Synthèse de l'intermédiaire 65.4

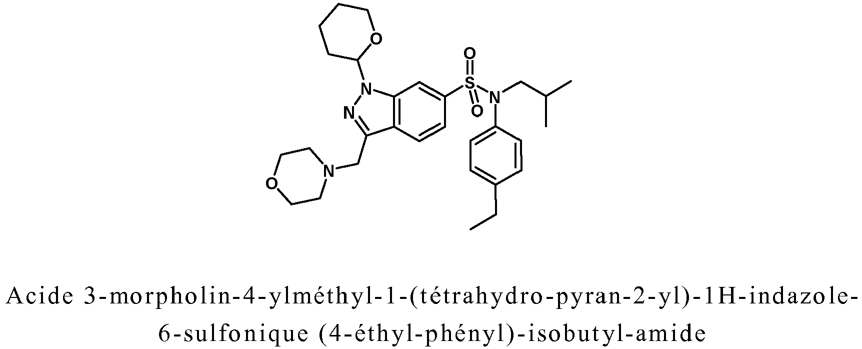

A une solution d'acide 3-formyl-1-(tétrahydro-pyran-2-yl)-1H-indazole-6-sulfonique (4-éthyl-phényl)-isobutyl-amide (120 mg; 0.26 mmol) dans du tétrahydrofurane (3 ml) sont ajoutés de la morpholine (45 µ1; 0.51 mmol) et du triacétoxyborohydrure de sodium (119 mg; 0.56 mmol). Le milieu réactionnel est agité pendant deux heures à température ambiante. Le milieu réactionnel est dilué avec de l'acétate d'éthyle (20ml) et de l'eau (10ml) et extrait.

La phase organique est lavée avec une solution saturée (NH₄Cl, 20ml), une solution saturée (NaHCO₃, 20ml) et de l'eau (20ml), séchée (MgSO₄) et concentrée.

L'acide 3-morpholin-4-ylméthy1-1-(tétrahydro-pyran-2-yl)-1H-indazole-6-sulfonique (4-éthyl-phényl)-isobutyl-amide (130 mg; 94%) est obtenu la forme d'une huile avec une RMN¹H conforme.
MS : [M+H] = 541

### 5. Synthèse du composé 64 selon l'invention

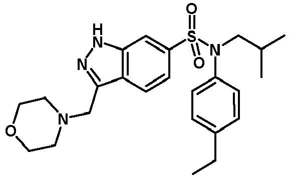

Un mélange d'acide 3-morpholin-4-ylméthyl-1-(tetrahydropyran-2-yl)-1H-indazole-6-sulfonique (4-éthyl-phényl)-isobutyl-amide (130 mg; 0.24 mmol), d'acide acétique (30ml) et d'eau (10ml) et quelques gouttes d'acide trifluoroacétique est agité pendant 4 jours à une température de 80°C. Le milieu réactionnel est dilué avec 50ml d'acétate d'éthyle et extrait.

La phase organique est lavée avec une solution saturée (NH₄Cl, 20ml), une solution saturée (NaHCO₃, 20ml), et de l'eau (20ml), séchée (MgSO₄), filtrée et concentrée.

Le produit brut est purifié par HPLC préparative (colonne C18, éluant : acétonitrile dans eau/0.1% d'acide formique). L'acide 3-morpholin-4-ylméthyl-1H-indazole-6-sulfonique (4-éthyl-phényl)-isobutyl-amide (70 mg; 64 %) est obtenu sous la forme d'un solide blanc après recristallisation dans un mélange acétone/ eau.
1H NMR (DMSO-d6) δ: 0.85 (d, J = 6.7 Hz, 6H), 1.18 (t, J = 7.6 Hz, 3H), 2.44 (t, J = 4.5 Hz, 4H), 2.61 (t, J = 7.6 Hz, 2H), 3.33 - 3.37 (m, 2H), 3.87 (s, 2H), 6.95 - 7.00 (m, 2H), 7.15 - 7.21 (m, 2H), 7.25 (dd, J = 8.5, 1.5 Hz, 1H), 8.06 (d, J = 8.5 Hz, 1H), 13.27 (s, 1H).
MS : [M+H] = 457

### Exemple 66 : Synthèse de l'acide 3-((cis)-2,6-dimethylmorpholin-4-ylmethyl)-1H-indazole-6-sulfonique (4-ethyl-phenyl)-isobutyl-amide

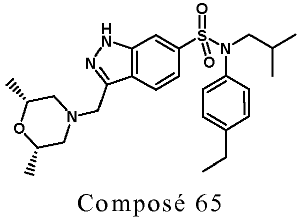

### 1. Synthèse de l'intermédiaire 66.1

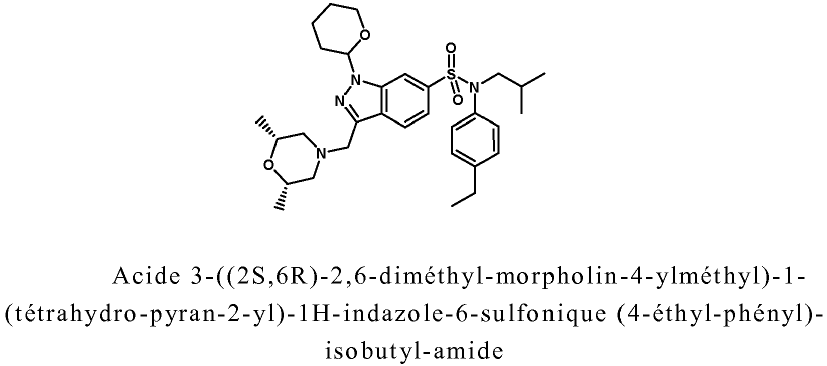

A une solution d'acide 3-formyl-1-(tétrahydro-pyran-2-yl)-1H-indazole-6-sulfonique (4-éthyl-phényl)-isobutyl-amide (90 mg; 0.19 mmol) dans du tétrahydrofurane (2 ml) sont ajoutés de la cis-2,6-dimethylmorpholine (50 µl; 0.38 mmol) et du triacétoxyborohydrure de sodium (122 mg; 0.57 mmol).

Le milieu réactionnel est agité pendant 16 heures à température ambiante. Le milieu réactionnel est dilué avec de l'acétate d'éthyle (20ml) et de l'eau (10ml) et extrait. La phase organique est lavée avec une solution saturée (NH₄Cl,20ml), une solution saturée (NaHCO₃, 20ml) et de l'eau (20ml), séchée (MgSO₄) et concentrée. L'acide 3-((2S,6R)-2,6-diméthyl-morpholin-4-ylmethyl)-1-(tétrahydro-pyran-2-yl)-1H-indazole-6-sulfonique (4-éthyl-phényl)-isobutyl-amide (90 mg; 83%) est obtenu la forme d'une huile avec une RMN¹H conforme.
MS : [M+H] = 570

### 2. Synthèse du composé 65 selon l'invention

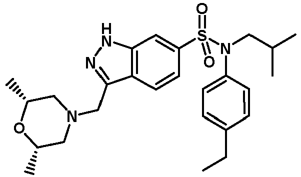

Un mélange d'acide 3-((2S,6R)-2,6-diméthyl-morpholin-4-ylmethyl)-1-(tétrahydro-pyran-2-yl)-1H-indazole-6-sulfonique (4-éthyl-phényl)-isobutyl-amide (90 mg; 0.16 mmol), d'acide acétique (10ml) et d'eau (10ml) et 1 goutte d'acide trifluoroacétique est agité pendant 16 heures à une température de 80°C. Le milieu réactionnel est dilué avec 50ml d'acétate d'éthyle et extrait.

La phase organique est lavée avec une solution saturée (NH₄Cl, 20ml), une solution saturée (NaHCO₃, 20ml), et de l'eau (20ml), séchée (MgSO₄), filtrée et concentrée.

Le produit brut est purifié par HPLC préparative (colonne C18, éluant : acétonitrile dans eau/0.1% d'acide formique). L'acide 3-((cis)-2,6-diméthyl-morpholin-4-ylméthyl)-1H-indazole-6-sulfonique (4-éthyl-phényl)-isobutyl-amide (52 mg; 67 %) est obtenu sous la forme d'une huile incolore.
1H NMR (DMSO-d6) δ: 0.85 (d, J = 6.5 Hz, 6H), 1.02 (d, J = 6.1 Hz, 6H), 1.18 (t, J = 7.5 Hz, 3H), 1.42 (dt, J = 13.3, 7.1 Hz, 1H), 1.74 (t, J = 10.7 Hz, 2H), 2.55 - 2.65 (m, 2H), 2.70 - 2.80 (m, 2H), 3.33 - 3.44 (m, 2H), 3.55 (t, J = 7.8 Hz, 2H), 3.85 (s, 2H), 6.98 (d, J = 8.0 Hz, 2H), 7.17 (d, J = 8.3 Hz, 2H), 7.23 (d, J = 8.6 Hz, 1H), 7.66 (s, 1H), 8.03 (d, J = 8.6 Hz, 1H), 8.20 (s, 1H), 13.25 (s, 1H).
MS : [M+H] = 485

### Exemple 67 : Synthèse de l'acide 3-((S)-3-méthylmorpholin-4-ylméthyl)-1H-indazole-6-sulfonique (4-éthyl-phényl)-isobutyl-amide

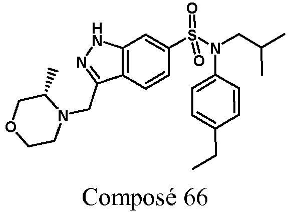

### 1. Synthèse de l'intermédiaire 67.1

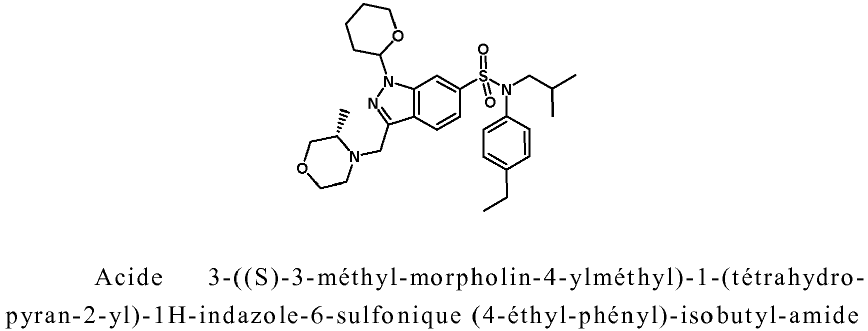

Avec un mode opératoire analogue à celui décrit pour l'intermédiaire 67.1, l'acide 3-((S)-3-méthyl-morpholin-4-ylméthyl)-1-(tétrahydro-pyran-2-yl)-1H-indazole-6-sulfonique (4-éthyl-phényl)-isobutyl-amide (90 mg; 85%) est obtenu la forme d'une huile avec une RMN¹H conforme.
MS : [M+H] = 555

### 2. Synthèse du composé 66 selon l'invention

Avec un mode opératoire analogue à celui décrit pour l'exemple 66, l'acide 3-((S)-3-méthyl-morpholin-4-ylméthyl)-1H-indazole-6-sulfonique (4-éthyl-phényl)-isobutyl-amide (42 mg; 54 %) est obtenu sous la forme d'une huile incolore.
1H NMR (DMSO-d6) δ: 0.86 (d, J = 6.8 Hz, 6H), 1.09 - 1.22 (m, 7H), 1.42 (dt, J = 13.9, 6.9 Hz, 1H),2.16 - 2.27 (m, 1H), 2.61 (t, J = 7.6 Hz, 2H), 3.38 (d, J = 19.0 Hz, 5H), 3.58 - 3.72 (m, 3H), 6.98 (d, J = 7.9 Hz, 2H), 7.18 (d, J = 7.9 Hz, 2H), 7.26 (d, J = 8.5 Hz, 1H), 7.65 (s, 1H), 8.04 (d, J = 8.6 Hz, 1H), 8.24 (s, 1H), 13.24 (s, 1H).
MS : [M+H] = 471

## Revendications

1. Composé de formule (I), ses sels d'addition pharmaceutiquement acceptables, ses hydrates et/ou ses solvates : 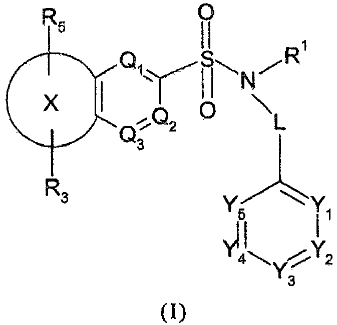 Formule (I) dans laquelle :
• L représente une liaison simple ou un groupe méthylène CH₂,
• X représente un radical cyclique choisi parmi les radicaux X₁ et X₂ suivants : 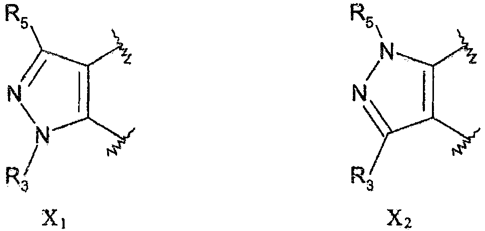
• un ou deux des éléments Y¹, Y², Y³, Y⁴ et Y⁵ représente(nt) un atome d'azote et les autres éléments correspondent à un groupement -CR², ou chacun des éléments Y¹, Y², Y³, Y⁴ et Y⁵ correspond à un groupement -CR²,
• un ou deux des éléments Q¹, Q² et Q³ représente(nt) un atome d'azote et le ou les autres éléments corresponde(nt) à un groupement - CR^{2a}, ou chacun des éléments Q¹, Q² et Q³ correspond à un groupement -CR^{2a},
• R¹ représente un radical alkyle, linéaire ou ramifié, en C₃-C₅, éventuellement substitué par un groupement hydroxyle et/ou un atome d'halogène, un radical cycloalkyle en C₃-C₅, un radical alkényle, linéaire ou ramifié, en C₂-C₅, un radical -CH₂-cycloalkyle en C₃-C₅, un radical hétérocycloalkyle en C₄-C₅, un radical -CH₂-hétérocycloalkyle en C₄-C₆,
• R² représente un atome d'hydrogène ou un atome d'halogène, un radical alkyle en C₁-C₅, linéaire ou ramifié, un radical alkényle en C₂-C₄, linéaire ou ramifié, un radical alcoxy en C₁-C₄, un groupement cyano -CN, un radical -C(=O)R'² avec R'² désignant un radical alcoxy en C₁-C₃, un radical -CF₃ ; lesdits radicaux alkyle, alkényle et alcoxy pouvant être substitués par un ou plusieurs atomes d'halogène ;
• R^{2a} représente un atome d'hydrogène ou un atome d'halogène, un radical alkyle en C₁-C₅, linéaire ou ramifié, un radical alkényle en C₂-C₄, linéaire ou ramifié, un radical alcoxy en C₁-C₄, un groupement -CN, un groupement hydroxy -OH, un groupement - CH(R^{3a})OH, un groupement carboxylique -COOH, un groupement carbamoyle -CONR^{2c}R^{2d}, un groupement amido -NR^{2c}COR^{2d} , un groupement -SO₂R^{2c}, un groupement -SOR^{2c}, un groupement - S(=O)(=NH-R^{2c}), lesdits radicaux alkyle, alkényle et alcoxy pouvant être substitués par un ou plusieurs atomes d'halogène,
• R^{2c} et R^{2d}, identiques ou différents, représentent un atome d'hydrogène ou un radical alkyle, linéaire ou ramifié, en C₁-C₅ ;
• R^{3a} représente un atome d'hydrogène ou un radical alkyle, linéaire ou ramifié, en C₁-C₅ ;
• R³ représente un atome d'hydrogène, un atome d'halogène, un groupement (CHR⁶)ₙ-(Z)ₒ-(CHR'⁶)ₚ-R⁷ ou un groupement CH=R⁷,
• n, ο et p, identiques ou différents, représentent zéro ou un entier naturel allant de 1 à 3,
• Z représente un groupement divalent choisi parmi un groupement méthylène -CH₂-, un groupement amino -NH- et un atome d'oxygène - O-,
• R⁶ et R'⁶, identiques ou différents, représentent un atome d'hydrogène, un groupement méthyle CH₃, un groupement -OH, un groupement hydroxyméthyle, une fonction carboxylique -COOH,
• R⁷ représente :
- un atome d'hydrogène ou un atome d'halogène,
- un groupement COOR'⁷ avec R'⁷ désignant alkyl(C1)hétérocycle(C₆),
- un radical hétérocycloalkyle non cationique éventuellement substitué par un ou plusieurs atomes d'halogène, un ou plusieurs groupements alkyle en C₁-C₃, linéaire ou ramifié, un ou plusieurs groupements -OH, une ou plusieurs fonctions carbonyles, un ou plusieurs groupements hydroxyalkyle en C₁-C₄, linéaire ou ramifié, un ou plusieurs groupements amino, un ou plusieurs groupements -C(=O)R^{7a}, un ou plusieurs groupements S(=O)₂R^{7a} ; R^{7a} représentant un radical alkyle, linéaire ou ramifié, en C₁-C₃, un radical alcoxy en C₁-C₃, linéaire ou ramifié, ou un radical amino N(R^{8a})(R^{8b}),
- un radical cycloalkyle non cationique en C₃-C₆ éventuellement substitué par un ou plusieurs radicaux méthyle, un ou plusieurs atomes d'halogène, un groupement cyano -CN ou un ou plusieurs groupements -COR¹³ ; R¹³ désignant un radical alcoxy en C₁-C₃, linéaire ou ramifié, ou un groupement hydroxy,
- un radical aromatique ou hétéroaromatique, non cationique, éventuellement substitué par un ou plusieurs atomes d'halogène, un ou plusieurs groupements alkyle en C₁-C₃, linéaire ou ramifié, éventuellement substitué par un ou plusieurs atomes d'halogène, un ou plusieurs groupements alcoxy en C₁-C₃, un ou plusieurs groupements amino -NR¹¹R¹², un ou plusieurs groupements -COR¹¹, un ou plusieurs groupements -COOR¹¹, un ou plusieurs groupements amido - CONR¹¹R¹², un ou plusieurs groupements -SOR¹¹, un ou plusieurs groupements -SO₂R¹¹, un ou plusieurs groupements - NHCOR¹¹, un ou plusieurs groupements -NHCOOR¹¹, un ou plusieurs groupements -SO₂NR¹¹R¹² ou un ou plusieurs groupements -CN; R¹¹ et R¹², identiques ou différents, représentant un atome d'hydrogène, un radical hydroxy -OH, un radical alkyle, linéaire ou ramifié, en C₁-C₃, éventuellement substitué par un ou plusieurs atomes d'halogène,
• lorsque R³ représente un groupement -CH=R⁷ alors R⁷ ne représente pas un atome d'hydrogène, un atome d'halogène ou un groupement COOR'⁷,
• R⁵ représente un atome d'hydrogène ou un atome d'halogène, un radical alkyle en C₁-C₃, linéaire ou ramifié éventuellement substitué par un ou plusieurs atomes d'halogène; un radical amino -NH₂, un radical hétérocyclique en C₄-C₅, un radical OCH₂-hétérocyclique en C₄-C₅, un radical CH₂R'^{7a} avec R'^{7a} désignant un radical méthoxy, un groupement hydroxy -OH, un groupement -CH₂COOH, un groupement -CH(R^{Sb})OH, un groupement carboxylique -COOH, un groupement - CN, une fonction thioxo,
• R^{5b} représente un atome d'hydrogène ; un radical alkyle, linéaire ou ramifié, en C₁-C₃ éventuellement substitué par une ou plusieurs fonctions carboxyliques ; un radical cyclopropyle,
• R^{8a} et R^{8b} identiques ou différents, désignent un atome d'hydrogène, un radical alkyle, linéaire ou ramifié, en C₁-C₃ ou un radical cyclopropyle.

2. Composé de formule (I) selon la revendication 1, **caractérisé en ce que** R⁷ représente un radical hétérocyclique choisi parmi les hétérocycles suivants : 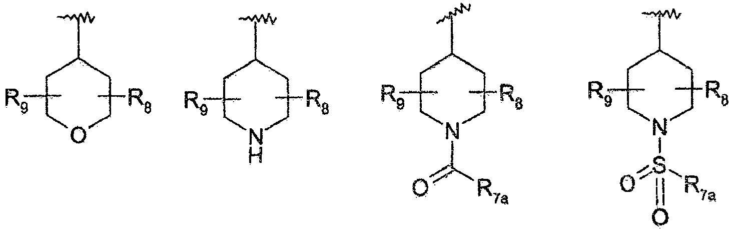 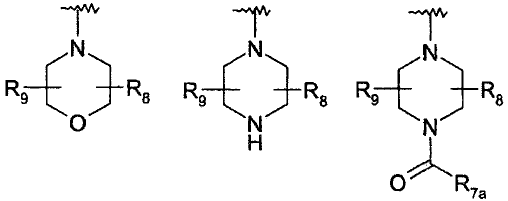 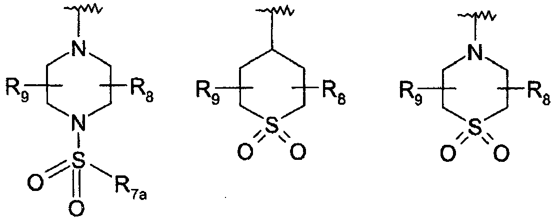 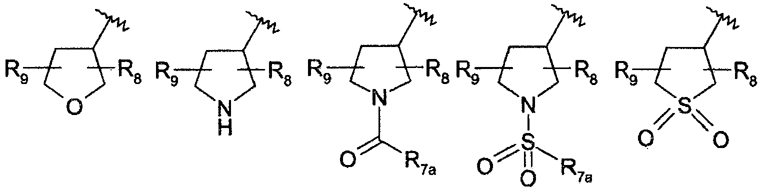 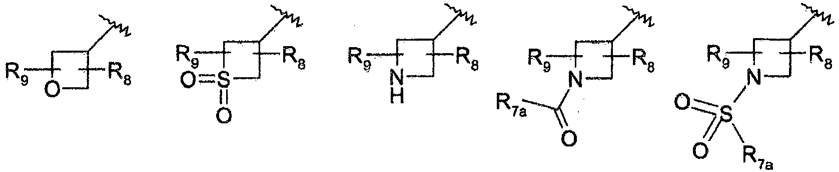 dans lesquels :
- R₇ₐ représente un radical alkyle, linéaire ou ramifié, en C₁-C₃, un radical alcoxy, linéaire ou ramifié, en C₁-C₃ ou un radical amino en N(R^{8a})(R^{8b}),
- R^{8a} et R^{8b}, identiques ou différents, désignent un atome d'hydrogène, un radical alkyle, linéaire ou ramifié, en C₁-C₃ ou un radical cyclopropyle,
- R₈ et R₉, identiques ou différents, représentent un atome d'hydrogène, un radical alkyle, linéaire ou ramifié, en C₁-C₃, un groupement hydroxy -OH, une fonction carbonyle =O, un radical hydroxyalkyle en C₁ (-CH₂OH), un groupe amino NH₂,
- R₈ et R₉ peuvent former ensemble avec les atomes de carbone auxquels ils sont attachés un cycle carbocyclique comportant de 5 à 7 chaînons,

3. Composé de formule (I) selon la revendication 1, **caractérisé en ce que** R⁷ représente un radical aromatique ou hétéroaromatique choisi parmi : 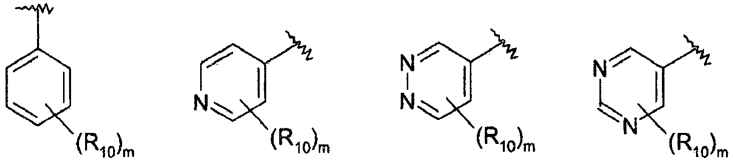 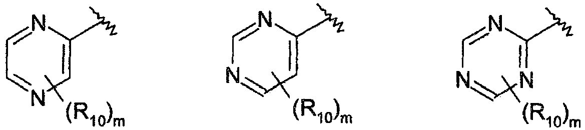 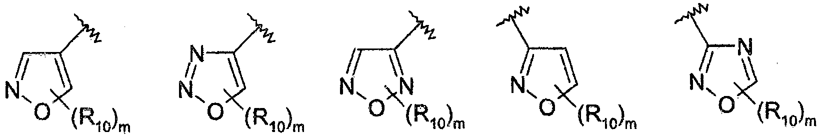 dans lesquels :
- R₁₀ représente un atome d'hydrogène ou un atome d'halogène, un groupement alkyle en C₁-C₃, linéaire ou ramifié, éventuellement substitué par un ou plusieurs atomes d'halogène ; un groupement alcoxy en C₁-C₃, un groupement amino -NR¹¹R¹², un groupement -COR¹¹, un groupement -COOR¹¹, un groupement amido - CONR¹¹R¹², un groupement -SOR¹¹, un groupement -SO₂R¹¹, un groupement -NHCOR¹¹, un groupement -NHCOOR¹¹, un groupement - SO₂NR¹¹R¹² ou un groupement -CN; R¹¹ et R¹², identiques ou différents, représentant un atome d'hydrogène ou un radical alkyle, linéaire ou ramifié, en C₁-C₃ éventuellement substitué par un ou plusieurs atomes d'halogène,
m désigne zéro ou un entier naturel allant de 1 à 3.

4. Composé de formule (I) selon la revendication 1, **caractérisé en ce qu'**il est choisi parmi le ou les composés de formule (II) ainsi que leurs sels d'addition pharmaceutiquement acceptables, leurs hydrates et/ou leurs solvates : 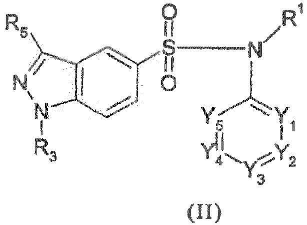 Formule (II) dans laquelle R¹, R³, R⁵ et Y¹ à Y⁵ ont les mêmes significations que dans la formule (I) telle que définie selon la revendication 1.

5. Composé de formule (I) selon la revendication 1, **caractérisé en ce qu'**il est choisi parmi le ou les composés de formule (III) ainsi que leurs sels d'addition pharmaceutiquement acceptables, leurs hydrates et/ou leurs solvates : 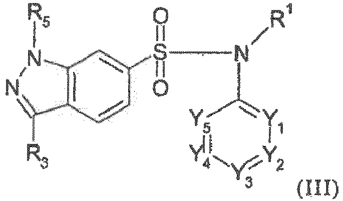 Formule (III) dans laquelle R¹, R³, R⁵ et Y¹ à Y⁵ ont les mêmes significations que dans la formule (I) telle que définie selon la revendication 1.

6. Composé de formule (I) selon l'une quelconque des revendications précédentes, **caractérisé en ce qu'**il est choisi parmi les composés suivants :
| | |
|---|---|
| 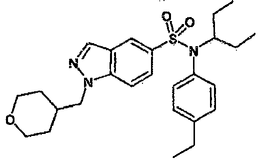 | Acide 1-(tétrahydro-pyran-4-ylméthyl)-1H-indazole-5-sulfonique (4-éthyl-phényl)-(1-éthyl-propyl)-amide |
| | Composé 1 |
| 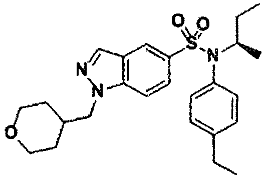 | Acide 1-(tetrahydro-pyran-4-ylmethyl)-1H-indazole-5-sulfonique ((R)-sec-butyl)-(4-ethyl-phenyl)-amide |
| | Composé 2 |
| 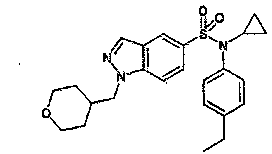 | Acide 1-(tétrahydro-pyran-4-ylméthyl)-1H-indazole-5-sulfonique cyclopropyl-(4-éthyl-phényl)-amide |
| | Composé 3 |
| 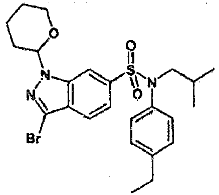 | Acide 3-bromo-1-(tetrahydro-pyran-2-yl)-1H-indazole-6-sulfonique(4-éthyl-phényl)-isobutyl-amide |
| | Composé 4 |
| 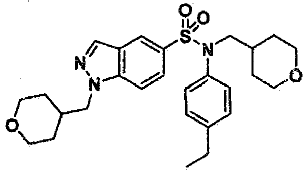 | Acide 1-(tétrahydro-pyran-4-ylméthyl)-1H-indazole-5-sulfonique (4-éthyl-phényl)-(tétrahydro-py]ran-4-ylméthyl)-amide |
| | Composé 5 |
| 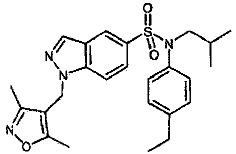 | 1-((3,5-diméthylisoxazol-4-yl)méthyl)-N-(4-éthylphényl)-N-isobutyl-1H-indazole-5-sulfonamide |
| | Composé 6 |
| 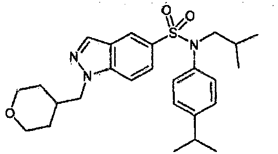 | Acide 1-(tétrahydro-pyran-4-ylméthyl)-1H-indazole-5-sulfonique butyl-(4-isopropyl-phényl)-amide |
| | Composé 7 |
| 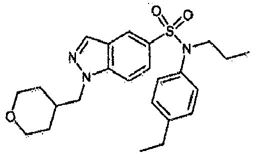 | Acide 1-(tétrahydro-pyran-4-ylmethyl)-1H-indazole-5- sulfonique (4-éthyl-phényl)-propyl |
| | Composé 8 |
| 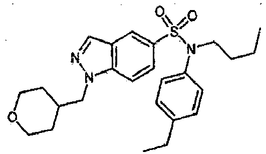 | Acide 1-(tétrahydro-pyran-4-ylmethyl)-1H-indazole-5-sulfonique butyl-(4-éthyl-phényl)-amide |
| | Composé 9 |
| 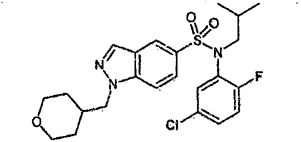 | Acide 1-(tétrahydro-pyran-4-ylméthyl)-1H-indazole-5- sulfonique (5-ehloro-2-fluoro-phényl)-isobutyl-amide |
| | Composé 10 |
| 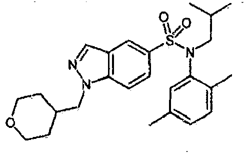 | Acide 1-(tétrahydro-pyran-4-ylmethyl)-1H-indazole-5-sulfonique (2,5-diméthyl-phényl)-isobutyl-amide |
| | Composé 11 |
| 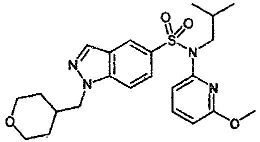 | Acide 1-(Tétrahydro-pyran-4-ylméthyl)-1H-indazole-5-sulfonique (3-méthoxy-pyridin-2-yl)-isobutyl-amide |
| | Composé 12 |
| 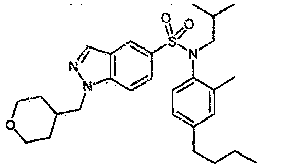 | Acide 1-(tétrahydro-pyran-4-ylméthyl)-1H-indazole-5-sulfonique (4-butyl-2-méthyl-phényl)-isobutyl-amide |
| | Composé 13 |
| 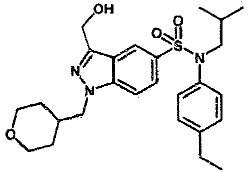 | N-(4-éthylphényl)-N-isobutyl-1-((tétrahydro-2H-pyran-4-yl)méthyl)-1H-indazole-5-sulfonamide |
| | Composé 14 |
| 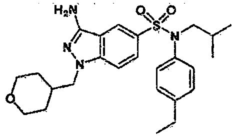 | Acide 3-amino-1H-iadazole-5-sulfonique(4-éthyl-phényl)-isobutyl-amide |
| | Composé 15 |
| 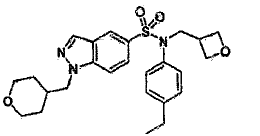 | Acide 1-(tétrahydro-pyran-4-ylméthyl)-1H-indazole-5-sulfonique (4-éthyl-phényl)-oxetan-3-ylmethyl-amide |
| | Composé 16 |
| 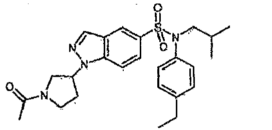 | Acide 1-(1-acétyl-pyrrolidin-3-yl)-1H-indazole-5-sulfonique (4,éthyl-phényl)-isobutyl-amide |
| | Composé 17 |
| 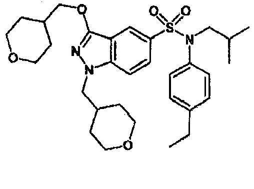 | Acide 3-(tétrahydro-pyran-4-ylméthoxy)-1-(tétrahydro-pyran-4-ylméthyl)-1H-indazole-5-sulfonique (4-éthyl-phényl),isobutyl-amide |
| | Composé 18 |
| 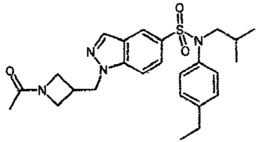 | Acide 1-(3,5-diméthyl-isoxazol-4-ylméthyl)-1H-indazole-5-sulfonique (4-éthyl-phényl)-isobutyl-amide |
| | Composé 19 |
| 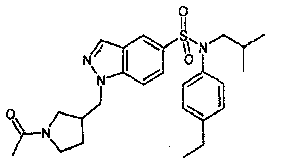 | 1-((1-acétylpyrrolidin-3yl)méthyl)-N-(4-éthylphényl)-N-isobutyl-1H-indazole-5-sulfonamide |
| | Composé 20 |
| 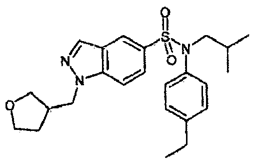 | N-(4-éthylphényl)-N-isobutyl-1 - ((tétrahydro-furan-3-yl)méthyl)-1H-indazole-5-sulfonamide |
| | Composé 21 |
| 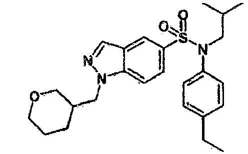 | N-(4-éthylphényl)-N-isobutyl-1-((tétrahydro-2H-pyran-3-yl)méthyl)-1H-indazole-5-sulfonamide |
| | Composé 22 |
| 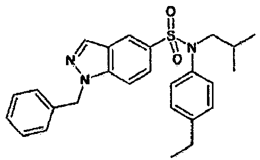 | 1-benzyl-N-(4-éthylphényl)-N-isobutyl-1H-indazole-5-sulfonamide |
| | Composé 23 |
| 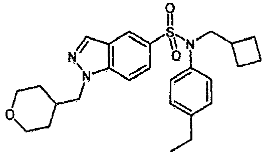 | N-(cyclobutylméthyl)-N-(4-éthylphényl)-1-((tétrahydro-2H-pyran-4-yl)méthyl)-1H-indazole-5-sulfonamide |
| | Composé 24 |
| 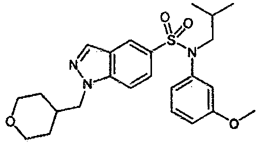 | Acide 1-(tétrahydro-pyran-4-ylméthyl)-1H-indazole-5-sulfonique (3-méthoxy-phényl)-isobutyl-amide |
| | Composé 25 |
| 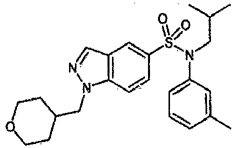 | Acide 1-(tétrahydro-pyran-4-ylméthyl)-1H-indazole-5-sulfonique (3-méthyl-phényl)-isobutyl-amide |
| | Composé 26 |
| 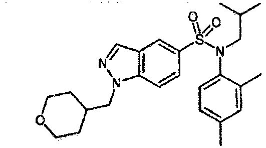 | Acide 1-(tétrahydro-pyran-4-ylméthyl)-1H-indazole-5-sulfonique (2,4-diméthyl-phényl)-isobutyl-amide |
| | Composé 27 |
| 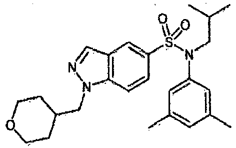 | Acide 1-(tétrahydro-pyran-4-ylmethyl)-1H-indazole-5-sulfonique (3,5-diméthyl-phéayl)-isobutyl-amide |
| | Composé 28 |
| 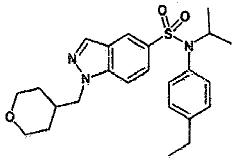 | N-(-((tétrahydro-2H-pyran-4-yl)méthyl)-1H -indazole-5-sulfonamide |
| | Composé 29 |
| 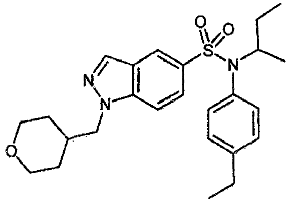 | Acide 1-(tétrahydro-pyran-4-ylméthyl)-1H-indazole-5-sulfonique sec-butyl-(4-éthyl-phényl)-amide |
| | Composé 30 |
| 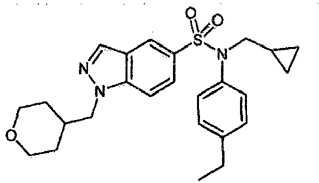 | N-(cyclopropylméthyl)-N-(4-éthylphényl)-1-((tétrahydro-2H-pyran-4-yl)méthyl)-1H-indazole-5-sulfonamide |
| | Composé 31 |
| 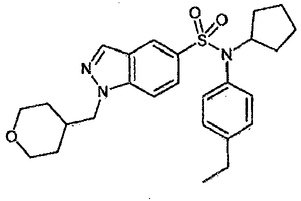 | Acide 1 -(tétrahydro-pyran-4-ylméthyl)-1H-indazole-5-sulfonique cyclopentyl-(4-éthyl-phényl)-amide |
| | Composé 32 |
| 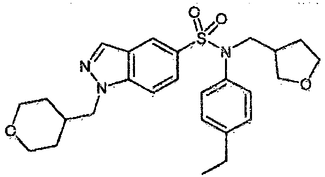 | Acide 1-(tétrahydro-pyran-4-ylméthyl)-1H-indagole-5-sulfonique (4-éthyl-phényl)-(tétrahydro-furan-3-ylméthyl)-amide |
| | Composé 33 |
| 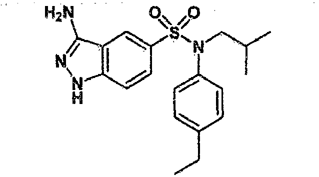 | Acide 3-amino-1H-indazole-5-sulfonique(4-éthyl-phényl)-isobutyl-amide |
| | Composé 34 |
| 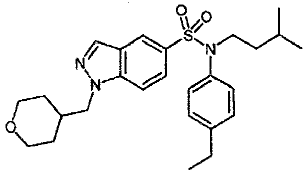 | N-(4-éthylphényl)-N-isopentyl-1-((tétrahydro-2H-pyran-4-yl)méthyl)-1H-indazole-5-sulibnamide |
| | Composé 35 |
| 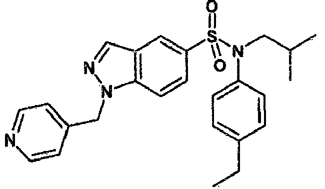 | N-(4-éthylphényl)-N-isobutyl-1-(pyridin-4-ylméthyl)-1H-indazole-5-sulfonamide |
| | Composé 36 |
| 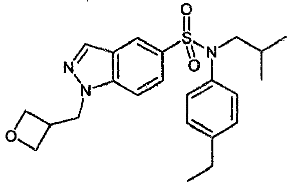 | N-(4-éthylphényl)-N-isobutyl-1- (oxetan-3-ylméthyl)-1H-indazole-5-sulfonamide |
| | Composé 37 |
| 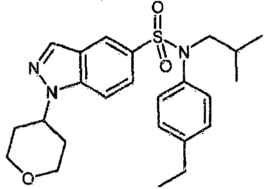 | Acide 1-(tétrahydro-pyran-4-yl)-1H-indazole-5-sulfonique (4-éthyl-phényl)-isobutyl-amide |
| | Composé 38 |
| 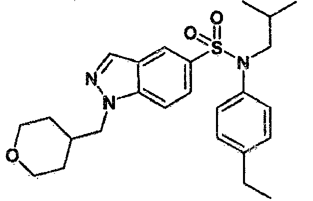 | N-(-((tétrahydro-2H -pyran-4-yl)méthyl)-1H-indazole-5-sulfonamide |
| | Composé 39 |
| 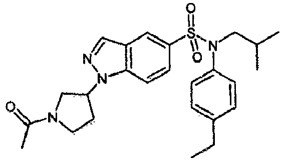 | acide 1-(1-acétyl-pyrrolidin-3-yl)-1H-indazole-5-sulfonique(4-éthyl-phényl)-isobutyl-amide Composé 40 |
| 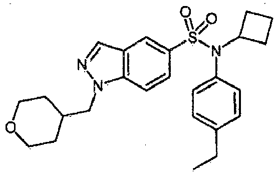 | Acide 1-(tétrahydro-pyran-4-ylméthyl)-1H-indazole-5-sulfonique cyclobutyl-(4-éthyl-phényl)-amide Composé 41 |
| 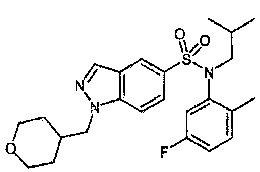 | Acide 1-(tétrahydro-pyran-4-ylméthyl)-1H-indazole-5-sulfonique (5-fluoro-2-méthyl-phényl)-isobutyl-amide |
| | Composé 42 |
| 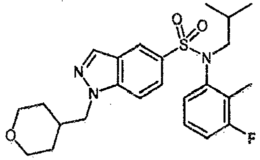 | Acide 1-(tétrahydro-pyran-4-ylméthyl)-2H-indazole-5-sulfonique (3-fluoro-2-méthyl-phényl)-isobutyl-amide |
| | Composé 43 |
| 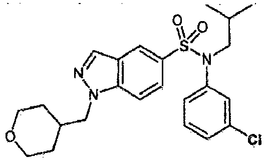 | Acide 1-(tétrahydro-pyran-4-ylmethyl)-1H-indazole-5-sulfonique (5-chloro-phényl)-isobutyl-amide |
| | Composé 44 |
| 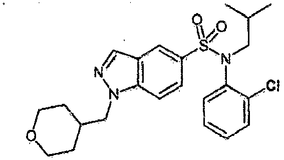 | Acide 1-(tétrahydro-pyran-4-ylméthyl)-1H-indazole-5-sulfonique (2-chloro-phenyl)-isobutyl-amide |
| | Composé 45 |
| 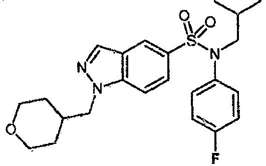 | Acide 1-(tétrahydro-pyran-4-ylméthyl)-1H-indazole-5-sulfonique (4-fluoro-phényl)-isobutyl-amide Composé 46 |
| 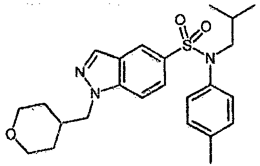 | Acide 1-(tétrahydro-pyran-4-ylméthyl)-1H-indazole-5-sulfonique isobutyl-p-tolyl-amide |
| | Composé 47 |
| 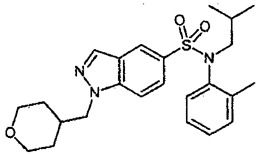 | Acide 1-(tétrahydro-pyran-4-ylméthyl)-1H-indazole-5-sulfonique isobutyl-o-tolyl-amide |
| | Composé 48 |
| 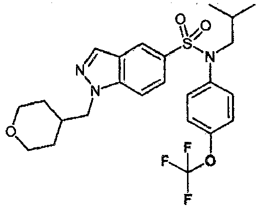 | Acide 1-(tétrahydro-pyran-4-ylméthyl)-1H-indazole-5-sulfonique (4-trifluorométhoxy-phényl)-isobutyl-amide |
| | Composé 49 |
| 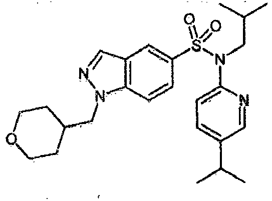 | Acide 1-(tétrahydro-pyran-4-ylméthyl)-1H-indazole-5-sulfonique isobutyl-(5-isopropyl-pyridin-2-yl)-amide |
| | Composé 50 |
| 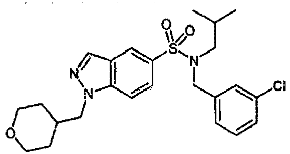 | Acide 1-(tétrahydro-pyran-4-ylméthyl)-1H-indazole-5-sulfonique (3-chloro-benzyl)-isobutyl-amide |
| | Composé 51 |
| 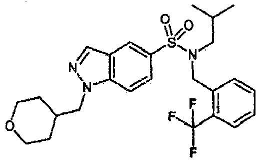 | Acide 1-(tétrahydro-pyran-4-ylméthyl)-1H-indazole-5-sulfonique isobutyl-(2-trifluorométhyl-benzyl)-amide |
| | Composé 52 |
| 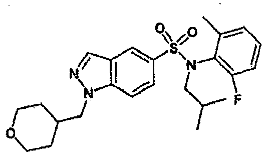 | Acide 1-(tétrahydro-pyran-4-ylméthyl)-1H-indazole-5-sulfonique (2-fluoro-6-méthyl-phényl)-isobutyl-amide |
| | Composé 53 |
| 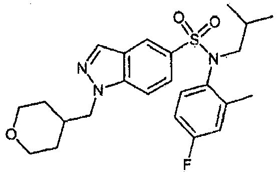 | Acide 1-(tétrahydro-pyran-4-ylméthyl)-1H-indazole-5-sulfonique (4-fluoro-2-méthyl-phényl)-isobutyl-amide |
| | Composé 54 |
| 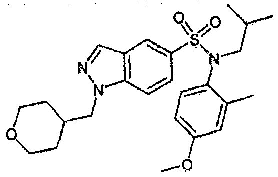 | Acide 1-(tétrahydro-pyran-4-ylméthyl)-1H-indazole-5-sulfonique isobutyl-(4-methoxy-2-methyl-phenyl)-amide |
| | Composé 55 |
| 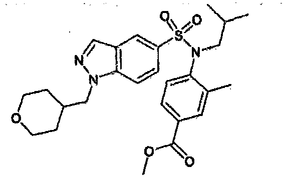 | Ester de l'acide méthyl 4-{sobutyl-[1-(tetrahydro-pyran-4-ylmethyl)-1H-indazole-5-sulfonyl]-amino}-3-methyl-benzoïque |
| | Composé 56 |
| 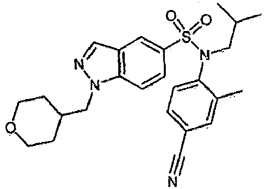 | Acide 1-(Tétrahydro-pyran-4-ylméthyl)-1H-indazole-5-sulfonique (4-cyano-2-méthyl-phényl)-isobutyl-amide |
| | Composé 57 |
| 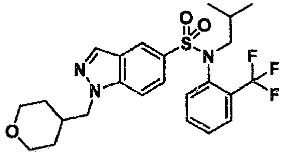 | Acide 1-(tétrahydro-pyran-4-ylméthyl)-1H-indazole-5-sulfonique isobutyl-(2-trifluorométhyl-phenyl)-amide |
| | Composé 58 |
| 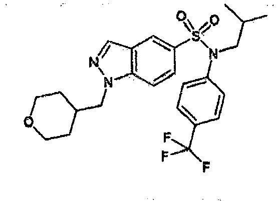 | Acide 1-(tétrahydro-pyran-4-ylméthyl)-1H-indazole-5-sulfonique isobutyl-(4-trifluorométhyl-phenyl)-amide |
| | Composé 59 |
| 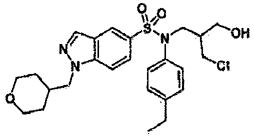 | Acide 1-(tétrahydro-pyran-4-ylmethyl)-1H-indazole-5-sulibnique (3-chloro-2-hydroxymethyl-propyl)-(4-éthyl-phényl)-amide |
| | Composé 60 |
| 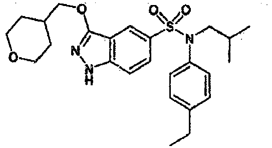 | Acide 3-(tétrahydro-pyran-4-ylméthoxy)-1H-indazole-5-sulfonique (4-éthyl-phényl)-isobutyl-amide |
| | Composé 61 |
| 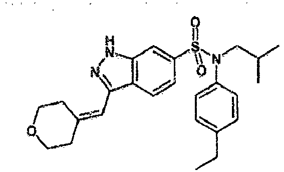 | Acide 3-(tétrahydro-pyran-4-ylideneméthyl)-1H-indazole-6-sulfonique(4-éthyl-phényl)-isobutyl-amide |
| | Composé 62 |
| 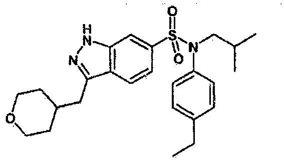 | Acide 3-(tétrahydro-pyran-4-ylméthyl)-1H-indazole-6-sulfonique (4-éthyl-phényl)-isobutyl-amide |
| | Composé 63 |
| 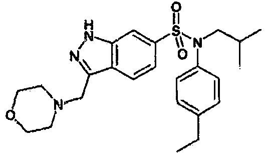 | Acide 3-morpholin-4-ylmethyl-1H-indazole-6-sulfonique (4-éthyl-phényl)-isobutyl-amide Composé 64 |
| 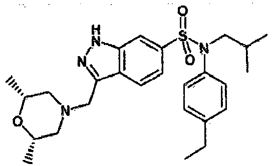 | Acide 3-((cis)-2,6-Dimethylmorpholin-4-ylmethyl)-1H-indazole-6-sulfonique(4-ethyl-phenyl)-isobutyl-amide Composé 65 |
| 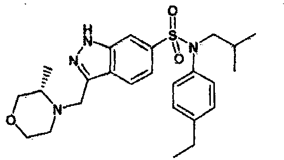 | Acide 3-((S)-3-méthyl-morpholin-4-ylméthyl)-1H-indazole-6-sulfonique (4-éthyl-phényl)-isobutyl-amide Composé 66 |
| 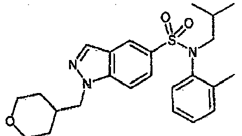 | Acide 1-(tétrahydro-pyran-4-ylméthyl)-1H-indazole-5-sulfonique isobutyl-o-tolyl-amide |
| | Composé 69 |
| 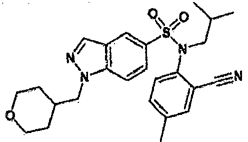 | Acide 1-(tétrahydro-pyran-4-ylméthyl)-1H-indazole-5-sulfonique(2-cyano-4-méthyl-phényl)-isobutyl-amide |
| | Composé 70 |
| 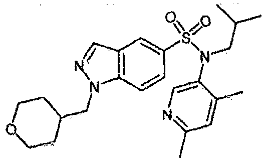 | Acide 1-(tetrabydro-pyran-4-ylmethyl-1H-indazole-5-sulfonique (4,6-diméthyl-pyridin-3-yl)-isobutyl-amide |
| | Composé 71 |
| 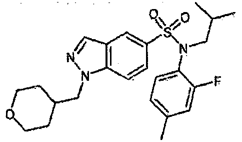 | Acide 1-(tétrahydro-pyran-4-ylméthyl)-1H-indazole-5-sulfonique(2-fluoro-4-méthyl-phényl)-isobutyl-amide |
| | Composé 73 |
| 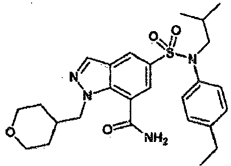 | Acide 5-[(4-éthyl-phényl)-isobutyl-sulfamoyl]-1-(tétrahydro-pyran-4-ylméthyl)-1H-indazole-7-carboxylique amide |
| | Composé 75 |

7. Composé selon l'une quelconque des revendications précédentes pour son utilisation en tant que médicament.

8. Composé selon l'une quelconque des revendications 1 à 6 pour son utilisation dans le traitement de l'acné.

9. Composé selon l'une quelconque des revendications 1 à 6 pour son utilisation dans le traitement du psoriasis.

10. Composition pharmaceutique comprenant un ou plusieurs composés tels que définis selon l'une quelconque des revendications 1 à 6.

11. Composition pharmaceutique telle que définie selon la revendication 10 pour son utilisation dans le traitement de l'acné, la dermatite atopique et/ou le psoriasis.

## Patentansprüche

1. Verbindung der Formel (I), pharmazeutisch unbedenkliche Additionssalze davon, Hydrate davon und/oder Solvate davon: 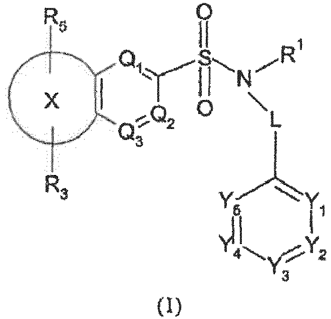 wobei in der Formel (I):
• L für eine Einfachbindung oder eine Methylengruppe CH₂ steht,
• X für einen cyclischen Rest steht, der aus den folgenden Resten X¹ und X² ausgewählt ist: 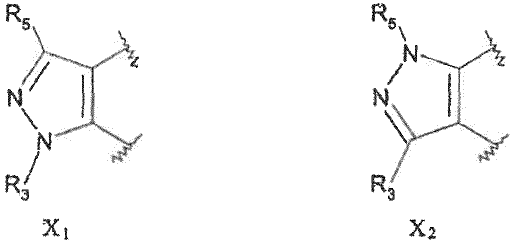
• ein oder zwei der Elemente Y¹, Y², Y³, Y⁴ und Y⁵ für ein Stickstoffatom steht bzw. stehen und die anderen Elemente einer Gruppe -CR² entsprechen oder jedes der Elemente Y¹, Y², Y³, Y⁴ und Y⁵ einer Gruppe -CR² entspricht,
• ein oder zwei der Elemente Q¹, Q² und Q³ für ein Stickstoffatom steht bzw. stehen und das bzw. die anderen Elemente einer Gruppe -CR^{2a} entspricht bzw. entsprechen oder jedes der Elemente Q¹, Q² und Q³ einer Gruppe -CR^{2a} entspricht,
• R¹ für einen linearen oder verzweigten C₃-C₅-Alkylrest, der gegebenenfalls durch eine Hydroxylgruppe und/oder ein Halogenatom substituiert ist, einen C₃-C₅-Cycloalkylrest, einen linearen oder verzweigten C₂-C₅-Alkenylrest, einen -CH₂-C₃-C₅-Cycloalkylrest, einen C₄-C₅-Heterocycloalkylrest oder einen -CH₂-C₄-C₆-Heterocycloalkylrest steht,
• R² für ein Wasserstoffatom oder ein Halogenatom, einen linearen oder verzweigten C₁-C₅-Alkylrest, einen linearen oder verzweigten C₂-C₄-Alkenylrest, einen C₁-C₄-Alkoxyrest, eine Cyanogruppe -CN, einen Rest -C(=O)R'², wobei R'² einen C₁-C₃-Alkoxyrest bedeutet, oder einen -CF₃-Rest steht; wobei die Alkyl-, Alkenyl- und Alkoxyreste durch ein oder mehrere Halogenatome substituiert sein können;
• R^{2a} für ein Wasserstoffatom oder ein Halogenatom, einen linearen oder verzweigten C₁-C₅-Alkylrest, einen linearen oder verzweigten C₂-C₄-Alkenylrest, einen C₁-C₄-Alkoxyrest, eine -CN-Gruppe, eine Hydroxygruppe -OH, eine Gruppe -CH(R^{3a}) OH, eine Carboxylgruppe -COOH, eine Carbamoylgruppe -CONR^{2c}R^{2d}, eine Amidogruppe -NR^{2c}COR^{2d}, eine Gruppe -SO₂R^{2c}, eine Gruppe -SOR^{2c} oder eine Gruppe -S(=O) (=NH-R^{2c}) steht, wobei die Alkyl-, Alkenyl- und Alkoxyreste durch ein oder mehrere Halogenatome substituiert sein können,
• R^{2c} und R^{2d} gleich oder verschieden sind und für ein Wasserstoffatom oder einen linearen oder verzweigten C₁-C₅-Alkylrest stehen;
• R^{3a} für ein Wasserstoffatom oder einen linearen oder verzweigten C₁-C₅-Alkylrest steht;
• R³ für ein Wasserstoffatom, ein Halogenatom, eine Gruppe (CHR⁶)ₙ-(Z)ₒ- (CHR'⁶)ₚ-R⁷ oder eine Gruppe CH=R⁷ stehen,
• n, o und p gleich oder verschieden sind und für null oder eine natürliche ganze Zahl im Bereich von 1 bis 3 stehen,
• Z für eine zweiwertige Gruppe steht, die aus einer Methylengruppe -CH₂-, einer Aminogruppe -NH- und einem Sauerstoffatom -O- ausgewählt ist,
• R⁶ und R'⁶ gleich oder verschieden sind und für ein Wasserstoffatom, eine Methylgruppe -CH₃, eine -OH-Gruppe, eine Hydroxymethylgruppe oder eine Carboxylfunktion -COOH stehen,
• R⁷ für:
- ein Wasserstoffatom oder ein Halogenatom,
- eine Gruppe COOR'⁷, wobei R'⁷ (C₁) Alkyl (C₆) - heterocyclus bedeutet,
- einen nichtkationischen Heterocycloalkylrest, der gegebenenfalls durch ein oder mehrere Halogenatome, eine oder mehrere lineare oder verzweigte C₁-C₃-Alkylgruppen, eine oder mehrere -OH-Gruppen, eine oder mehrere Carbonylfunktionen, eine oder mehrere lineare oder verzweigte C₁-C₄-Hydroxyalkylgruppen, ein oder mehrere Amino, eine oder mehrere Gruppen -C(=O)R^{7a} oder eine oder mehrere Gruppen S(=O)₂R^{7a} substituiert ist; wobei R^{7a} für einen linearen oder verzweigten C₁-C₃-Alkylrest, einen linearen oder verzweigten C₁-C₃-Alkoxyrest oder einen Aminorest N(R^{8a}) (R^{8b}) steht,
- einen nichtkationischen C₃-C₆-Cycloalkylrest, der gegebenenfalls durch einen oder mehrere Methylreste, ein oder mehrere Halogenatome, eine Cyanogruppe -CN oder eine oder mehrere Gruppen -COR¹³ substituiert ist; wobei R¹³ für einen linearen oder verzweigten C₁-C₃-Alkoxyrest oder eine Hydroxygruppe steht,
- einen nichtkationischen aromatischen oder heteroaromatischen Rest, der gegebenenfalls durch ein oder mehrere Halogenatome, eine oder mehrere C₁-C₃-Alkylgruppen, die gegebenenfalls durch ein oder mehrere Halogenatome substituiert sind, eine oder mehrere C₁-C₃-Alkoxygruppen, eine oder mehrere Aminogruppen -NR¹¹R¹², eine oder mehrere Gruppen -COR¹¹, eine oder mehrere Gruppen -COOR¹¹, eine oder mehrere Amidogruppen -CONR¹¹R¹², eine oder mehrere Gruppen -SOR¹¹, eine oder mehrere Gruppen -SO₂R¹¹, eine oder mehrere Gruppen -NHCOR¹¹, eine oder mehrere Gruppen -NHCOOR¹¹, eine oder mehrere Gruppen -SO₂NR¹¹R¹² oder eine oder mehrere -CN-Gruppen substiuiert ist; wobei R¹¹ und R¹² gleich oder verschieden sind und für ein Wasserstoffatom, einen Hydroxyrest -OH oder einen linearen oder verzweigten C₁-C₃-Alkylrest, der gegebenenfalls durch ein oder mehrere Halogenatome substituiert ist, stehen, steht,
• dann, wenn R³ für eine Gruppe -CH=R⁷ steht, R⁷ nicht für ein Wasserstoffatom, ein Halogenatom oder eine Gruppe COOR'⁷ steht,
• R⁵ für ein Wasserstoffatom oder ein Halogenatom, einen linearen oder verzweigten C₁-C₃-Alkylrest, der gegebenenfalls durch ein oder mehrere Halogenatome substituiert ist, einen Aminorest -NH₂, einen C₄-C₅-Heterocyclylrest, einen OCH₂-C₄-C₅-Heterocyclylrest oder einen Rest CH₂R'^{7a} steht, wobei R'^{7a} einen Methoxyrest, eine Hydroxygruppe -OH, eine Gruppe -CH₂COOH, eine Gruppe -CH(R^{5b})OH, eine Carboxylgruppe -COOH, eine -CN-Gruppe oder eine Thioxofunktion bedeutet,
• R^{5b} für ein Wasserstoffatom, einen linearen oder verzweigten C₁-C₃-Alkylrest, der durch eine oder mehrere Carboxylfunktionen substituiert ist, oder einen Cyclopropylrest steht;
• R^{8a} und R^{8b} gleich oder verschieden sind und ein Wasserstoffatom, einen linearen oder verzweigten C₁-C₃-Alkylrest oder einen Cyclopropylrest bedeuten.

2. Verbindung der Formel (I) nach Anspruch 1, **dadurch gekennzeichnet, dass** R⁷ für einen heterocyclischen Rest steht, der aus den folgenden Heterocyclen ausgewählt ist: 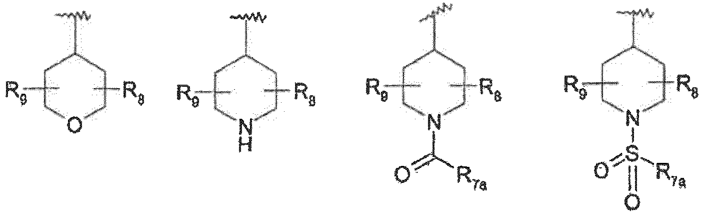 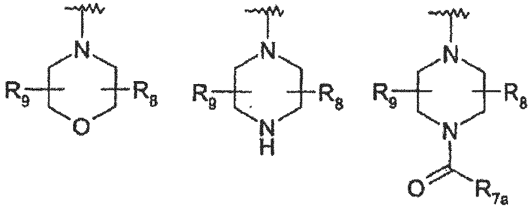 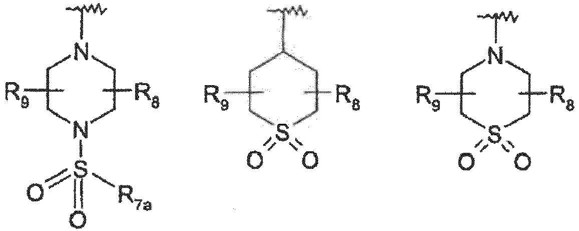 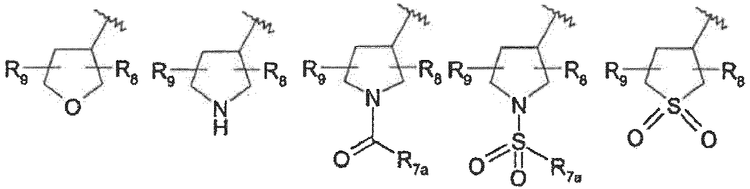 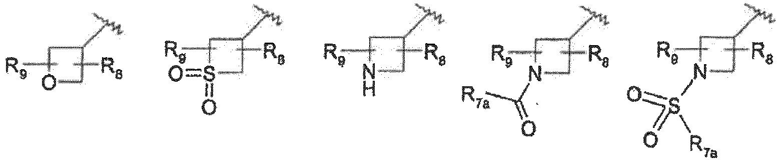 in denen:
- R₇ₐ für einen linearen oder verzweigten C₁-C₃-Alkylrest, einen linearen oder verzweigten C₁-C₃-Alkoxyrest oder einen Aminorest N(R^{8a}) (R^{8b}) steht,
- R^{8a} und R^{8b} gleich oder verschieden sind und ein Wasserstoffatom, einen linearen oder verzweigten C₁-C₃-Alkylrest oder einen Cyclopropylrest bedeuten,
- R₈ und R₉ gleich oder verschieden sind und für ein Wasserstoffatom, einen linearen oder verzweigten C₁-C₃-Alkylrest, eine Hydroxygruppe -OH, eine Carbonylfunktion =O, einen C₁-Hydroxyalkylrest (-CH₂OH) oder eine Aminogruppe NH₂ stehen,
- R₈ und R₉ zusammen mit den Kohlenstoffatomen, an die sie gebunden sind, einen 5- bis 7-gliedrigen heterocyclischen Ring bilden können.

3. Verbindung der Formel (I) nach Anspruch 1, **dadurch gekennzeichnet, dass** R⁷ für einen aromatischen oder heteroaromatischen Rest steht, der aus den folgenden Resten ausgewählt ist: 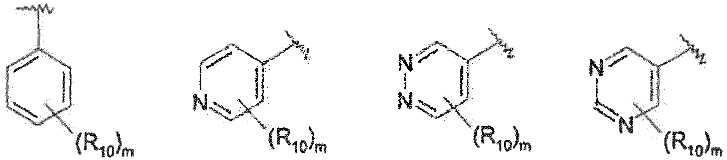 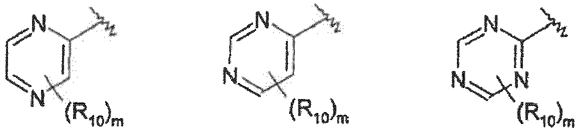 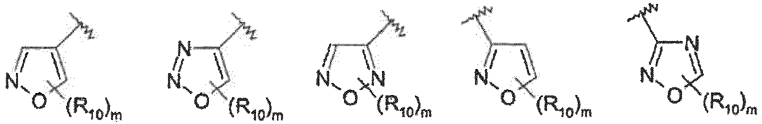 in denen:
- R₁₀ für ein Wasserstoffatom oder ein Halogenatom, eine lineare oder verzweigte C₁-C₃-Alkylgruppe, die gegebenenfalls durch ein oder mehrere Halogenatome substituiert ist, eine C₁-C₃-Alkoxygruppe, eine Aminogruppe -NR¹¹R¹², eine Gruppe -COR¹¹, eine Gruppe -COOR¹¹, eine Amidogruppe -CONR¹¹R¹², eine Gruppe -SOR¹¹, eine Gruppe -SO₂R¹¹, eine Gruppe -NHCOR¹¹, eine Gruppe -NHCOOR¹¹, eine Gruppe -SO₂NR¹¹R¹² oder eine -CN-Gruppe substiuiert ist; wobei R¹¹ und R¹² gleich oder verschieden sind und für ein Wasserstoffatom oder einen linearen oder verzweigten C₁-C₃-Alkylrest, der gegebenenfalls durch ein oder mehrere Halogenatome substituiert ist, stehen,
- m für null oder eine natürliche ganze Zahl im Bereich von 1 bis 3 steht.

4. Verbindung der Formel (I) nach Anspruch 1, **dadurch gekennzeichnet, dass** sie aus den Verbindungen der Formel (II) sowie den pharmazeutisch unbedenklichen Additionssalzen davon, Hydraten davon und/oder Solvaten davon ausgewählt ist bzw. sind: 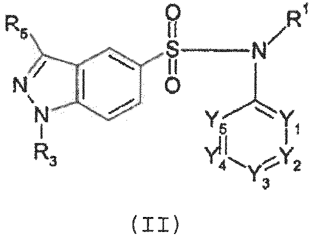 wobei in der Formel (II) R¹, R³, R⁵ und Y¹ bis Y⁵ die gleichen Bedeutungen wie in der Formel (I) gemäß Anspruch 1 haben.

5. Verbindung der Formel (I) nach Anspruch 1, **dadurch gekennzeichnet, dass** sie aus den Verbindungen der Formel (III) sowie den pharmazeutisch unbedenklichen Additionssalzen davon, Hydraten davon und/oder Solvaten davon ausgewählt ist: 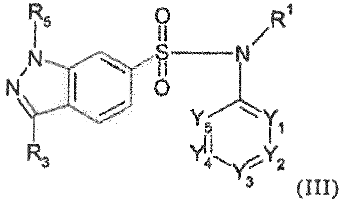 wobei in der Formel (III) R¹, R³, R⁵ und Y¹ bis Y⁵ die gleichen Bedeutungen wie in der Formel (I) gemäß Anspruch 1 haben.

6. Verbindung der Formel (I) nach einem der vorhergehenden Ansprüche, **dadurch gekennzeichnet, dass** sie aus den folgenden Verbindungen ausgewählt ist:
| | |
|---|---|
| 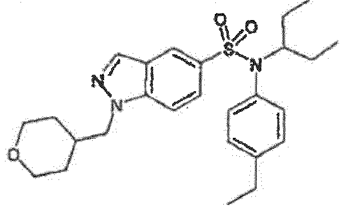 | 1-(Tetrahydropyran-4-ylmethyl)-1H-indazol-5-sulfonsäure(4-ethylphenyl)(1-ethylpropyl)-amid |
| | Verbindung 1 |
| 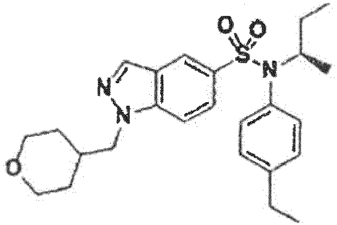 | 1-(Tetrahydropyran-4-ylmethyl)-1H-indazol-5-sulfonsäure((R)-sec-butyl) (4-ethylphenyl)amid |
| | Verbindung 2 |
| 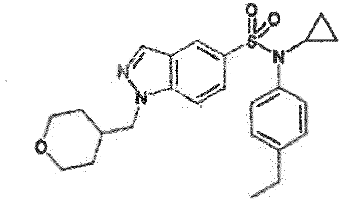 | 1-(Tetrahydropyran-4-ylmethyl)-1H-indazol-5-sulfonsäurecyclopropyl-(4-ethylphenyl) amid |
| | Verbindung 3 |
| 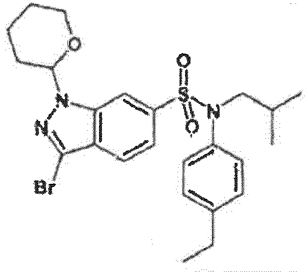 | 3-Brom-1-(tetrahydro-pyran-2-yl)-1H-indazol-6-sulfonsäure(4-ethyl-phenyl)isobutylamid |
| | Verbindung 4 |
| 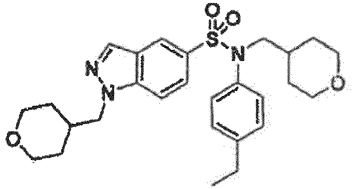 | 1-(Tetrahydropyran-4-ylmethyl)-1H-indazol-5-sulfonsäure(4-ethylphenyl)(tetrahydropyran-4-ylmethyl)amid |
| | Verbindung 5 |
| 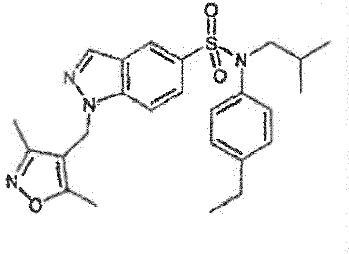 | 1-((3,5-Dimethyl-isoxazol-4-yl)methyl)-N-(4-ethylphenyl)-N-isobutyl-1H-indazol-5-sulfonamid |
| | Verbindung 6 |
| 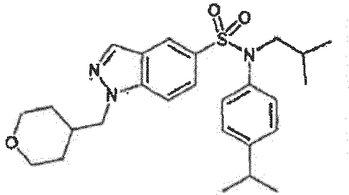 | 1-(Tetrahydropyran-4-ylmethyl)-1H-indazol-5-sulfonsäurebutyl(4-isopropylphenyl)amid |
| | Verbindung 7 |
| 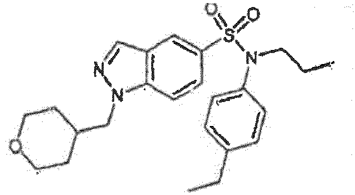 | 1-(Tetrahydropyran-4-ylmethyl)-1H-indazol-5-sulfonsäure(4-ethylphenyl)propyl |
| | Verbindung 8 |
| 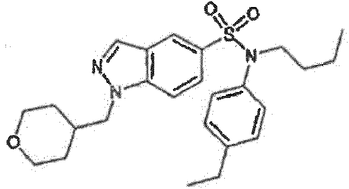 | 1-(Tetrahydropyran-4-ylmethyl)-1H-indazol-5-sulfonsäurebutyl(4-ethylphenyl)amid |
| | Verbindung 9 |
| 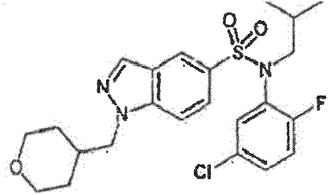 | 1-(Tetrahydropyran-4-ylmethyl)-1H-indazol-5-sulfonsäure(5-chlor-2-fluorphenyl)isobutylamid |
| | Verbindung 10 |
| 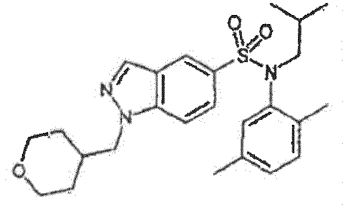 | 1-(Tetrahydropyran-4-ylmethyl)-1H-indazol-5-sulfonsäure(2,5-dimethylphenyl)isobutylamid |
| | Verbindung 11 |
| 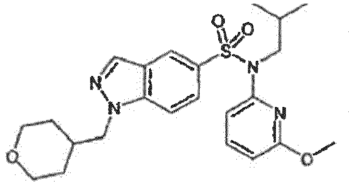 | 1-(Tetrahydropyran-4-ylmethyl)-1H-indazol-5-sulfonsäure(3-methoxypyridin-2-yl)-isobutylamid |
| | Verbindung 12 |
| 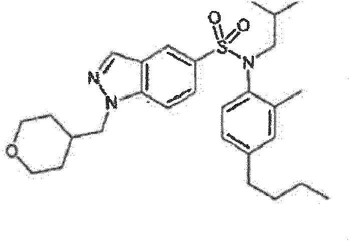 | 1-(Tetrahydropyran-4-ylmethyl)-1H-indazol-5-sulfonsäure(4-butyl-2-methylphenyl)isobutylamid |
| | Verbindung 13 |
| 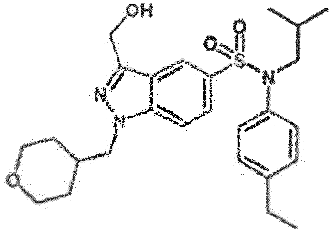 | N-(4-Ethylphenyl)-N-isobutyl-1-((tetrahydro-2H-pyran-4-yl)methyl)-1H-indazol-5-sulfonamid |
| | Verbindung 14 |
| 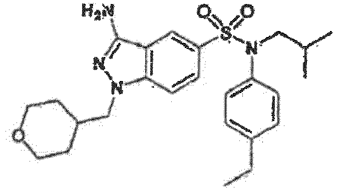 | 3-Amino-1H-indazol-5-sulfonsäure(4-ethylphenyl)isobutylamid |
| | Verbindung 15 |
| 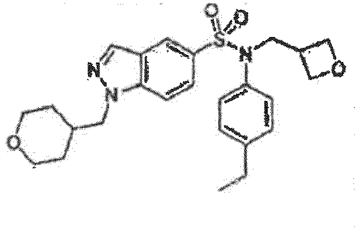 | 1-(Tetrahydropyran-4-ylmethyl)-1H-indazol-5-sulfonsäure(4-ethylphenyl)oxetan-3-ylmethylamid |
| | Verbindung 16 |
| 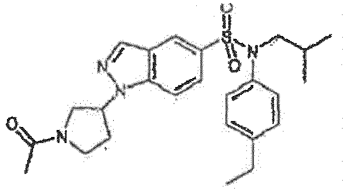 | 1-(1-Acetylpyrrolidin-3-yl)-1H-indazol-5-sulfonsäure(4-ethylphenyl)isobutylamid |
| | Verbindung 17 |
| 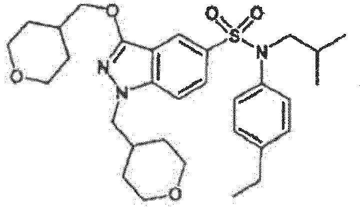 | 3-(Tetrahydropyran-4-ylmethoxy)-1-(tetra-hydropyran-4-ylmethyl)-1H-indazol-5-sulfonsäure(4-ethylphenyl)-isobutylamid |
| | Verbindung 18 |
| 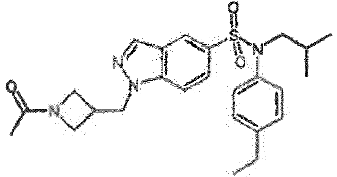 | 1-(3,5-Dimethylisoxazol-4-ylmethyl)-1H-indazol-5-sulfonsäure(4-ethylphenyl)isobutylamid |
| | Verbindung 19 |
| 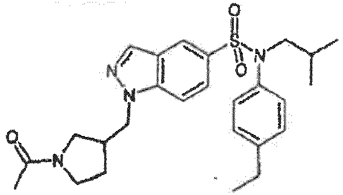 | 1-((1-Acetylpyrrolidin-3-yl)methyl)-N-(4-ethylphenyl)-N-isobutyl-1H-indazol-5-sulfonamid |
| | Verbindung 20 |
| 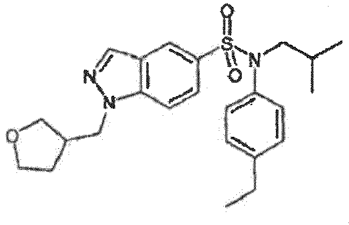 | N-(4-Ethylphenyl)-N-isobutyl-1-((tetra-hydrofuran-3-yl)methyl)-1H-indazol-5-sulfonamid |
| | Verbindung 21 |
| 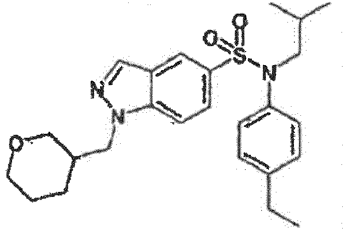 | N-(4-Ethylphenyl)-N-isobutyl-1-((tetrahydro-2H-pyran-3-yl)methyl)-1H-indazol-5-sulfonamid |
| | Verbindung 22 |
| 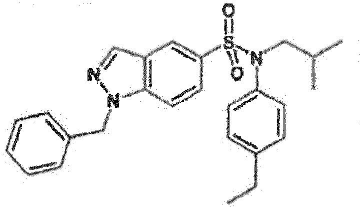 | 1-Benzyl-N-(4-ethylphenyl)-N-isobutyl-1H-indazol-5-sulfonamid |
| | Verbindung 23 |
| 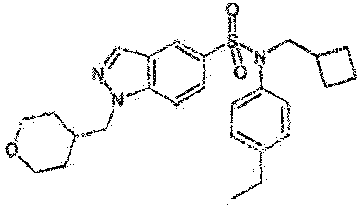 | N-(Cyclobutylmethyl)-N-(4-ethylphenyl)-1-((tetrahydro-2H-pyran-4-yl)methyl)-1H-indazol-5-sulfonamid |
| | Verbindung 24 |
| 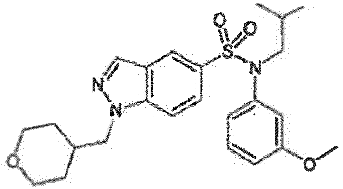 | 1-(Tetrahydropyran-4-ylmethyl)-1H-indazol-5-sulfonsäure(3-methoxy-phenyl)isobutylamid |
| | Verbindung 25 |
| 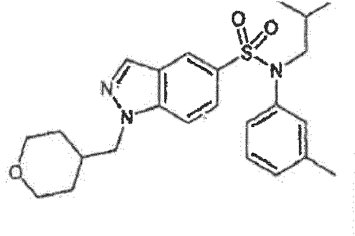 | 1-(Tetrahydropyran-4-ylmethyl)-1H-indazol-5-sulfonsäure(3-methyl-phenyl)isobutylamid |
| | Verbindung 26 |
| 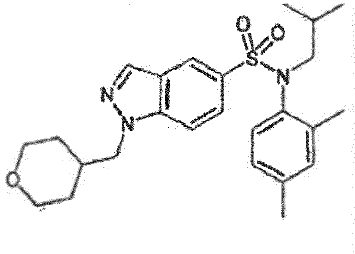 | 1-(Tetrahydropyran-4-ylmethyl)-1H-indazol-5-sulfonsäure(2,4-dimethylphenyl)isobutylamid |
| | Verbindung 27 |
| 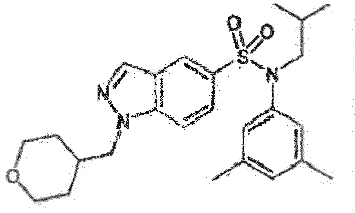 | 1-(Tetrahydropyran-4-ylmethyl)-1H-indazol-5-sulfonsäure(3,5-dimethylphenyl)isobutylamid |
| | Verbindung 28 |
| 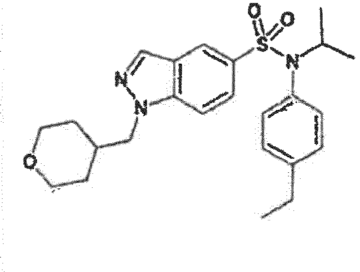 | N-(4-Ethylphenyl)-N-isopropyl-1-((tetrahydro-2H-pyran-4-yl)methyl)-1H-indazol-5-sulfonamid |
| | Verbindung 29 |
| 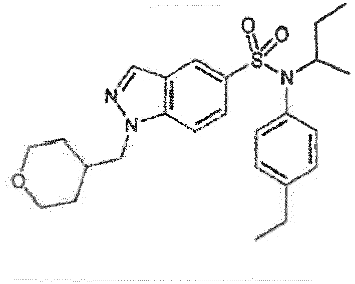 | 1-(Tetrahydropyran-4-ylmethyl)-1H-indazol-5-sulfonsäuresec-butyl(4-ethylphenyl) amid |
| | Verbindung 30 |
| 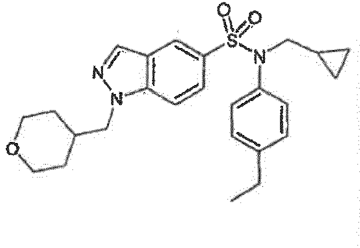 | N-(Cyclopropylmethyl)-N-(4-ethylphenyl)-1-((tetrahydro-2H-pyran-4-yl)methyl)-1H-indazol-5-sulfonamid |
| | Verbindung 31 |
| 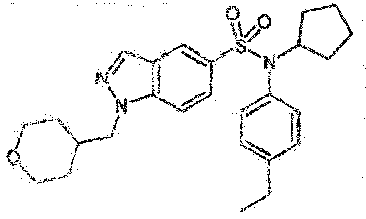 | 1-(Tetrahydropyran-4-ylmethyl)-1H-indazol-5-sulfonsäurecyclopentyl-(4-ethylphenyl) amid |
| | Verbindung 32 |
| 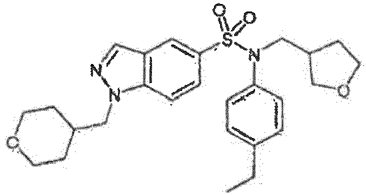 | 1-(Tetrahydropyran-4-ylmethyl)-1H-indazol-5-sulfonsäure(4-ethyl-phenyl)(tetrahydrofuran-3-ylmethyl)amid |
| | Verbindung 33 |
| 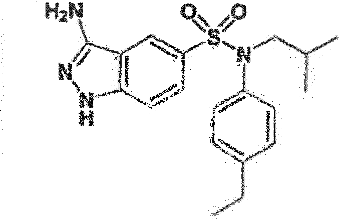 | 3-Amino-1H-indazol-5-sulfonsäure(4-ethyl-phenyl)isobutylamid |
| | Verbindung 34 |
| 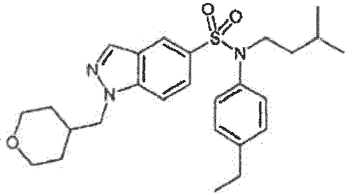 | N-(4-Ethylphenyl)-N-isopentyl-1-((tetrahydro-2H-pyran-4-yl)methyl)-1H-indazol-5-sulfonamid |
| | Verbindung 35 |
| 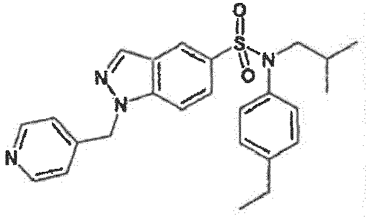 | N-(4-Ethylphenyl)-N-isobutyl-1-(pyridin-4-ylmethyl)-1H-indazol-5-sulfonamid |
| | Verbindung 36 |
| 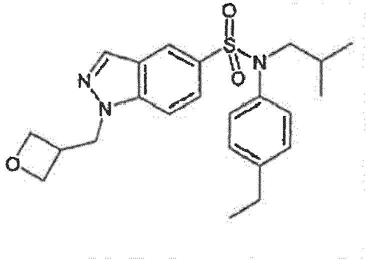 | N-(4-Ethylphenyl)-N-isobutyl-1-(oxetan-3-ylmethyl)-1H-indazol-5-sulfonamid |
| | Verbindung 37 |
| 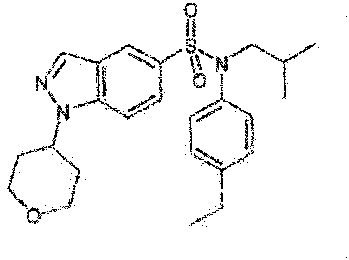 | 1-(Tetrahydropyran-4-yl)-1H-indazol-5-sulfonsäure(4-ethylphenyl)-isobutylamid |
| | Verbindung 38 |
| 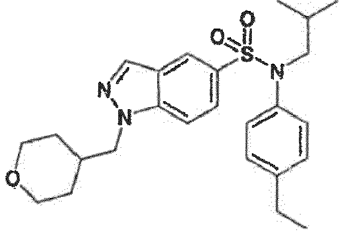 | N-(4-Ethylphenyl)-N-isobutyl-1-((tetrahydro-2H-pyran-4-yl)methyl)-1H-indazol-5-sulfonamid |
| | Verbindung 39 |
| 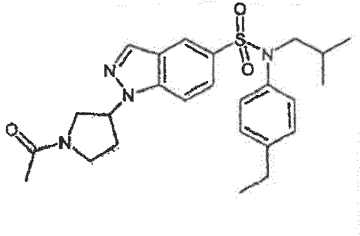 | 1-(1-Acetylpyrrolidin-3-yl)-1H-indazol-5-sulfonsäure(4-ethyl-phenyl)isobutylamid |
| | Verbindung 40 |
| 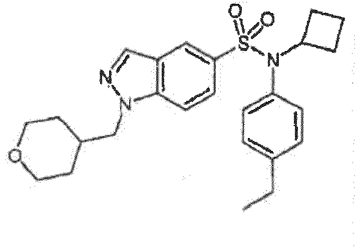 | 1-(Tetrahydropyran-4-ylmethyl)-1H-indazol-5-sulfonsäurecyclobutyl(4-ethylphenyl)amid |
| | Verbindung 41 |
| 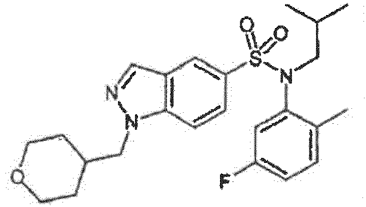 | 1-(Tetrahydropyran-4-ylmethyl)-1H-indazol-5-sulfonsäure(5-fluor-2-methylphenyl)isobutylamid |
| | Verbindung 42 |
| 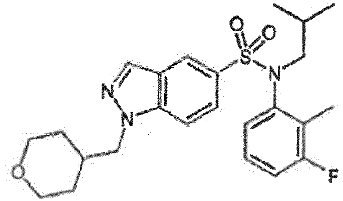 | 1-(Tetrahydropyran-4-ylmethyl)-2H-indazol-5-sulfonsäure(3-fluor-2-methylphenyl)isobutylamid |
| | Verbindung 43 |
| 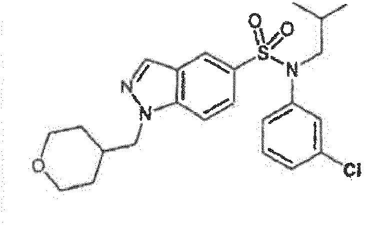 | 1-(Tetrahydropyran-4-ylmethyl)-1H-indazol-5-sulfonsäure(5-chlorphenyl)isobutylamid |
| | Verbindung 44 |
| 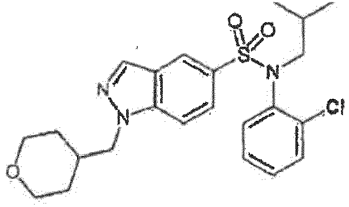 | 1-(Tetrahydropyran-4-ylmethyl)-1H-indazol-5-sulfonsäure(2-chlorphenyl)isobutylamid |
| | Verbindung 45 |
| 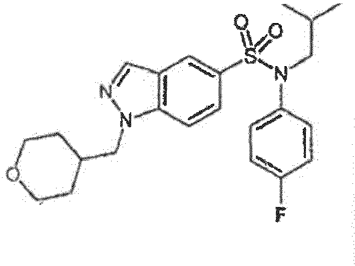 | 1-(Tetrahydropyran-4-ylmethyl)-1H-indazol-5-sulfonsäure(4-fluorphenyl)isobutylamid |
| | Verbindung 46 |
| 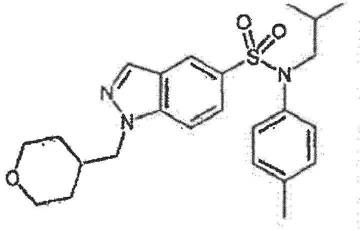 | 1-(Tetrahydropyran-4-ylmethyl)-1H-indazol-5-sulfonsäureisobutyl-p-tolylamid |
| | Verbindung 47 |
| 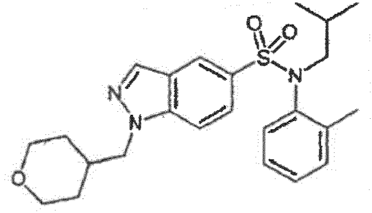 | 1-(Tetrahydropyran-4-ylmethyl)-1H-indazol-5-sulfonsäureisobutyl-o-tolylamid |
| | Verbindung 48 |
| 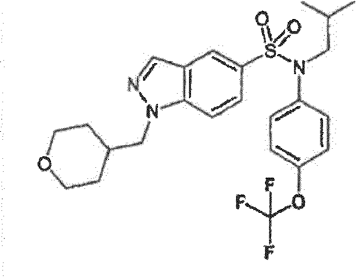 | 1-(Tetrahydropyran-4-ylmethyl)-1H-indazol-5-sulfonsäure(4-trifluor-methoxyphenyl)isobutylamid |
| | Verbindung 49 |
| 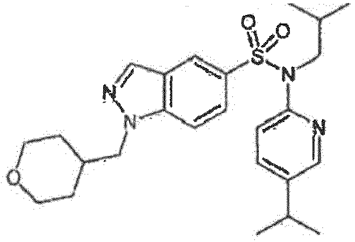 | 1-(Tetrahydropyran-4-ylmethyl)-1H-indazol-5-sulfonsäureisobutyl(5-isopropylpyridin-2-yl)amid |
| | Verbindung 50 |
| 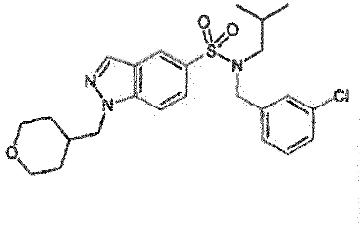 | 1-(Tetrahydropyran-4-ylmethyl)-1H-indazol-5-sulfonsäure(3-chlorbenzyl)isobutylamid |
| | Verbindung 51 |
| 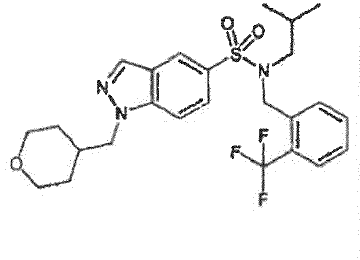 | 1-(Tetrahydropyran-4-ylmethyl)-1H-indazol-5-sulfonsäureisobutyl(2-trifluormethylbenzyl)-amid |
| | Verbindung 52 |
| 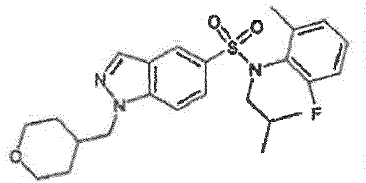 | 1-(Tetrahydropyran-4-ylmethyl)-1H-indazol-5-sulfonsäure(2-fluor-6-methylphenyl)isobutylamid |
| | Verbindung 53 |
| 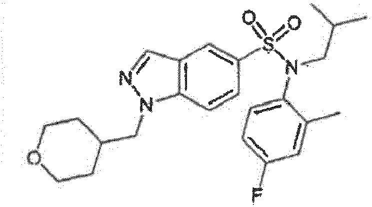 | 1-(Tetrahydropyran-4-ylmethyl)-1H-indazol-5-sulfonsäure(4-fluor-2-methylphenyl)isobutylamid |
| | Verbindung 54 |
| 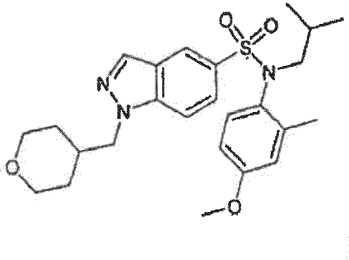 | 1-(Tetrahydropyran-4-ylmethyl)-1H-indazol-5-sulfonsäureisobutyl(4-methoxy-2-methylphenyl)amid |
| | Verbindung 55 |
| 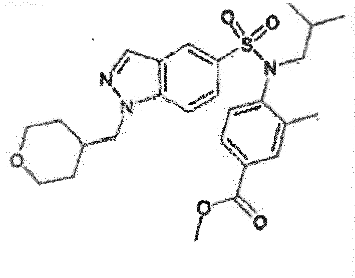 | 4-{Isobutyl[1-(tetrahydropyran-4-ylmethyl)-1H-indazol-5-sulfonyl]-amino}-3-methylbenzoesäuremethylester |
| | Verbindung 56 |
| 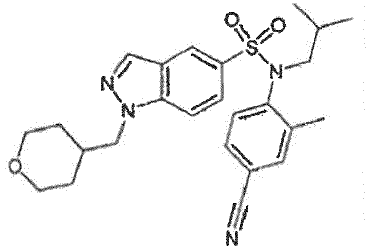 | 1-(Tetrahydropyran-4-ylmethyl)-1H-indazol-5-sulfonsäure(4-cyano-2-methylphenyl)isobutylamid |
| | Verbindung 57 |
| 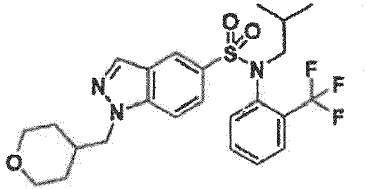 | 1-(Tetrahydropyran-4-ylmethyl)-1H-indazol-5-sulfonsäureisobutyl(2-trifluormethylphenyl)-amid |
| | Verbindung 58 |
| 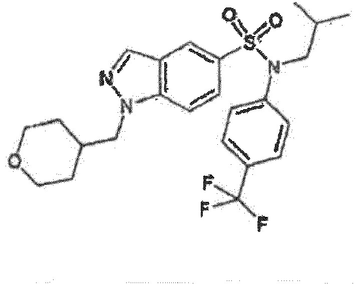 | 1-(Tetrahydropyran-4-ylmethyl)-1H-indazol-5-sulfonsäureisobutyl(4-trifluormethylphenyl)-amid |
| | Verbindung 59 |
| 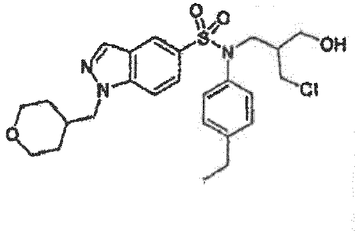 | 1-(Tetrahydropyran-4-ylmethyl)-1H-indazol-5-sulfonsäure(3-chlor-2-hydroxymethylpropyl)(4-ethylphenyl)amid |
| | Verbindung 60 |
| 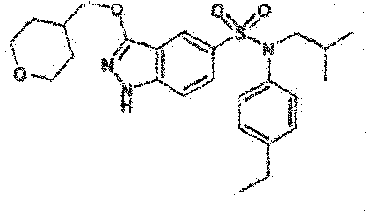 | 3-(Tetrahydropyran-4-ylmethoxy)-1H-indazol-5-sulfonsäure(4-ethyl-phenyl)isobutylamid |
| | Verbindung 61 |
| 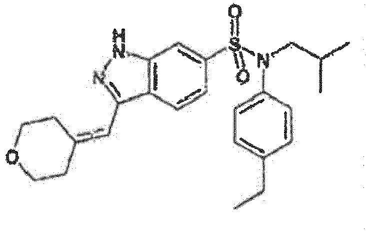 | 3-(Tetrahydropyran-4-ylidenmethyl)-1H-indazol-6-sulfonsäure(4-ethylphenyl)isobutylamid |
| | Verbindung 62 |
| 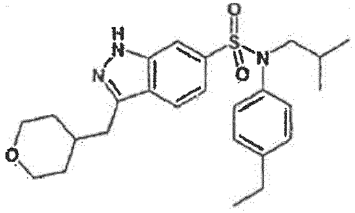 | 3-(Tetrahydropyran-4-ylmethyl)-1H-indazol-6-sulfonsäure(4-ethyl-phenyl)isobutylamid |
| | Verbindung 63 |
| 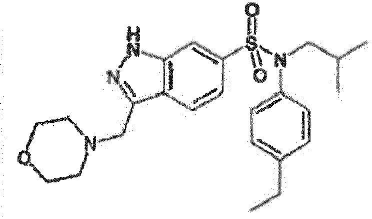 | 3-Morpholin-4-ylmethyl-1H-indazol-6-sulfonsäure(4-ethylphenyl)-isobutylamid |
| | Verbindung 64 |
| 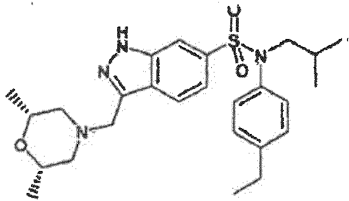 | 3-((cis)-2,6-Dimethyl-morpholin-4-ylmethyl)-1H-indazol-6-sulfonsäure(4-ethylphenyl)-isobutylamid |
| | Verbindung 65 |
| 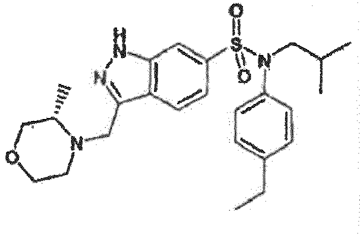 | 3-((S)-3-Methyl-morpholin-4-ylmethyl)-1H-indazol-6-sulfonsäure(4-ethylphenyl)-isobutylamid |
| | Verbindung 66 |
| 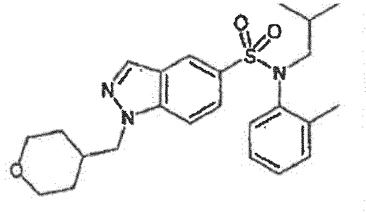 | 1-(Tetrahydropyran-4-ylmethyl)-1H-indazol-5-sulfonsäureisobutyl-o-tolylamid |
| | Verbindung 69 |
| 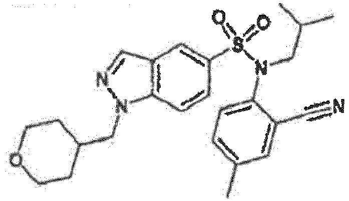 | 1-(Tetrahydropyran-4-ylmethyl)-1H-indazol-5-sulfonsäure(2-cyano-4-methylphenyl)isobutylamid |
| | Verbindung 70 |
| 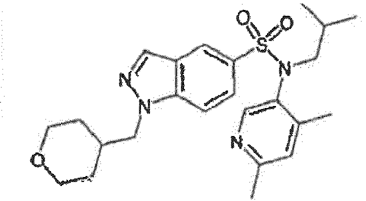 | 1-(Tetrahydropyran-4-ylmethyl)-1H-indazol-5-sulfonsäure(4,6-dimethylpyridin-3-yl)-isobutylamid |
| | Verbindung 71 |
| 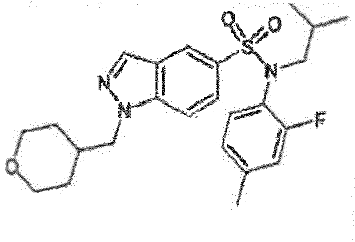 | 1-(Tetrahydropyran-4-ylmethyl)-1H-indazol-5-sulfonsäure(2-fluor-4-methylphenyl)isobutylamid |
| | Verbindung 73 |
| 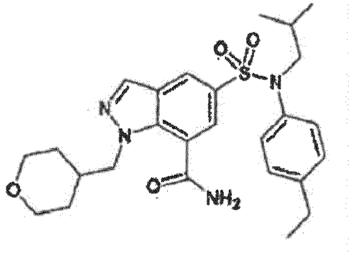 | 5-[(4-Ethylphenyl)-isobutylsulfamoyl]-1-(tetrahydropyran-4-ylmethyl)-1H-indazol-7-carbonsäureamid |
| | Verbindung 75 |

7. Verbindung nach einem der vorhergehenden Ansprüche zur Verwendung als Medikament.

8. Verbindung nach einem der Ansprüche 1 bis 6 zur Verwendung bei der Behandlung von Akne.

9. Verbindung nach einem der Ansprüche 1 bis 6 zur Verwendung bei der Behandlung von Psoriasis.

10. Pharmazeutische Zusammensetzung, umfassend eine oder mehrere Verbindungen gemäß einem der Ansprüche 1 bis 6.

11. Pharmazeutische Zusammensetzung gemäß Anspruch 10 zur Verwendung bei der Behandlung von Akne, atopischer Dermatitis und/oder Psoriasis.

## Claims

1. Compound of formula (I), and the pharmaceutically acceptable addition salts thereof, hydrates thereof and/or solvates thereof: 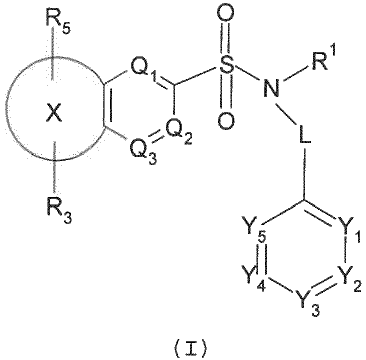 in which formula (I):
• L represents a single bond or a methylene group CH₂,
• X represents a cyclic radical chosen from the radicals X₁ and X₂ below: 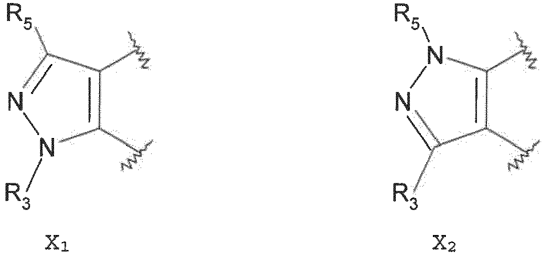
• one or two of the elements Y¹, Y², Y³, Y⁴ and Y⁵ represent a nitrogen atom and the other elements correspond to a group -CR², or each of the elements Y¹, Y², Y³, Y⁴ and Y⁵ corresponds to a group -CR²,
• one or two of the elements Q¹, Q² and Q³ represent a nitrogen atom and the other elements correspond to a group -CR^{2a}, or each of the elements Q¹, Q² and Q³ corresponds to a group -CR^{2a},
• R¹ represents a linear or branched C₃-C₅ alkyl radical, optionally substituted with a hydroxyl group and/or a halogen atom, a C₃-C₅ cycloalkyl radical, a linear or branched C₂-C₅ alkenyl radical, a -CH₂-(C₃-C₅)cycloalkyl radical, a C₄-C₅ heterocycloalkyl radical, a -CH₂-(C₄-C₆)heterocycloalkyl radical,
• R² represents a hydrogen atom or a halogen atom, a linear or branched C₁-C₅ alkyl radical, a linear or branched C₂-C₄ alkenyl radical, a C₁-C₄ alkoxy radical, a cyano group -CN, a radical - C(=O)R'² with R'² denoting a C₁-C₃ alkoxy radical, a -CF₃ radical; said alkyl, alkenyl and alkoxy radicals possibly being substituted with one or more halogen atoms,
• R^{2a} represents a hydrogen atom or a halogen atom, a linear or branched C₁-C₅ alkyl radical, a linear or branched C₂-C₄ alkenyl radical, a C₁-C₄ alkoxy radical, a-CN group, a hydroxyl group -OH, a group -CH (R^{3a}) OH, a carboxylic group -COOH, a carbamoyl group -CONR^{2c}R^{2d}, an amido group -NR^{2c}COR^{2d}, a group -SO2R^{2c}, a group -SOR^{2c}, a group -S(=O) (=NH-R^{2c}), said alkyl, alkenyl and alkoxy radicals possibly being substituted with one or more halogen atoms,
• R^{2c} and R^{2d}, which may be identical or different, represent a hydrogen atom or a linear or branched C₁-C₅ alkyl radical;
• R^{3a} represents a hydrogen atom or a linear or branched C₁-C₅ alkyl radical,
• R³ represents a hydrogen atom, a halogen atom, a group (CHR⁶)ₙ-(Z)ₒ-(CHR'⁶)ₚ-R⁷ or a group CH=R⁷,
• n, o and p, which may be identical or different, represent zero or a natural integer ranging from 1 to 3,
• Z represents a divalent group chosen from a methylene group -CH₂-, an amino group -NH- and an oxygen atom -O-,
• R⁶ and R'⁶, which may be identical or different, represent a hydrogen atom, a methyl group -CH₃, a group -OH, a hydroxymethyl group or a carboxylic function-COOH,
• R⁷ represents:
- a hydrogen or a halogen atom,
- a group COOR'⁷ with R'⁷ denoting (C₁) alkyl (C₆) heterocycle,
- a non-cationic heterocycloalkyl radical optionally substituted with one or more halogen atoms, one or more linear or branched C₁-C₃ alkyl groups, one or more -OH groups, one or more carbonyl functions, one or more linear or branched C₁-C₄ hydroxyalkyl groups, one or more amino groups, one or more groups - C(=O)R^{7a}, one or more groups S(=O)₂R^{7a}; R^{7a} representing a linear or branched C₁-C₃ alkyl radical, a linear or branched C₁-C₃ alkoxy radical, or an amino radical N(R^{8a})(R^{8b}),
- a non-cationic C₃-C₆ cycloalkyl radical optionally substituted with one or more methyl radicals, one or more halogen atoms, a cyano group -CN or one or more groups -COR¹³; R¹³ denoting a linear or branched C₁-C₃ alkoxy radical, or a hydroxyl group,
- an aromatic or heteroaromatic, non-cationic radical optionally substituted with one or more halogen atoms, one or more linear or branched C₁-C₃ alkyl groups optionally substituted with one or more halogen atoms, one or more C₁-C₃ alkoxy groups, one or more amino groups -NR¹¹R¹², one or more groups - COR¹¹, one or more groups -COOR¹¹, one or more amido groups-CONR¹¹R¹², one or more groups - SOR¹¹, one or more groups -SO₂R¹¹, one or more groups -NHCOR¹¹, one or more groups -NHCOOR¹¹, one or more groups -SO₂NR¹¹R¹² or one or more -CN groups; R¹¹ and R¹², which may be identical or different, representing a hydrogen atom, a hydroxyl radical -OH or a linear or branched C₁-C₃ alkyl radical optionally substituted with one or more halogen atoms;
• when R³ represents a group -CH=R⁷, then R⁷ does not represent a hydrogen atom, a halogen atom or a group COOR'⁷,
• R⁵ represents a hydrogen atom or a halogen atom, a linear or branched C₁-C₃ alkyl radical optionally substituted with one or more halogen atoms; an amino radical -NH₂, a C₄-C₅ heterocyclic radical, an OCH₂-(C₄-C₅) heterocyclic radical, a radical CH₂R'^{7a} with R'^{7a} denoting a methoxy radical, a hydroxyl group -OH, a -CH₂COOH group, a group - CH(R^{5b})OH, a carboxylic group -COOH, a -CN group, a thioxo function,
• R^{5b} represents a hydrogen atom, a linear or branched C₁-C₃ alkyl radical optionally substituted with one or more carboxylic functions; a cyclopropyl radical,
• R^{8a} and R^{8b}, which may be identical or different, denote a hydrogen atom, a linear or branched C₁-C₃ alkyl radical or a cyclopropyl radical.

2. Compound of formula (I) according to Claim 1, **characterized in that** R⁷ represents a heterocyclic radical chosen from the following heterocycles: 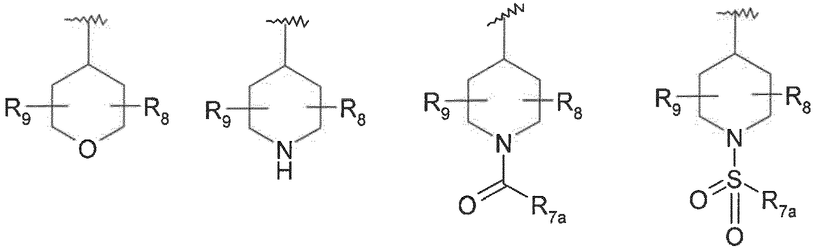 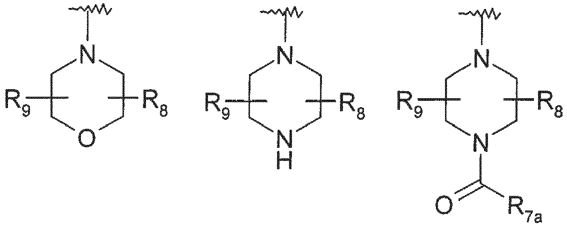 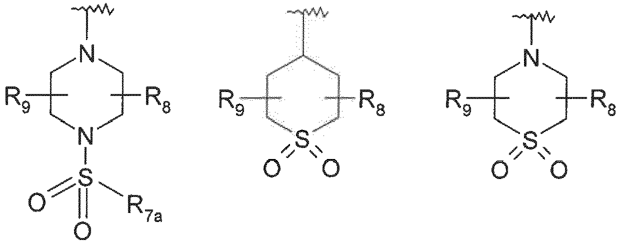 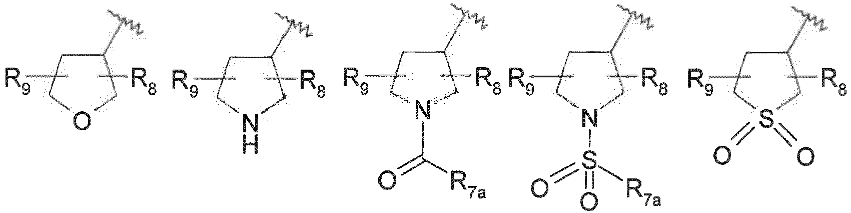 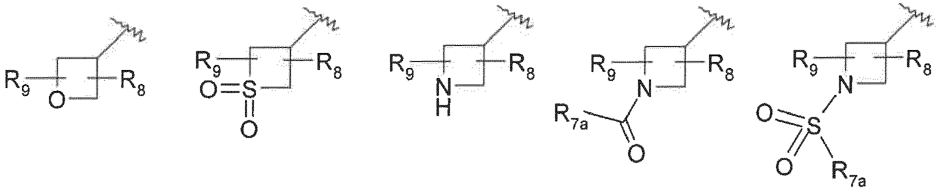 in which:
- R₇ₐ represents a linear or branched C₁-C₃ alkyl radical, a linear or branched C₁-C₃ alkoxy radical or an amino radical N(R^{8a})(R^{8b}),
- R^{8a} and R^{8b}, which may be identical or different, denote a hydrogen atom, a linear or branched C₁-C₃ alkyl radical or a cyclopropyl radical,
- R₈ and R₉, which may be identical or different, represent a hydrogen atom, a linear or branched C₁-C₃ alkyl radical, a hydroxyl group -OH, a carbonyl function =O, a hydroxyalkyl radical C₁ (-CH₂OH) or an amino group NH2,
- R₈ and R₉ can form, together with the carbon atoms to which they are attached, a 5- to 7-membered, carbocyclic ring.

3. Compound of formula (I) according to Claim 1, **characterized in that** R⁷ represents an aromatic or heteroaromatic radical chosen from: 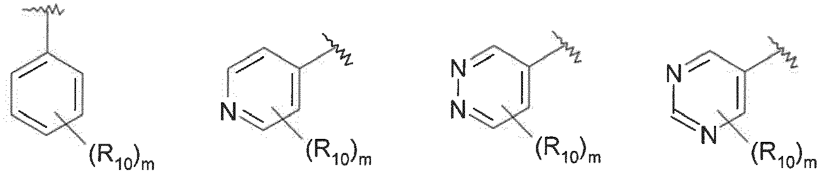 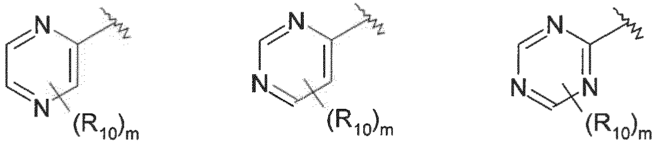 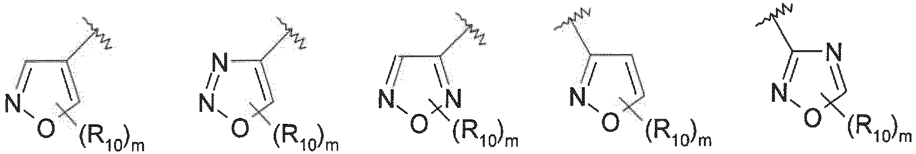 in which:
- R₁₀ represents a hydrogen atom or a halogen atom, one linear or branched C₁-C₃ alkyl group optionally substituted with one or more halogen atoms, one C₁-C₃ alkoxy group, one amino group - NR¹¹R¹², one group -COR¹¹, one group -COOR¹¹, one amido group -CONR¹¹R¹², one group -SOR¹¹, one group - SO₂R¹¹, one group -NHCOR¹¹, one group -NHCOOR¹¹, one group -SO₂NR¹¹R¹² or one -CN group; R¹¹ and R¹², which may be identical or different, representing a hydrogen atom or a linear or branched C₁-C₃ alkyl radical optionally substituted with one or more halogen atoms,
m denotes zero or a natural integer ranging
from 1 to 3.

4. Compound of formula (I) according to Claim 1, **characterized in that** it is chosen from the compound(s) of formula (II), and also the pharmaceutically acceptable addition salts thereof, hydrates thereof and/or solvates thereof: 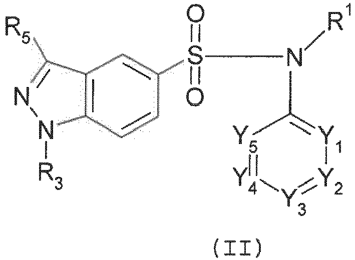 in which formula (II) R¹, R³, R⁵ and Y¹ to Y⁵ have the same meanings as in formula (I) as defined in claim 1.

5. Compound of formula (I) according to Claim 1, **characterized in that** it is chosen from the compound(s) of formula (III), and also the pharmaceutically acceptable addition salts thereof, hydrates thereof and/or solvates thereof: 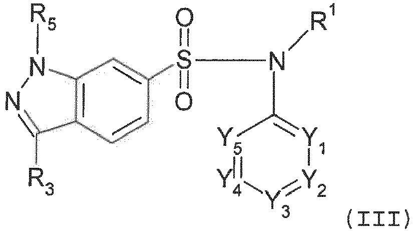 in which formula (III) R¹, R³, R⁵ and Y¹ to Y⁵ have the same meanings as in formula (I) as defined in claim 1.

6. Compound of formula (I) according to any one of the preceding claims, **characterized in that** it is chosen from the following compounds:
| | |
|---|---|
| 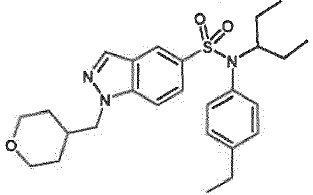 | 1-(tetrahydropyran-4-ylmethyl)-1H-indazole-5-sulfonic acid (4-ethylphenyl)(1-ethylp ropyl)amide |
| | Compound 1 |
| 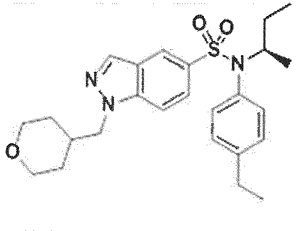 | 1-(tetrahydropyran-4-ylmethyl)-1H-indazole-5-sulfonic acid ((R)-sec-butyl)(4-ethylphenyl)amide |
| | Compound 2 |
| 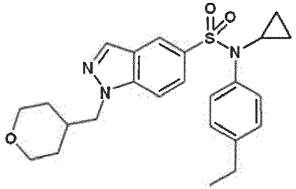 | 1-(tetrahydropyran-4-ylmethyl)-1H-indazole-5-sulfonic acid cyclopropyl(4-ethyl phenyl) amide |
| | Compound 3 |
| 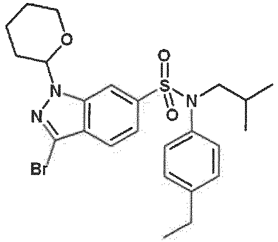 | 3-bromo-1-(tetrahydropyran-2-yl)-1H-indazole-6-sulfonic acid(4-ethylphenyl)isobutylamid e |
| | Compound 4 |
| 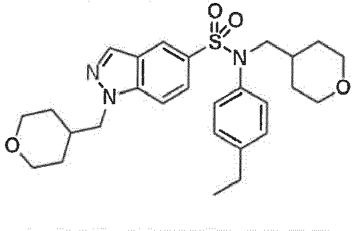 | 1-(tetrahydropyran-4-ylmethyl)-1H-indazole-5-sulfonic acid (4-ethylphenyl)(tetrahydrop yran-4-ylmethyl) amide |
| | Compound 5 |
| 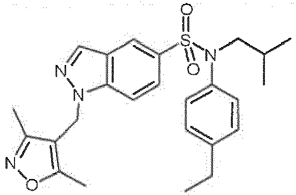 | 1-((3,5-dimethylisoxazol-4-yl)methyl)-N-(4-ethylphenyl)-N-isobutyl-1H-indazole-5-sulfonamide |
| | Compound 6 |
| 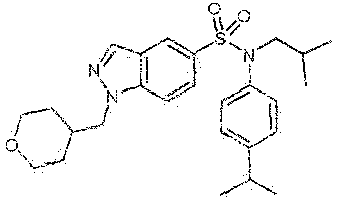 | 1-(tetrahydropyran-4-ylmethyl)-1H-indazole-5-sulfonic acid butyl(4-isopropylphenyl)amide |
| | Compound 7 |
| 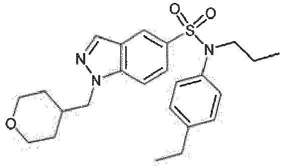 | 1-(tetrahydropyran-4-ylmethyl)-1H-indazole-5-sulfonic acid (4-ethylphenyl)propyl |
| | Compound 8 |
| 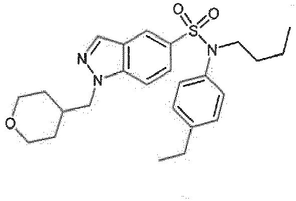 | 1-(tetrahydropyran-4-ylmethyl)-1H-indazole-5-sulfonic acid butyl(4-ethylphenyl)amide |
| | Compound 9 |
| 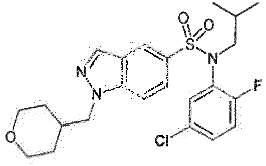 | 1-(tetrahydropyran-4-ylmethyl)-1H-indazole-5-sulfonic acid (5-chloro-2-fluorophenyl)isobutylami de |
| | Compound 10 |
| 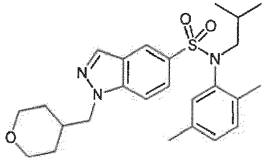 | 1-(tetrahydropyran-4-ylmethyl)-1H-indazole-5-sulfonic acid (2,5-dimethylphenyl)isobutyla mide |
| | Compound 11 |
| 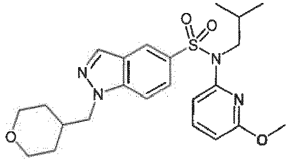 | 1-(tetrahydropyran-4-ylmethyl)-1H-indazole-5-sulfonic acid (3-methoxypyridin-2-yl)isobutylamide |
| | Compound 12 |
| 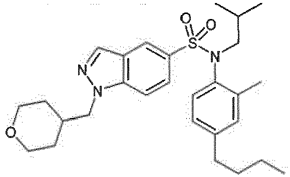 | 1-(tetrahydropyran-4-ylmethyl)-1H-indazole-5-sulfonic acid (4-butyl-2-methylphenyl)isobutylami de |
| | Compound 13 |
| 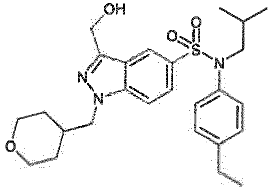 | N-(4-ethylphenyl)-N-isobutyl-1-((tetrahydro-2H-pyran-4-yl)methyl)-1H-indazole-5-sulfonamide |
| | Compound 14 |
| 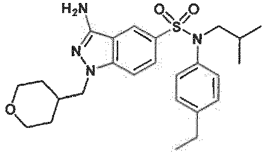 | 3-amino-1H-indazole-5-sulfonic acid (4-ethylphenyl)isobutylamid e |
| | Compound 15 |
| 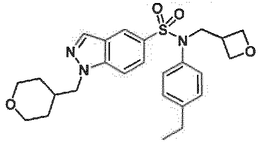 | 1-(tetrahydropyran-4-ylmethyl)-1H-indazole-5-sulfonic acid (4-ethylphenyl)oxetan-3-ylmethylamide |
| | Compound 16 |
| 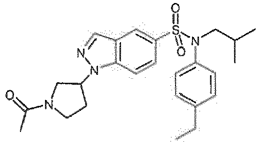 | 1-(1-acetylpyrrolidin-3-yl)-1H-indazole-5-sulfonic acid (4-ethylphenyl)isobutylamid e |
| | Compound 17 |
| 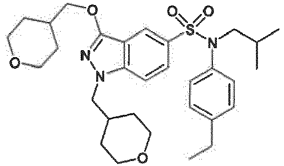 | 3-(tetrahydropyran-4-ylmethoxy)-1-(tetrahydropyran-4-ylmethyl)-1H-indazole-5-sulfonic acid (4-ethylphenyl)isobutylamid e |
| | Compound 18 |
| 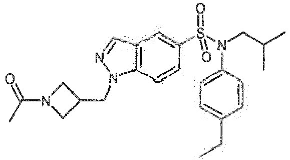 | 1-(3,5-dimethylisoxazol-4-ylmethyl)-1H-indazole-5-sulfonic acid (4-ethylphenyl)isobutylamid e |
| | Compound 19 |
| 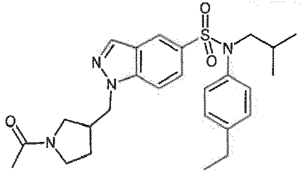 | 1-((1-acetylpyrrolidin-3-yl)methyl)-N-(4-ethylphenyl)-N-isobutyl-1H-indazole-5-sulfonamide |
| | Compound 20 |
| 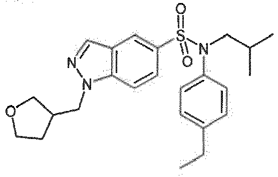 | N-(4-ethylphenyl)-N-isobutyl-1-((tetrahydrofuran-3-yl)methyl)-1H-indazole-5-sulfonamide |
| | Compound 21 |
| 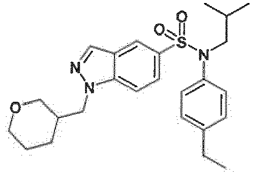 | N-(4-ethylphenyl)-N-isobutyl-1-((tetrahydro-2H-pyran-3-yl)methyl)-1H-indazole-5-sulfonamide |
| | Compound 22 |
| 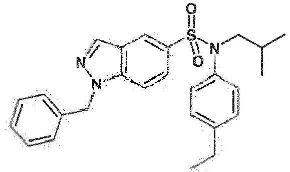 | 1-benzyl-N-(4-ethylphenyl)-N-isobutyl-1H-indazole-5-sulfonamide |
| | Compound 23 |
| 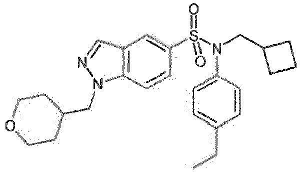 | N-(cyclobutylmethyl)-N-(4-ethylphenyl)-1-((tetrahydro-2H-pyran-4-yl)methyl)-1H-indazole-5-sulfonamide |
| | Compound 24 |
| 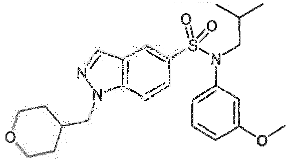 | 1-(tetrahydropyran-4-ylmethyl)-1H-indazole-5-sulfonic acid (3-methoxyphenyl)isobutylam ide |
| | Compound 25 |
| 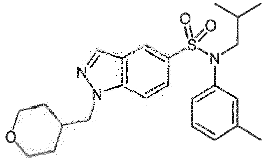 | 1-(tetrahydropyran-4-ylmethyl)-1H-indazole-5-sulfonic acid (3-methylphenyl)isobutylami de |
| | Compound 26 |
| 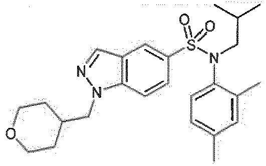 | 1-(tetrahydropyran-4-ylmethyl)-1H-indazole-5-sulfonic acid (2,4-dimethylphenyl)isobutyla mide |
| | Compound 27 |
| 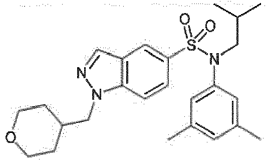 | 1-(tetrahydropyran-4-ylmethyl)-1H-indazole-5-sulfonic acid (3,5-dimethylphenyl)isobutyla mide |
| | Compound 28 |
| 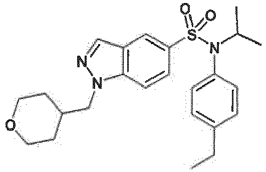 | N-(4-ethylphenyl)-N-isopropyl-1-((tetrahydro-2H-pyran-4-yl)methyl)-1H-indazole-5-sulfonamide |
| | Compound 29 |
| 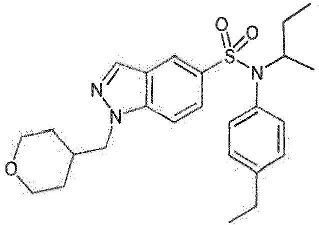 | 1-(tetrahydropyran-4-ylmethyl)-1H-indazole-5-sulfonic acid sec-butyl(4-ethylphenyl)amide |
| | Compound 30 |
| 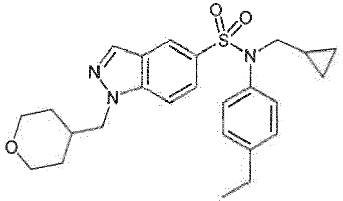 | N-(cyclopropylmethyl)-N-(4-ethylphenyl)-1-((tetrahydro-2H-pyran-4-yl)methyl)-1H-indazole-5-sulfonamide |
| | Compound 31 |
| 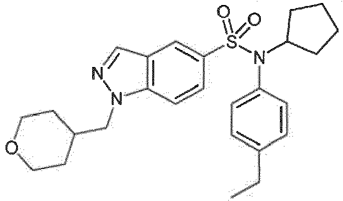 | 1-(tetrahydropyran-4-ylmethyl)-1H-indazole-5-sulfonic acid cyclopentyl(4-ethylphenyl) amide |
| | Compound 32 |
| 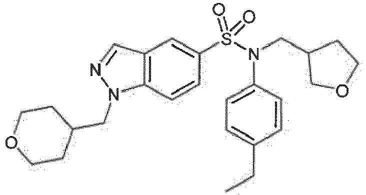 | 1-(tetrahydropyran-4-ylmethyl)-1H-indazole-5-sulfonic acid(4-ethylphenyl)(tetrahydrof uran-3-ylmethyl)amide |
| | Compound 33 |
| 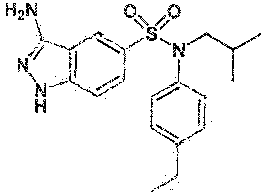 | 3-amino-1H-indazole-5-sulfonic acid (4-ethylphenyl)isobutylamid e |
| | Compound 34 |
| 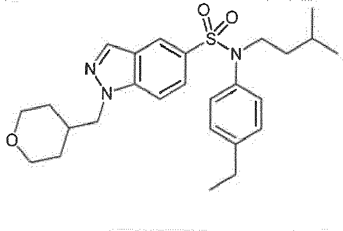 | N-(4-ethylphenyl)-N-isopentyl-1-((tetrahydro-2H-pyran-4-yl)methyl)-1H-indazole-5-sulfonamide |
| | Compound 35 |
| 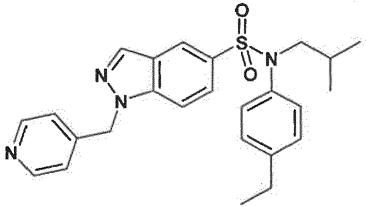 | N-(4-ethylphenyl)-N-isobutyl-1-(pyridin-4-ylmethyl)-1H-indazole-5-sulfonamide |
| | Compound 36 |
| 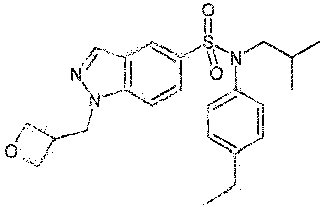 | N-(4-ethylphenyl)-N-isobutyl-1-(oxetan-3-ylmethyl)-1H-indazole-5-sulfonamide |
| | Compound 37 |
| 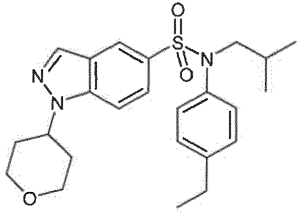 | 1-(tetrahydropyran-4-yl)-1H-indazole-5-sulfonic acid (4-ethylphenyl)isobutylamid e |
| | Compound 38 |
| 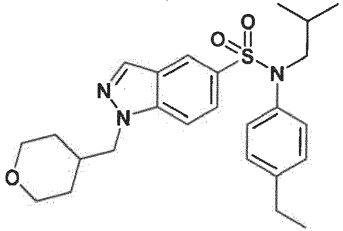 | N-(4-ethylphenyl)-N-isobutyl-1-((tetrahydro-2H-pyran-4-yl)methyl)-1H-indazole-5-sulfonamide |
| | Compound 39 |
| 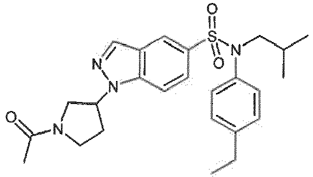 | 1-(1-acetylpyrrolidin-3-yl)-1H-indazole-5-sulfonic acid (4-ethylphenyl)isobutylamid e |
| | Compound 40 |
| 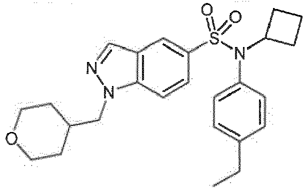 | 1-(tetrahydropyran-4-ylmethyl)-1H-indazole-5-sulfonic acid cyclobutyl(4-ethylphenyl) amide |
| | Compound 41 |
| 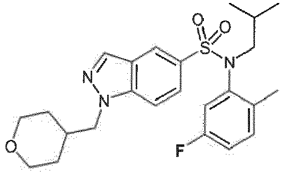 | 1-(tetrahydropyran-4-ylmethyl)-1H-indazole-5-sulfonic acid (5-fluoro-2-methylphenyl)isobutylami de |
| | Compound 42 |
| 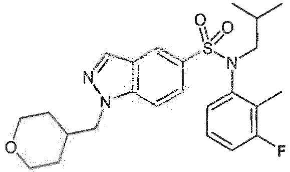 | 1-(tetrahydropyran-4-ylmethyl)-2H-indazole-5-sulfonic acid (3-fluoro-2-methylphenyl)isobutylami de |
| | Compound 43 |
| 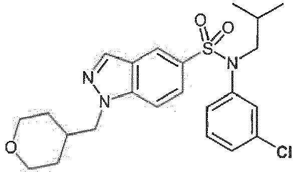 | 1-(tetrahydropyran-4-ylmethyl)-1H-indazole-5-sulfonic acid (5-chlorophenyl)isobutylami de |
| | Compound 44 |
| 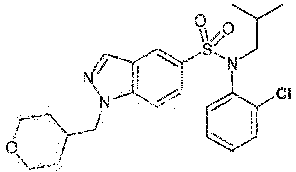 | 1-(tetrahydropyran-4-ylmethyl)-1H-indazole-5-sulfonic acid (2-chlorophenyl)isobutylami de |
| | Compound 45 |
| 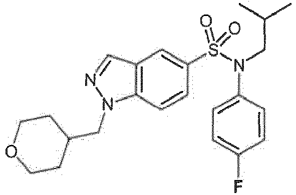 | 1-(tetrahydropyran-4-ylmethyl)-1H-indazole-5-sulfonic acid (4-fluorophenyl)isobutylami de |
| | Compound 46 |
| 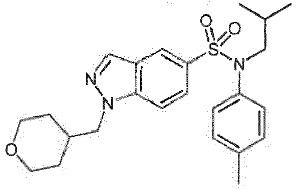 | 1-(tetrahydropyran-4-ylmethyl)-1H-indazole-5-sulfonic acid isobutyl-p-tolylamide |
| | Compound 47 |
| 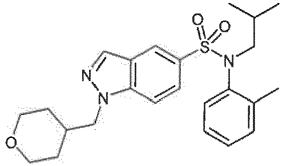 | 1-(tetrahydropyran-4-ylmethyl)-1H-indazole-5-sulfonic acid isobutyl-o-tolylamide |
| | Compound 48 |
| 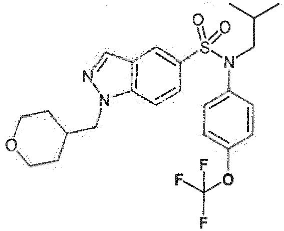 | 1-(tetrahydropyran-4-ylmethyl)-1H-indazole-5-sulfonic acid (4-trifluoromethoxyphenyl)i sobutylamide |
| | Compound 49 |
| 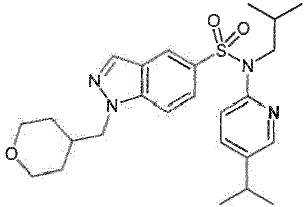 | 1-(tetrahydropyran-4-ylmethyl)-1H-indazole-5-sulfonic acid isobutyl(5-isopropylpyridin-2-yl)amide |
| | Compound 50 |
| 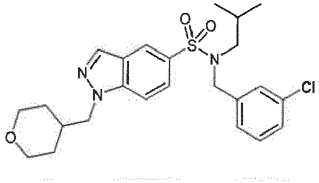 | 1-(tetrahydropyran-4-ylmethyl)-1H-indazole-5-sulfonic acid (3-chlorobenzyl)isobutylamide |
| | Compound 51 |
| 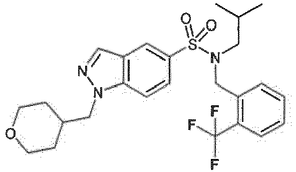 | 1-(tetrahydropyran-4-ylmethyl)-1H-indazole-5-sulfonic acid isobutyl(2-trifluoromethylbenzyl)am ide |
| | Compound 52 |
| 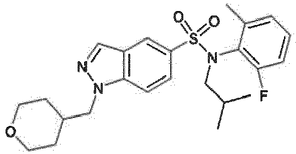 | 1-(tetrahydropyran-4-ylmethyl)-1H-indazole-5-sulfonic acid (2-fluoro-6-methylphenyl)isobutylami de |
| | Compound 53 |
| 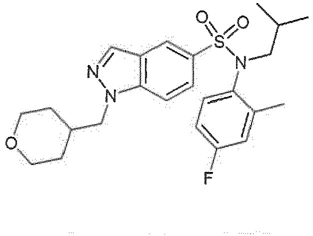 | 1-(tetrahydropyran-4-ylmethyl)-1H-indazole-5-sulfonic acid (4-fluoro-2-methylphenyl)isobutylami de |
| | Compound 54 |
| 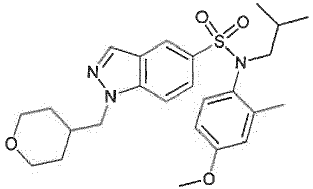 | 1-(tetrahydropyran-4-ylmethyl)-1H-indazole-5-sulfonic acid isobutyl(4-methoxy-2-methylphenyl) amide |
| | Compound 55 |
| 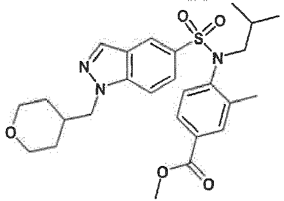 | methyl 4-{isobutyl[1-(tetrahydropyran-4-ylmethyl)-1H-indazole-5-sulfonyl]amino}-3-methylbenzoate |
| | Compound 56 |
| 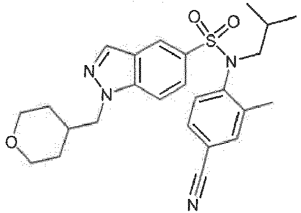 | 1-(tetrahydropyran-4-ylmethyl)-1H-indazole-5-sulfonic acid (4-cyano-2-methylphenyl)isobutylami de |
| | Compound 57 |
| 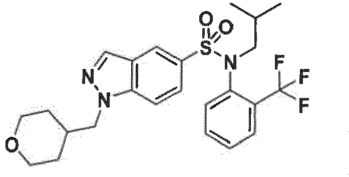 | 1-(tetrahydropyran-4-ylmethyl)-1H-indazole-5-sulfonic acid isobutyl(2-trifluoromethylphenyl)am ide |
| | Compound 58 |
| 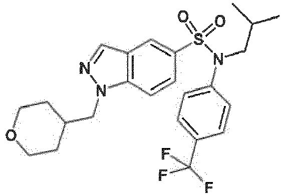 | 1-(tetrahydropyran-4-ylmethyl)-1H-indazole-5-sulfonic acid isobutyl(4-trifluoromethylphenyl)am ide |
| | Compound 59 |
| 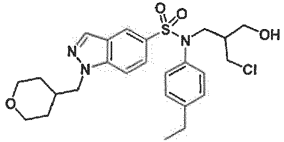 | 1-(tetrahydropyran-4-ylmethyl)-1H-indazole-5-sulfonic acid (3-chloro-2-hydroxymethylpropyl) (4-ethylphenyl) amide |
| | Compound 60 |
| 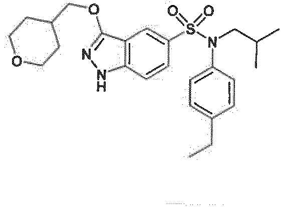 | 3-(tetrahydropyran-4-ylmethoxy)-1H-indazole-5-sulfonic acid (4-ethylphenyl)isobutylamid e |
| | Compound 61 |
| 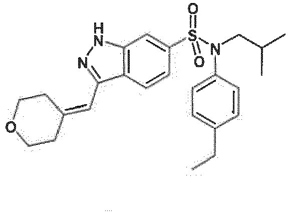 | 3-(tetrahydropyran-4-ylidenemethyl)-1H-indazole-6-sulfonic acid (4-ethylphenyl)isobutylamid e |
| | Compound 62 |
| 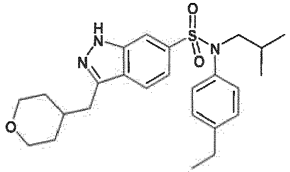 | 3-(tetrahydropyran-4-ylmethyl)-1H-indazole-6-sulfonic acid (4-ethylphenyl)isobutylamid e |
| | Compound 63 |
| 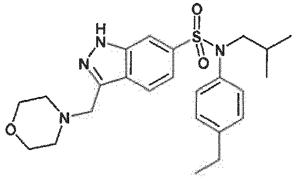 | 3-morpholin-4-ylmethyl-1H-indazole-6-sulfonic acid (4-ethylphenyl)isobutylamid e |
| | Compound 64 |
| 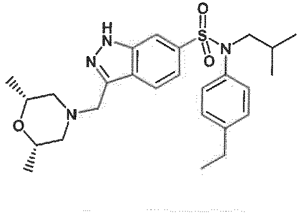 | 3-((cis)-2,6-dimethylmorpholin-4-ylmethyl)-1H-indazole-6-sulfonic acid (4-ethylphenyl)isobutylamid e |
| | Compound 65 |
| 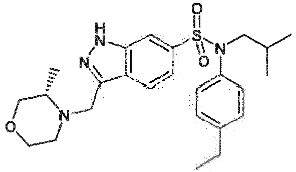 | 3-((S)-3-methylmorpholin-4-ylmethyl)-1H-indazole-6-sulfonic acid (4-ethylphenyl)isobutylamid e |
| | Compound 66 |
| 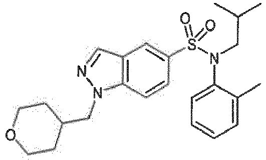 | 1-(tetrahydropyran-4-ylmethyl)-1H-indazole-5-sulfonic acid isobutyl-o-tolylamide |
| | Compound 69 |
| 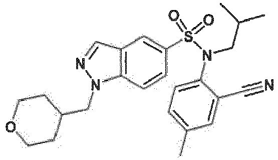 | 1-(tetrahydropyran-4-ylmethyl)-1H-indazole-5-sulfonic acid (2-cyano-4-methylphenyl)isobutylami de |
| | Compound 70 |
| 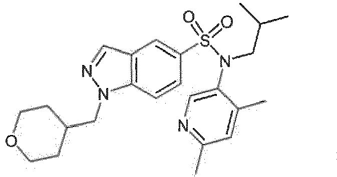 | 1-(tetrahydropyran-4-ylmethyl)-1H-indazole-5-sulfonic acid (4,6-dimethylpyridin-3-yl)isobutylamide |
| | Compound 71 |
| 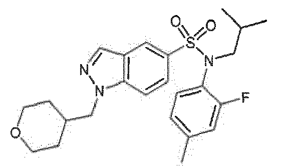 | 1-(tetrahydropyran-4-ylmethyl)-1H-indazole-5-sulfonic acid (2-fluoro-4-methylphenyl)isobutylami de |
| | Compound 73 |
| 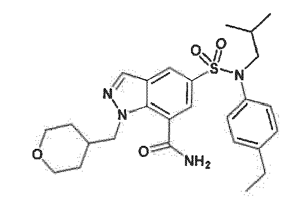 | 5-[(4-ethylphenyl)isobutylsu lfamoyl]-1-(tetrahydropyran-4-ylmethyl)-1H-indazole-7-carboxylic acid amide |
| | Compound 75 |

7. Compound according to any one of the preceding claims, for its use as a medicament.

8. Compound according to any one of Claims 1 to 6, for its use in the treatment of acne.

9. Compound according to any one of Claims 1 to 6, for its use in the treatment of psoriasis.

10. Pharmaceutical composition comprising one or more compounds as defined in any one of Claims 1 to 6.

11. Pharmaceutical composition as defined in Claim 10, for its use in the treatment of acne, atopic dermatitis and/or psoriasis.
